(19) Europäisches Patentamt
European Patent Office
Office européen des brevets

(11) Veröffentlichungsnummer: **0 163 854 B1**

(12) **EUROPÄISCHE PATENTSCHRIFT**

(45) Veröffentlichungstag der Patentschrift: **27.02.91**

(21) Anmeldenummer: **85104253.1**

(22) Anmeldetag: **09.04.85**

(51) Int. Cl.5: **C07C 233/77**, C07C 233/01, C07F 9/12, C07D 213/81, C07D 251/70, C07D 213/26, C07C 69/26, C07C 69/48, C08G 73/18, C08F 8/32, C08F 8/44

(54) **Verfahren zur Fluoreszenzlöschung und neue kationische oder amphotere aromatische Nitroverbindungen.**

(30) Priorität: **21.04.84 DE 3415103**

(43) Veröffentlichungstag der Anmeldung:
**11.12.85 Patentblatt 85/50**

(45) Bekanntmachung des Hinweises auf die Patenterteilung:
**27.02.91 Patentblatt 91/09**

(84) Benannte Vertragsstaaten:
**AT BE CH DE FR GB IT LI NL SE**

(56) Entgegenhaltungen:
**EP-A- 0 029 003**
**DE-A- 1 912 647**
**DE-A- 2 448 293**
**FR-A- 2 525 620**

(73) Patentinhaber: **BAYER AG**

**D-5090 Leverkusen 1 Bayerwerk(DE)**

(72) Erfinder: **Harnisch, Horst, Dr.**
**Heinenbusch 4**
**D-5203 Much(DE)**
Erfinder: **Siegel, Edgar, Dr.**

**verstorben(DE)**

**EP 0 163 854 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Löschung der durch anionische optische Aufheller erzeugten Fluoreszenz durch Einwirkung von praktisch farblosen, wasserlöslichen kationischen oder amphoteren Verbindungen, die mindestens eine Ammoniumgruppe, mindestens zwei Aryl-, Arylen-, Hetaryl- und/oder Hetarylengruppen und mindestens eine Nitrogruppe im Molekül enthalten.

Bevorzugte Nitroverbindungen enthalten 2 bis 6 Ammoniumgruppen, 2 bis 12 Aryl-, Arylen-, Hetaryl- und/oder Hetarylengruppen und 2 bis 9 Nitrogruppen im Molekül, handelt es sich um oligomere oder polymere Verbindungen, 1 bis 2 Ammoniumgruppen, 1 bis 3 Aryl- oder Arylen-Gruppen und 1 bis 3 Nitrogruppen pro wiederkehrende Einheit. Die genannten Gruppen sind direkt oder über Brückenglieder miteinander verbunden. Die Aromaten können durch nichtionische Reste oder durch anionische Reste wie Carbon- oder Sulfonsäuregruppen substituiert sein. Dabei ist die Summe der kationischen Gruppen gleich oder vorzugsweise größer als die der anionischen Gruppen. Bevorzugt sind nichtionische Substituenten.

Die Nitrogruppen können an aliphatische, olefinische, aromatische oder cycloaliphatische Reste gebunden sein; bevorzugt stehen sie jedoch an den Aryl-, Arylen-, Hetaryl- oder Hetarylen-Resten, wobei auf jeden dieser Reste bis zu 3, vorzugsweise bis zu 2 Nitrogruppen, kommen können.

Bevorzugte Aromaten sind Aryl und Arylen.

Aryl- und Arylen-Reste gehören beispielsweise der Benzol- oder Naphthalin-Reihe an, denen auch bis zu zwei 5- bis 6-gliedrige gesättigte carbo- oder heterocyclische Ringe wie Cyclopentano, Cyclohexano, Dioxano oder Tetrahydrofurano anelliert sein können.

Geeignete Hetaryl- oder Hetarylen-Reste sind beispielsweise der Furan-, Pyran-, Thiophen-, Pyrrol-, Pyrazol-, Oxazol-, Thiazol-, 1,2,4-Oxadiazol-, 1,3,4-Oxadiazol-, 1,3,4-Thiadiazol-, Imidazol-, 1,2,4-Triazol-, Pyridin-, Pyrimidin-, Pyrazin- und s-Triazin-Rest. Diese Ringe können miteinander kondensiert sein oder ihnen können 1 bis 2 Benzolringe anelliert sein, wie beispielsweise der Indolyl-(3)-, Carbazolyl-(2)- oder -(3)-, Benzthiazolyl-(2)-, Chinolyl-(3)-, Dibenzofuranyl-(3)- und Dibenzothiophen-S-dioxid- und 9-H-Thioxanthen-S-dioxid (3,6)-Rest. Bei den benzanellierten Heterocyclen stehen die Nitrogruppen vorteilhaft am Benzolring.

Diese Ringe und Ringsysteme können außer der Nitrogruppe weitere 1 bis 4 nichtionische Substituenten wie $C_1$-$C_4$-Alkyl, Halogen wie Chlor, Brom und Fluor, Hydroxy, $C_1$-$C_4$-Alkoxy, Cyan, Carbamoyl, Sulfamoyl, $C_1$-$C_4$-Alkoxy-carbonyl, $C_1$-$C_4$-Alkylsulfonyl und Trifluormethyl und können außerdem eine saure Gruppe, insbesondere eine Sulfonsäuregruppe tragen.

Die Ammoniumgruppen können endständig an das Molekül gebunden sein oder sie können ein Brückenglied darstellen. Sie können acyclisch oder in gesättigte, teilgesättigte oder heteroaromatische Ringe als Ringglieder eingebunden sein. Es können auch zwei derartige Cyclammoniumgruppen einem biskationischen Piperazin- oder Triethylendiaminring angehören.

Als Beispiele für gesättigte Cyclammoniumgruppen sind Piperazinium-, Morpholinium-, Piperidinium- und Pyrrolidinium-Reste, für teilgesättigte Imidazolinium- und Tetrahydropyrimidinium-Reste und für Heteroaromaten Pyridinium-, Chinolinium-, Imidazolium- und Thiazolium-Reste zu nennen.

Als Beispiel für acyclische Ammoniumgruppen seien Trialkylammonium-, Tetraalkylammonium-, N,N,N-Trialkyl-N-aryl-ammonium-, N,N,N-Trialkyl-hydrazinium-, N,N,N-Trialkyl-N-hydroxylammonium, Amidinium-, Guanidinium- und Thiuronium-Reste genannt. Die Ammoniumgruppen können innere Salze bilden, beispielsweise auch als $-\overset{\oplus}{N}-O^{\ominus}$-Verbindungen vorliegen.

Besonders zu nennende acyclische Ammoniumgruppen haben die Formeln

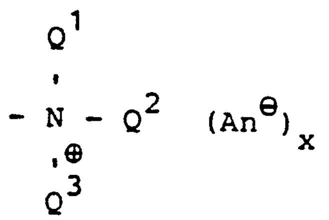 , 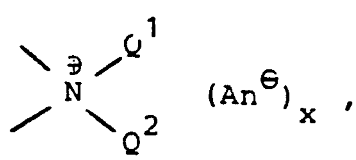 , 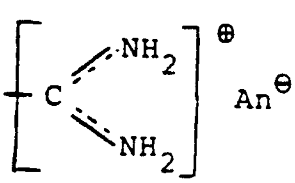 ,

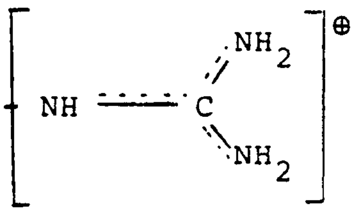 $An^{\ominus}$ und 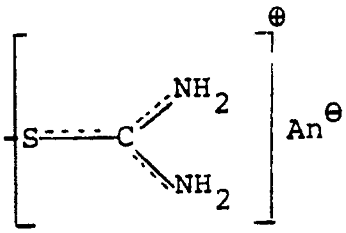 $An^{\ominus}$

worin

$Q^1$, $Q^2$ und $Q^3$ für Wasserstoff, $C_1$-$C_4$-Alkyl, das durch OH, $NH_2$, $C_1$-$C_4$-Alkoxy, Halogen, $CONH_2$, CN, COOH oder $C_1$-$C_4$-Alkoxycarbonyl substituiert sein kann, $C_2$-$C_4$-Alkenyl, Cyclohexyl, Phenyl-$C_1$-$C_3$-alkyl oder Benzoylmethyl, die durch Nitro, $C_1$-$C_4$-Alkyl, $C_1$-$C_4$-Alkoxy, Chlor oder Brom kernsubstituiert sein können,

$Q^1$ außerdem für - $\overline{O}|^{\ominus}$,

$An^{\ominus}$ für ein farbloses Anion und

x für 0 oder 1 stehen.

Besonders zu nennende Cyclammoniumgruppen haben die Formeln

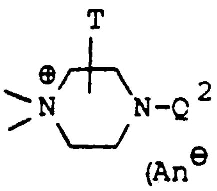 $(An^{\ominus})_x$ 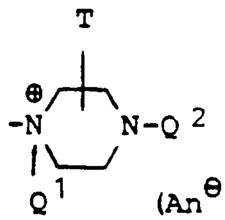 $(An^{\ominus})_x$ 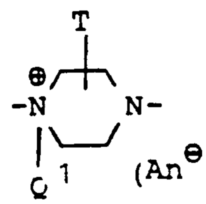 $(An^{\ominus})$ 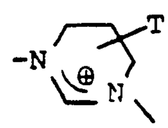 $(An^{\ominus})$

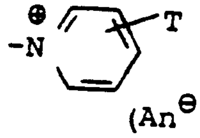 $(An^{\ominus})_x$ 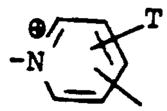 $(An^{\ominus})_x$ 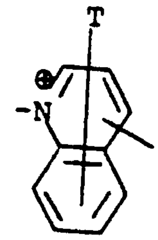 $(An^{\ominus})_x$

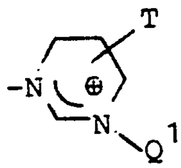 $(An^{\ominus})_x$ 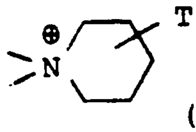 $(An^{\ominus})_x$ 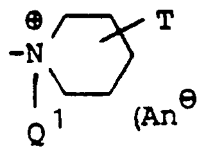 $(An^{\ominus})_x$

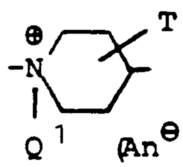 $(An^{\ominus})$ 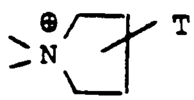 $(An^{\ominus})_x$ 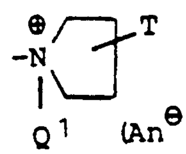 $(An^{\ominus})_x$

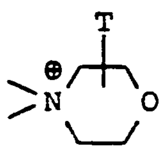 $(An^{\ominus})_x$ 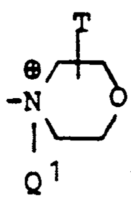 $(An^{\ominus})_x$ 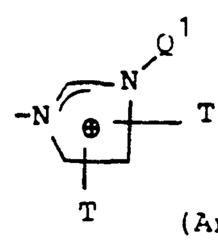 $(An^{\ominus})_x$ 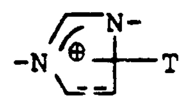 $(An^{\ominus})_x$

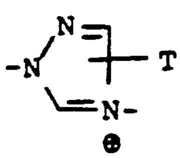 $(An^{\ominus})_x$ 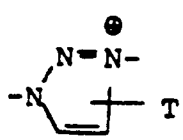 $(An^{\ominus})_x$ oder 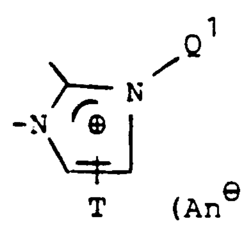 $(An^{\ominus})_x$

worin

T für H oder - in Abhängigkeit von der Art des Ringes - 1 - 4 Methylgruppen steht und x, $An^{\ominus}$, $Q^1$ und $Q^2$ die oben angegebene Bedeutung haben.

Besonders bevorzugt sind quartäre Ammoniumgruppen, also solche, die nicht durch Protonierung sondern durch Alkylierung oder Aralkylierung eines tertiären Stickstoffatoms entstehen ($Q^1 \neq H$).

Als Anionen kommen die üblichen farblosen anorganischen oder organischen wasserlöslichmachenden Anionen in Betracht wie Chlorid, Bromid, Jodid, Chlorozinkat, Tetrafluoroborat, Sulfat, Hydrogensulfat, Methosulfat, Ethosulfat, Benzolsulfonat, p-Toluolsulfonat, Methylsulfonat, Amidosulfonat, Nitrat, Hydrogenphosphat, Methylphosphonat, Acetat, Lactat, Formiat, Maleat, Succinat, Citrat, Tartrat und Oxalat.

Geeignete Brückenglieder, die die (Het)aryl(en)-Gruppen miteinander oder diese Gruppen mit den Ammoniumgruppen verbinden, sind solche, die unter Applikationsbedingungen beständig sind, also beispielsweise in Wasser nicht hydrolysiert werden. Sie sind in der Regel zwei- oder drei-, in seltenen Fällen

auch vierbindig.

Die Brückenglieder können acyclisch und cycloaliphatisch sein. Als Beispiele seien genannt:

$-O-$, $-S-$, $-NR^5-$, $-\dot{N}-$, $-CO-$, $-SO-$, $-SO_2-$, $-COO-$, $-CONR^5-$, $-CONR^5-NH-$, $-NH-CO-NR^5-$, $-SO_2NR^5-$, $-\dot{C}H-$, $-\dot{C}-$, $C_1-C_7$-Alkylen, das geradkettig oder verzweigt sein kann, $-O-C_1-C_4$-Alkylen-O-,

$$-O-\overset{\overset{\displaystyle O}{\|}}{\underset{\underset{\displaystyle O-}{|}}{P}}-O-$$

, -CH=CH-, 5- oder 6-gliedrige gesättigte isocyclische oder heterocyclische Ringe, insbesondere 1,4- oder 1,1-Cyclohexylen. $R^5$ steht für Wasserstoff, $C_1$-$C_4$-Alkyl , $C_3$- oder $C_4$-Alkenyl, Phenyl, Benzyl oder Cyclohexyl.

Die genannten Kohlenwasserstoffreste können auch zusammen mit einem oder zwei der übrigen Brückenglieder eine gemeinsame Brücke bilden.

Nichtchromophore Brückenglieder, die sich in der Chemie und Anwendung der kationisch-substantiven Farbstoffe bewährt haben, sind auch für die kationischen und amphoteren Nitroverbindungen des erfindungsgemäßen Verfahrens als Brückenglieder bestens geeignet.

Die Wasserlöslichkeit der Verbindungen beträgt in der Regel mindestens 1 g/l bei 20 °C.

Eine Gruppe von Verbindungen, die vorzugsweise zur Löschung der Fluoreszenz verwendet werden, besitzt die Formel

$$(O_2N)_{\overline{n}}\left[ (\overset{\oplus}{K}{}^1-Z^2-Y^2-X^2-G-)_r\ W \right] (r-a).An^{\ominus}$$

(I)

worin

$\overset{\oplus}{K}{}^1$ für $\overset{\oplus}{E}{}^1$- oder $A-X^1-Y^1-Z^1-\overset{\oplus}{E}-$ ,

A für einen Benzol-, Biphenyl-, Naphthalin-, Furan-, Benzofuran, Thiophen-, Benzothiophen-, Thiazol-, Benzthiazol-, Benzimidazol-, 1,3,4-Thiadiazol- oder Indol-Rest, der außer durch Nitro durch $C_1$-$C_4$-Alkyl , Halogen, Carbamoyl, Sulfamoyl, Cyan, Hydroxy, $C_1$-$C_4$-Alkoxy oder eine Sulfonsäuregruppe substituiert sein kann,

$X^1$ für $-(CH_2)_t-CO-$, $-CH_2-Y^1-CO-$, $-(CH_2)_t-SO_2-$, $-O-CH_2-CO-$, $-O-CH_2-SO_2-$, $-S-CH_2-CO-$, $-S-CH_2-SO_2-$ oder eine Einfachbindung,

t für 0, 1 oder 2,

$X^2$ für -CO-, -CO-NH-CO-, $-SO_2-$ oder eine Einfachbindung,

$Y^1$ und $Y^2$ je für -O-, -S-, -N(R)-, -N(R)-NH- oder eine Einfachbindung,

R für Wasserstoff, $C_1$-$C_2$-Alkyl oder Cyanethyl oder in $Y^1$ und $Y^2$ auch für -CO- oder $SO_2$, die mit der o- oder peri-Stellung von A bzw. eines Benzolringes W verbunden sein können,

$Z^1$ und $Z^2$ je für $C_1$-$C_6$-Alkylen, p-Benzylen, p-Xylylen oder eine Einfachbindung stehen,

$E^{\oplus}$ für $-\overset{\oplus}{N}(R^1R^2)-$,

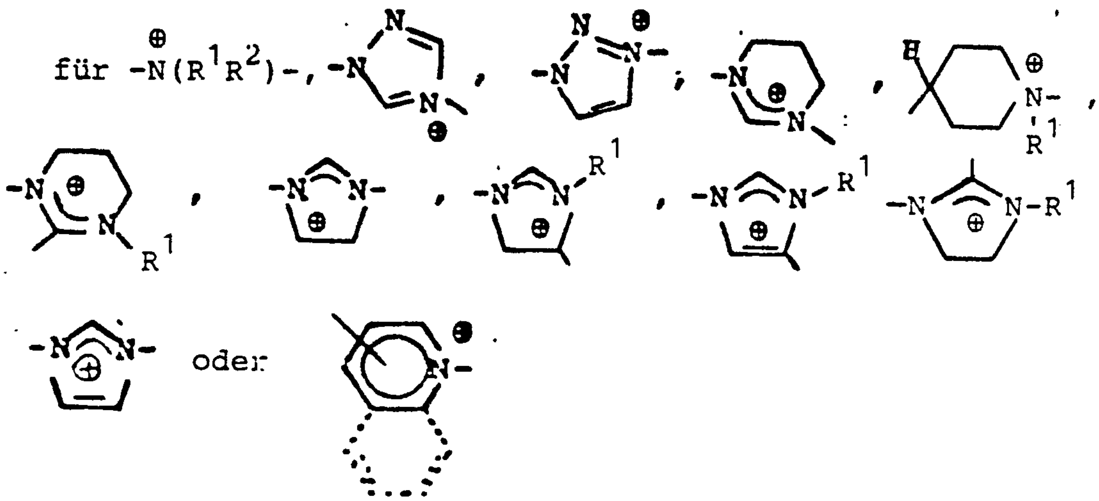

oder

stehen, wobei diese Ringe gegebenenfalls durch 1 - 4 Methylgruppen substituiert sein können, und jede der beiden freien Valenzen an $-X^1-Y^1-Z^1-$oder an $-Z^2-Y^2-X^2-G-$ gebunden sein kann,

$\overset{\oplus}{E}{}^1$ für $-\overset{\oplus}{N}(R^1R^2R^2)$,

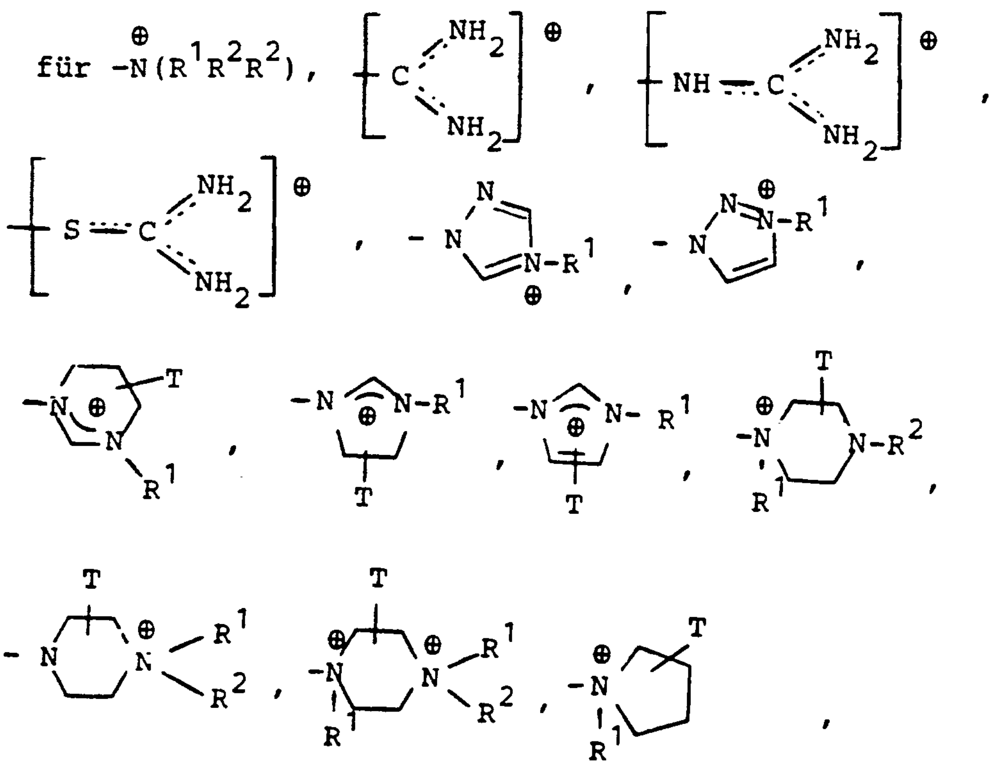

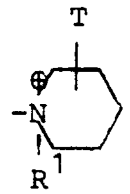,
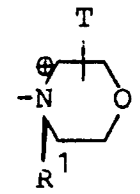,
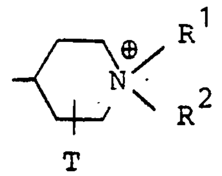,

oder 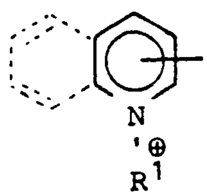, 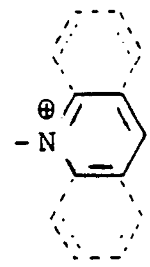, 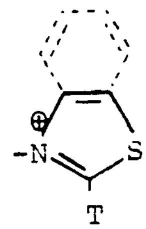

oder 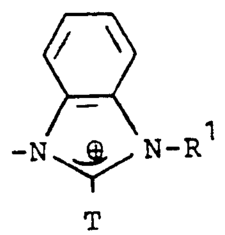

T für Wasserstoff oder - in Abhängigkeit von der Art des Ringes - für 1-4 Methylgruppen stehen,

worin die aromatischen Reste außer durch 1 bis 2 Nitro auch durch $C_1$-$C_4$-Alkyl , Halogen, Cyan oder Carbamoyl substituiert sein können und die Amidinium-, Guanidinium- und Thiuronium-Reste auch durch 1-2 Reste $R^2$ substituiert sein können.

$R^1$ und $R^2$ unabhängig voneinander für Wasserstoff, $C_1$-$C_4$-Alkyl , das durch OH, $NH_2$,Halogen, $C_1$-$C_4$-Alkoxy, COOH, $CONH_2$, CN oder $C_1$-$C_4$-Alkoxycarbonyl substituiert sein kann, $C_2$-$C_4$-Alkenyl, Cyclohexyl, Phenyl-$C_1$-$C_3$-alkyl oder Benzoylmethyl, die außer durch Nitro auch durch $C_1$-$C_4$-Alkyl, $C_1$-$C_4$-Alkoxy, Cl oder Br kernsubstituiert sein können,

$R^1$ außerdem für - $O^{\ominus}$ oder A-$X^1$-$Y^1$-$Z^1$-,

R und $R^1$ auch ringgeschlossen sind und dann zusammen mit -N-$Z^1$-N- oder -N-$Z^2$-N- einen gegebenenfalls durch 1 bis 2 Methylgruppen substituierten Piperazin-Rest bilden, oder

$R^1$ und $R^2$ auch ringgeschlossen sind und dann zusammen mit dem Stickstoffatom, an das sie gebunden sind, einen gegebenenfalls durch 1 bis 4 Methylgruppen substituierten Pyrrolidin-, Piperidin-, Morpholin- oder Piperazin-Ring bilden, der am zweiten N-Atom durch gegebenenfalls durch OH oder $NH_2$ substituiertes $C_1$- bis $C_3$-Alkyl substituiert sein kann,

G für -NH-, -NH-$CH_2$-, -O- oder eine Einfachbindung,

W für einen r-bindigen Rest aus der Reihe Benzol, Naphthalin, Anthracen, Phenanthren, 9,10-Dihydrophenanthren, Cyclohexan, Fluoren-9-on(3,6), Thiophen(2,5), Dibenzofuran(3,6), Dibenzothiophen(3,6), Dibenzothiophen-S-dioxid(2,7) ,9-H-Thioxanthen-S-dioxid (3,6), Carbazol(3,6),9-H-Xanthen-9-on(2.7), 9-Acridon(2,7),1, 3,4-Oxadiazol(2,5),1,2,4-Oxadiazol(3,5), 1,3,4-Thiadiazol(2,5), s-Triazin(2,4,6), Piperazin-(1,4), 1 ,2-Dihydro-1,2,4,5-tetrazin(3,6) oder für einen Rest der Formeln

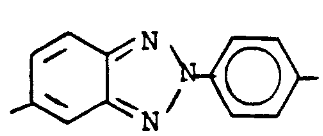, 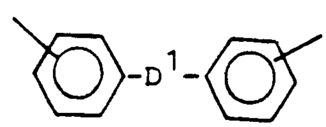 oder 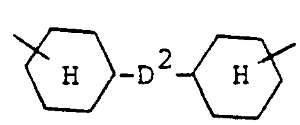

oder

für den Fall, daß -$\overset{\oplus}{E}$- einen zweibindigen Pyridinium- oder Chinolinium-Rest bedeutet, auch für eine Einfachbindung,

$D^1$ und $D^2$ unabhängig voneinander für einen geradkettigen oder verzweigten, gegebenenfalls durch -O-

unterbrochenen $C_1$-$C_7$-Alkylenrest , -O-$C_2$-$C_4$-Alkylen-O-, -O-, -NH-, -N($C_1$-$C_2$-Alkyl)-, -CO-, -CO-NH-, -NH-CO-NH-, 1,1-Cyclohexylen oder eine direkte Bindung,
$D^1$ außerdem für -CH($C_6H_5$)-, -CH($C_6H_4$-)-, -N($C_6H_5$)-, -CH=CH-, -S-, -$SO_2$-, -SO-, m- oder p-Phenylen, Thiophen(2,5), 1,3,4-Oxadiazol(2,5), 1,3,4-Thiadiazol(2,5), Oxazol(2,5) , Thiazol(2,5), 1,2-Dihydro-1,2,4,5-tetrazin(3,6) oder

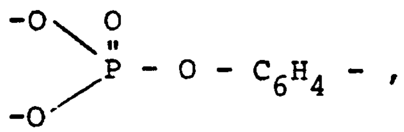

$D^2$ außerdem für -CH($C_6H_{11}$)- oder -CH($C_6H_{10}$-)-, wobei
die unter W, $D^1$ und $D^2$ genannten Ringe außer durch Nitro auch durch $C_1$-$C_4$-Alkyl, $C_1$-$C_4$-Alkoxy, Halogen und/oder eine Sulfonsäuregruppe substituiert sein können,
n für eine ganze Zahl von 1 bis 6,
a der Anzahl der anionischen und/oder $\overset{\oplus}{N}-\overset{\ominus}{O}$ -Gruppen entsprechend für 0, 1, 2 oder 3,
r für 2 oder 3, wobei r > a ist, und
$An^{\ominus}$ falls vorhanden, für ein farbloses Anion stehen, und worin die r-fach vorhandenen Ketten gleich oder verschieden sein können, und worin im Falle r = 2 die Gruppierung

$$-\overset{\oplus}{E}-Z^2-Y^2-X^2-G-W-G-X^2-Y^2-Z^2-\overset{\oplus}{E}-$$ insgesamt auch für

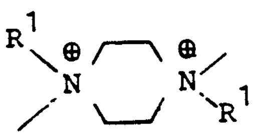 oder 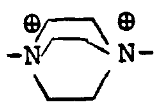

stehen kann.

Ebenfalls bevorzugt verwendet werden Verbindungen der Formel

$$(O_2N)_{\overline{n}}\left[(A-X^1-Y^1-Z^1-Y'-)_e-W^1-(G^2-Y^2-Z^2-\overset{\oplus}{E}{}^1)_f\right](An^{\ominus})_x$$

worin
Y' für -O-, -S-, -NH-, -N($CH_3$) - oder eine Einfachbindung,
e für 1, 2 oder, wenn W' und/oder $\overset{\oplus}{E}{}^1$ insgesamt mindestens eine Nitrogruppe enthalten und W' ≠ W ist, auch für 0,
f für 2 oder 3,
x je nach der Anzahl der anionischen und/oder $\overset{\oplus}{N}-\overset{\ominus}{O}$ -Gruppen im Molekül und abhängig von f, für 0, 1, 2 oder 3

$W^1$ für 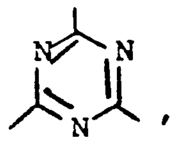 , 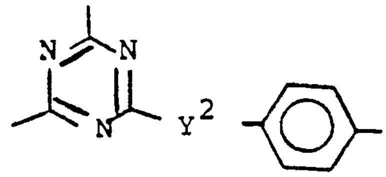 ,

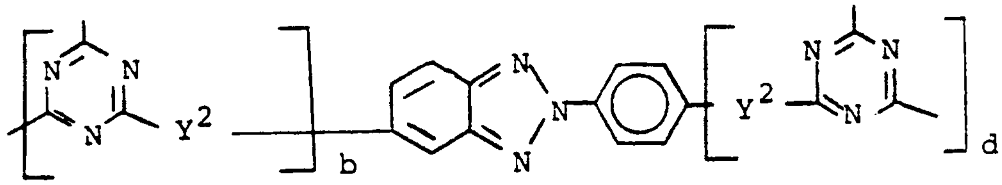 ,

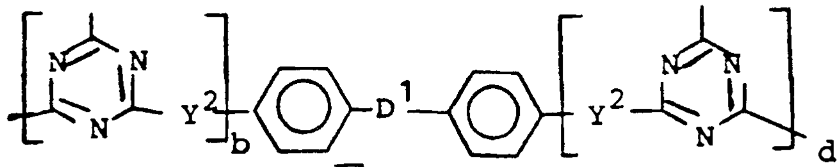 ,

oder 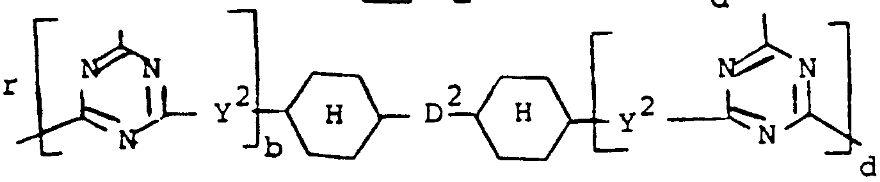

b und d für 0 oder 1 und

$G^2$ für -NH-CO- oder eine Einfachbindung stehen und worin n, A, $\overset{\oplus}{E}{}^1$, $x^1$, $Y^1$, $Y^2$, $Z^1$, $Z^2$, $R^1$, $R^2$, x und $An^{\ominus}$ die gleiche Bedeutung wie in Formel (I) besitzen.

Y', $Y^1$ und $Y^2$ stehen insbesondere für -N(R)-,

$\overset{\oplus}{E}{}^1$ steht insbesondere für $-\overset{\oplus}{N}(R^1R^2R^2)$, 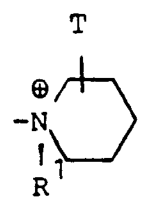 , 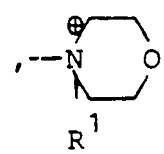 ,

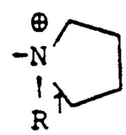 , 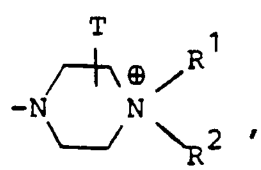 , 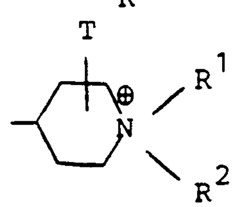 ,

oder 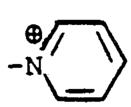

worin Pyridinium außer durch 1 bis 2 Nitro auch durch Methyl, Ethyl, Chlor, Brom, Cyan oder Carbamoyl substituiert sein kann.

Geeignete $C_1$-$C_7$-Alkylenreste $D^1$ und $D^2$, die auch durch -O- unterbrochen sein können, sind beispielsweise $-CH_2-$, $-CH_2-CH_2-$, $-CH(CH_3)-$, $-C(CH_3)2-$, $-CH_2-CH_2-CH_2-$, $-CH_2-C(CH_3)_2-CH_2-$, $-C(CH_3)_2-CH_2-C(CH_3)_2$ - und $-CH_2-CH_2-O-CH_2-CH_2-$.

$R^1$ und $R^2$ stehen bevorzugt für $C_1$-$C_4$-Alkyl, das durch OH, $C_1$-$C_4$-Alkoxycarbonyl, CN, Carboxy oder Carbamoyl substituiert sein kann, Allyl, Benzyl oder Benzoylmethyl, die durch 1 bis 2 Nitrogruppen kernsubstituiert sein können.

Als Halogen ist insbesondere Chlor und Brom zu nennen.

Bevorzugt verwendet werden außerdem oligomere oder polymere Verbindungen, welche pro wiederkehrende $\overset{E}{\oplus}$ inheit 1 bis 3 Nitrogruppen und 1 bis 2 kationische $\overset{\oplus}{E}{}^1$ -Gruppen enthalten, wobei $\overset{\oplus}{E}{}^1$ die gleiche

Bedeutung wie in Formel (I) besitzt, dadurch gekennzeichnet, daß sie erhalten werden

a) durch Polymerisation oder Mischpolymerisation von einem oder mehreren Monomeren aus der Gruppe Maleinsäureanhydrid,

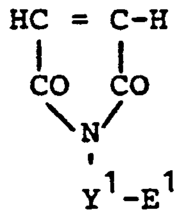 , 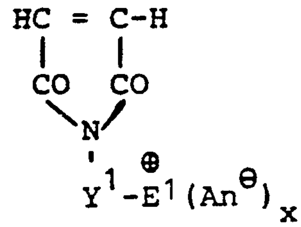 ,

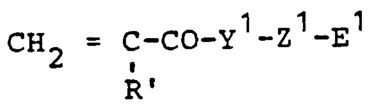 , 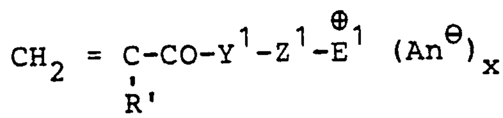 ,

N-Allylpyridiniumhalogenid oder dessen Addukte mit einem Nitrobenzaldehyd, $E^1$-CH=$CH_2$ wie 2- oder 4-Vinyl-pyridin und gegebenenfalls zusätzlich Vinylchlorid, Vinylacetat, Styrol, α-Methylstyrol oder 4,4'-$\overset{\oplus}{D}$ vinylbenzol, die durch Methyl, Chlor, $E^1$ -$(CH_2)_v$-$E^1(CH_2)$v- und/oder Nitro kernsubstituiert sein können,

wobei R' für Wasserstoff oder Methyl,

$E^1$ für eine durch Umsetzung mit $R^1$L in -$\overset{\oplus}{E}$ $^1$ überführbare basische Gruppe,

L für eine als Anion abspaltbare Gruppe und

v für 0 oder 1 stehen

und

$Y^1$ ,$Z^1$ ,$\overset{\oplus}{E}$ $^1$ ,$An^\ominus$ , x und $R^1$ die oben angegebene Bedeutung haben,

für den Fall, daß Maleinsäureanhydrid eingesetzt wird, durch weitere Umsetzung mit einer Verbindung der Formel HN(R)-$Z^1$-$E^1$ oder HN(R)-$Z^1$-$\overset{\oplus}{E}$ $^1$ $(An^\ominus)$x

worin

R,$Z^1$,$E^1$,$\overset{\oplus}{E}$ 1, $An^\ominus$ und x die oben angegebene Bedeutung haben,

und, falls noch nichtkationische $E^1$-Gruppen vorliegen, durch deren weitere Umsetzung mit $R^1$-L,

worin $R^1$ und L die oben angegebene Bedeutung haben, sowie gegebenenfalls durch anschließende Mono- oder Dinitrierung vorhandener aromatischer Gruppen

oder

b) durch Polykondensation von Di-($C_2$-$C_3$-alkylen)-triaminen oder Tri-($C_2$-$C_3$-alkylen)-tetraminen der Formel

$$H_2N-\left[(CH_2)_k-E\right]_w (CH_2)_k-NH_2$$

worin

k für 2 oder 3,

w für 1 oder 2 steht und ⊕

E für eine durch Umsetzung mit $R^1$-L in -$\overset{\oplus}{E}$ -überführbare basische Gruppe steht,

mit Dicarbonsäuren der Formel

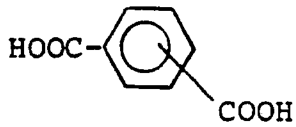 und/oder $HOOC-(CH_2)_g-COOH$

oder ihren Derivaten (wie Säurechloriden, Anhydriden, Niederalkylestern, Imino-niederalkylesterhydrochloriden oder Nitrilen), worin g für eine ganze Zahl von 0 bis 6 steht, und

durch weitere Umsetzung mit $R^1$-L, worin $R^1$ und L die oben angegebene Bedeutung haben,

sowie gegebenenfalls anschließende Mono- oder Dinitrierung vorhandener aromatischer Gruppen.

Die Anzahl der wiederkehrenden Einheiten im Molekül beträgt etwa 8 bis $8.10^4$, vorzugsweise 100 bis 5000. Die aromatisch gebundenen Nitrogruppen können beispielsweise in Phenylresten von gegebenenfalls copolymerisiertem Styrol, ∝-Methylstyrol oder 1,4-Divinylbenzol, in gegebenenfalls vorhandenen Phenylengruppen in $Z^1$ oder in $\overset{\oplus}{E}{}^1$ bzw. $-\overset{\oplus}{E}-$, vorzugsweise als

$-CH_2-(CO)_{q^1}$ 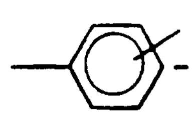-Rest, mit $q^1$ = 0 oder 1, oder als

Substituent in einem Terephthal- oder Isophthalsäureglied vorliegen.

Geeignete Ausgangsverbindungen der Formel

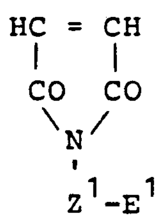 und 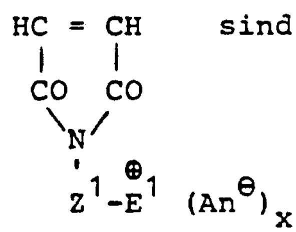 sind beispielsweise

solche, in denen $-Z^1-E^1$ bzw. $-Z^1-\overset{\oplus}{E}{}^1$ für Reste wie

$-(CH_2)_3-N(CH_3)_2$, $-(CH_2)_3-\overset{\oplus}{N}(CH_3)_3$

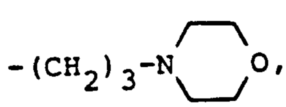, 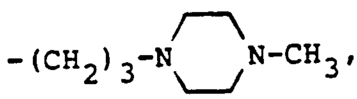, $-(CH_2)_3-N(C_2H_4OH)_2$,

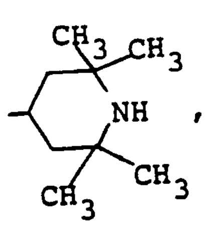, 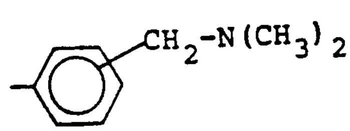, 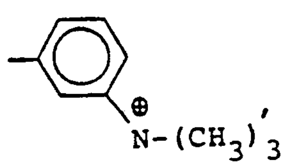,

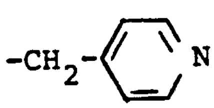 oder 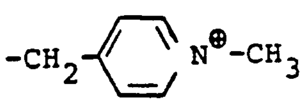 stehen.

Bevorzugte Ausgangsverbindungen der Formel

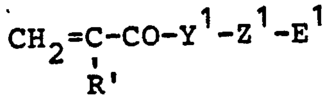 und 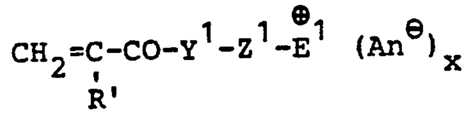 sind

Acryl- oder Methacrylsäureester oder -amide, die über ein Brückenglied $Z^1$ mit einem basischen Rest $-E^1$ oder eine kationische Gruppe $-\overset{\oplus}{E}{}^1$ verbunden sind, wie

$$CH_2{=}CH{-}CO{-}NH{-}(CH_2)_3{-}N(CH_3)_2,$$

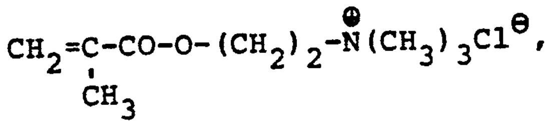

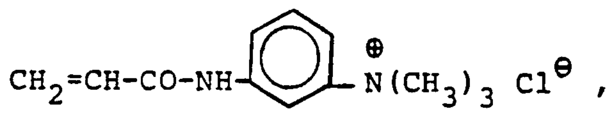

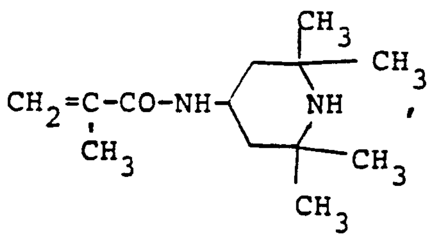

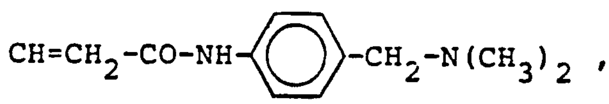

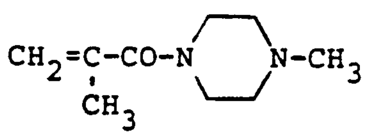 oder

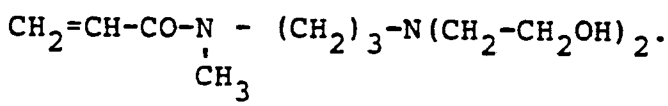

Die Polymerisation bzw. Mischpolymerisation von unter a) genannten Monomeren erfolgt nach üblichen Verfahren, beispielsweise in inerten Lösungsmitteln, wie Ethylacetat oder Toluol bei Temperaturen von 15 bis 120° C, vorzugsweise 50 bis 90° C, in Gegenwart eines Polymerisationsinitiators wie Benzoylperoxid.

Die Mischpolymerisation von Maleinsäureanhydrid mit Styrol oder Nitrostyrol kann beispielsweise nach DE-B- 1 495 850, die weitere Umsetzung mit HY'-Z'-E', wie N,N-Dimethylpropylendiamin, zweckmäßig nach DE-B-1 469 727 vorzugsweise ohne Zwischenisolierung des polymeren Vorprodukts im Temperaturbereich von 20 bis 100° C durchgeführt werden.

Gegebenenfalls kann ein so erhaltenes Amid auch mit Essigsäureanhydrid/Natriumacetat bei 40 - 120° C zum entsprechenden Imid cyclisiert werden.

Geeignete Di-($C_2$-$C_3$-alkylen)-triamine und Tri-($C_2$-$C_3$-alkylentetramine sind beispielsweise Diethylen-

triamin, Triethylentetramin, $[H_2N-(CH_2)_3]_2N-CH_3$, Dipropylentriamin oder

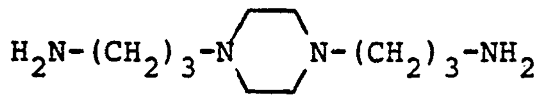.

Die Polykondensation der Dialkylentriamine bzw. Trialkylentetramine mit den unter b) angegebenen Dicarbonsäuren bzw. Dicarbonsäurederivaten wie Terephthalsäure, Isophthalsäure und/oder Adipinsäure erfolgt zweckmäßig gemäß DE-A-1 912 647 und DE-A-2 448 293 in einem inerten hochsiedenden Lösungsmittel wie Di- oder Triethylenglykol oder einem Polyglykolgemisch bei Temperaturen von 150 bis 230 °C, vorzugsweise unter Abdestillieren von Wasser.

Typische als Anion abspaltbare Gruppen L sind Halogenatome wie Chlor, Brom und Iod, ferner Arylsulfonatreste, wie Benzol- oder p-Toluolsulfonat, sowie Methosulfat und Ethosulfat.

Geeignete Verbindungen $R^1$-L sind beispielsweise Chlorwasserstoff und Bromwasserstoff oder Alkylierungsmittel wie Dimethylsulfat, Diethylsulfat, p-Toluolsulfonsäureethylester, Methylchlorid, Ethylbromid, Propyliodid, n-Butylbromid, Ethylenoxid, Propylenoxid, Aziridin, 3-Chlor-propylbromid, 2-Methoxy-ethylbromid, Bromessigsäure, Chloracetamid, Chloracetonitril, Acrylnitril, Chloressigsäureethylester, Allylbromid, Cyclohexylbromid, Benzylchlorid, Phenacylchlorid, 3-Nitrophenacylbromid und 4-Nitrobenzylchlorid.

Die Umsetzung der unter a) genannten Polymeren oder der unter b) genannten Polykondensate mit $R^1$-L wird zweckmäßig in einem inerten Lösungsmittel im Temperaturbereich von 20 bis 160 °C analog der weiter unten beschriebenen Umsetzung von (XI) mit (X) durchgeführt.

Geeignete inerte Lösungsmittel sind die gleichen, wie bei der Reaktion von (IX) mit (X). Die nachträgliche Nitrierung von Aryl- bzw. Arylenresten in unter a) beschriebenen Polymeren oder in unter b) beschriebenen Polykondensaten wird zweckmäßig mit nitrierend wirkenden Mitteln, insbesondere mit Salpetersäure oder Salpetersäure-Schwefelsäure-Gemischen im Temperaturbereich von -5 bis 90 °C durchgeführt, wobei in jeden Aryl- bzw. Arylenrest vorzugsweise 1 bis 2 Nitrogruppen eintreten können.

Eine besonders bevorzugt verwendete Gruppe von Verbindungen gemäß a) enthält wiederkehrende Einheiten der Formel

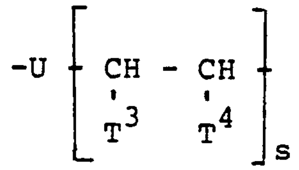 (III)

worin

U für 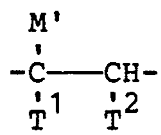 , 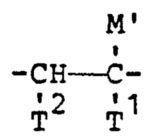 oder 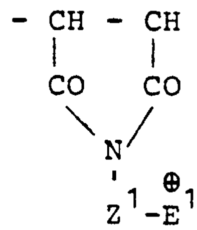 $(An^{\ominus})_x$ ,

s für 0 oder 1,

M' für Wasserstoff oder Methyl,

$T^1$ für $-CO-N(R)-Z^1-\overset{\oplus}{E}{}^1$ $(An^{\ominus})_x$ oder 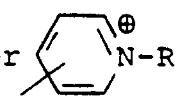 $(An^{\ominus})_x$,

$T^2$ für $T^1$, COOH oder - im Falle $T^1$ = 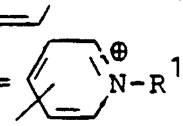 $(An^{\ominus})_x$ - für Wasserstoff

und einer der Reste $T^3$ und $T^4$ für Phenyl, Toluyl oder Chlorphenyl, die durch Nitro substituiert sein können, und der andere für Wasserstoff oder Methyl steht,

R, Z', $\overset{\oplus}{E}$', $An^{\ominus}$, x und R' die gleiche Bedeutung wie in Formel (1) besitzen,

die Anzahl der Sulfo- und/oder - $O^{\ominus}$ Gruppen gleich oder kleiner ist als die der kationischen Ladungen und worin pro wiederkehrende Einheit 1 bis 3 aromatisch gebundene Nitrogruppen vorhanden sind. Eine besonders bevorzugt verwendete Gruppe von Verbindungen gemäß b) enthält wiederkehrende Einheiten der Formel

$$-\left[NH-(CH_2)_k-B^1-CO\right]_z-B^2- \qquad (IV)$$

worin

$B^1$ 1) für 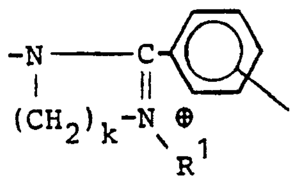 oder 2) für 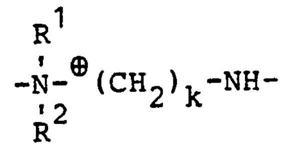

$(An^{\ominus})_x$ $(An^{\ominus})_x$

$B^2$ - falls $B^1$ die unter 1 angegebene Bedeutung hat und z = 0 oder 1 ist - für

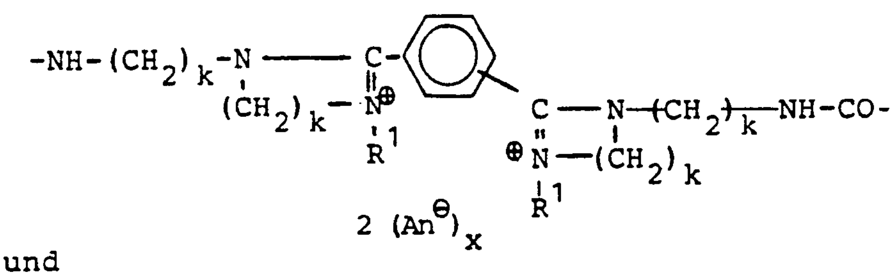

2 $(An^{\ominus})_x$

und

- falls $B^1$ die unter 2) angegebene Bedeutung hat und z = 1 ist - für 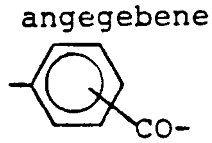 oder

$-(CH_2)_g-CO-$,

k für 2 oder 3 und

g für eine ganze Zahl von 0 bis 6 stehen und worin

x, $R^1$, $R^2$ und $An^{\ominus}$ die gleiche Bedeutung wie in Formel (I) besitzen,

die Anzahl der sauren und/oder - $O^{\ominus}$ Gruppen gleich oder kleiner ist als die der kationischen Ladungen und worin pro wiederkehrende Einheit 1 bis 3, vorzugsweise 1 bis 2 aromatisch gebundene Nitrogruppen vorhanden sind.

Typische anionische optische Aufheller sind solche, die der Stilben-, Distyrylbenzol-, Distyrylbiphenyl- und 1,3-Diphenyl-pyrazolin-Reihe angehören, insbesondere 4,4'-Bistriazinylaminostilben-2,2'-disulfosäuren, 4,4'-Bistriazolylstilben-2,2'-disulfonsäuren, Naphthotriazolylstilben-mono-oder -disulfonsäuren und 4,4'-Bis-(sulfostyryl)-biphenyl-Typen. Sie sind u.a. von H. Gold in K. Venkataraman, The Chemistry of Synthetic Dyes, Vol. V, p. 535-678 und in Ullmanns Encyclopädie der technischen Chemie (4. Auflage), Band 17, Seite 459 bis 473 beschrieben. Durch die Löschung der blauen Fluoreszenz der anionischen Weißtöner heben die kationischen Nitroverbindungen die Weißtönung auf den behandelten Substraten auf. Das Verfahren kann so durchgeführt werden, daß weißgetönte Substrate wie natürliche, halbsynthetische oder synthetische Substrate, beispielsweise natürliche oder regenerierte Cellulose, Papiermassen oder -blätter,

Papierbeschichtungen, Seide, Wolle, Jute, Hanf sowie auch synthetische Polyamide, oder auch Rückstände von Weißtönern mit den kationischen Nitroverbindungen behandelt werden.

Das erfindungsgemäße Verfahren kann auch zur Erhaltung heller, klarer Farbtöne auf Cellulosematerialien verwendet werden, wenn die kationischen Nitroverbindungen dem Färbebad zugesetzt werden, oder die Cellulosematerialien mit den Verbindungen nach- oder vorbehandelt werden. Auf diese Weise wird der nachteilige Einfluß von Waschmittelweißtönern auf den Farbton vermieden bzw. aufgehoben.

Das erfindungsgemäße Verfahren eignet sich besonders gut zur Herstellung von nicht aufgehelltem Papier unter Verwendung von weißgetöntem Papier als Rohstoff, zum raschen, bequemen Entfernen unerwünschter Restanteile von optisch aufgehellten Papiermassen in Papiermaschinen und zum Unwirksammachen unerwünschter Restanteile von wäßrigen oder organischen, mit Wasser mischbaren Lösungen oder Färbeflotten anionischer Weißtöner in Färbeapparaturen, Färbemaschinen, Vorratsgefäßen und Zuleitungen. Es wird verhindert, daß diese Restanteile bei anschließenden an sich weißtönerfreien Applikationsverfahren noch unerwünschte Weißtönungen oder Fluoreszenzeffekte bewirken.

Bei der Beschichtung von Papier kann man das kationische oder amphotere Nitroderivat in der Beschichtungsmasse (wie Stärke) verteilen und so durch die Beschichtungsoperation auf die Papieroberfläche aufbringen.

Bei der Herstellung von nichtweißgetöntem Papier aus weißtönerhaltigem Altpapier ziehen die kationischen

oder amphoteren Nitroderivate nach der Zugabe zur wäßrigen Cellulosefaser-Suspension auf Grund ihrer guten Substantivität rasch auf die Faser auf und bleiben somit bei der Papierblatt-Bildung nicht im Wasser zurück.

In weißgetöntem, fertigen Papier kann man die Weißtönung dadurch aufheben, daß man das Papier mit der wäßrigen Lösung des kationischen oder amphoteren Nitroderivates imprägniert. Dieser Effekt kann auch gezielt zur Erzeugung schrift- und bildmäßiger Aufzeichnungen oder Wasserzeichen verwendet werden.

Die Behandlung von Geweben, z.B. Baumwolle, kann nach bekannten Verfahren, z.B. dem Foulard- oder Ausziehverfahren in wäßrigem Medium erfolgen.

Die benötigte Menge von kationischer oder amphoterer Nitroverbindung beträgt etwa 1 - 5, vorzugsweise 1 - 3 Gew.-Teile, pro Gew.-Teil des optischen Aufhellers. Die Einsatzmenge bezogen auf trockenes Substrat liegt vorzugsweise zwischen 0,05 und 0,3 Gew.-%.

Die Applikation erfolgt beispielsweise bei pH-Werten von 3 bis 9,5 vorzugsweise 4 bis 8,5, bei Temperaturen von 10 - 100 °C, vorzugsweise 18 - 60 °C, und insbesondere bei Raumtemperatur.

Nitrogruppenfreie Fluoreszenzlöscher sind aus der DE-A-1 912 647 und der DE-A- 2 448 293 bekannt Gegenüber diesen Verbindungen zeigen die Verbindungen des erfindungsgemäßen Verfahrens eine gesteigerte und vollständigere Fluoreszenz-löschende Wirkung. Die Überlegenheit zeigt sich besonders im neutralen bis schwach alkalischen Anwendungsbereich von pH 7 bis 9,5. Dadurch, daß man mit geringeren Einsatzmengen an Fluoreszenzlöscher des erfindungsgemäßen Verfahrens bereits praktisch eine vollständige Aufhebung des durch anionische Weißtöner erzeugten Weißeffektes erreicht, tritt bei einer gewünschten nochmaligen Weißtönung des Cellulose-Substrates eine wesentlich geringere Beeinträchtigung des neu applizierten anionischen Weißtöners ein als nach der Vorbehandlung mit Fluoreszenzlöschern des Standes der Technik, so daß man zur Erzeugung eines bestimmten Weißeffektes mit geringeren Weißtönermengen auskommt.

Die erfindungsgemäß zu verwendenden Nitroverbindungen zeichnen sich außerdem durch eine gute Beständigkeit gegen Hydrolyse- und Lichteinflüsse und eine hohe Wasserlöslichkeit aus. Ihre wäßrigen Einstellungen sind lagerstabil, insbesondere in Gegenwart von Carbonamid- oder Carbonimidgruppen enthaltenden wasserlöslichen Verbindungen wie Harnstoff, Caprolactam, Pyrrolidon oder Cyanamiden der Formel

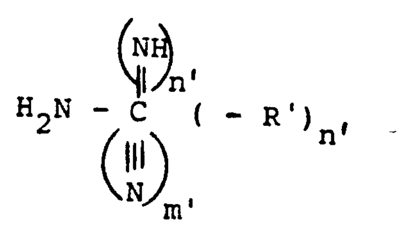

(V)

worin

R' = -NH-C = N, $-NH-CO-NH_2$ oder $-NH-CO-NH-CH_3$ und

n' = 0 und m' = 1 oder

n' = 1 und m' = 0 bedeutet,

wie sie in der DE-A- 34 25 813.2 beschrieben sind, beispielsweise Dicyandiamid.

Gegenstand der Erfindung sind außerdem Verbindungen

a) die der Formel I entsprechen mit der Einschränkung, daß 2 verschieden von $-CH_2-$ ist, wenn $x^2$, $Y^2$ und G für eine Einfachbindung, r für 2 und W für Phenylen oder Cyclohexylen stehen,

b) die der Formel (II) entsprechen,

c) die der Formel (III) entsprechen und

d) die der Formel (IV) entsprechen

sowie Verfahren zu ihrer Herstellung.

Unter den vorstehend unter a) genannten Verbindungen seien als technisch besonders wertvoll solche hervorgehoben, worin die r-fach vorhandenen Ketten gleich sind, und worin

$\overset{\oplus}{K}{}^1$ für $A-X^1-Y^1-Z^1-\overset{\oplus}{E}-$ oder 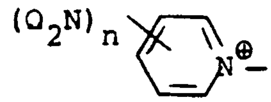

A für $A^1$ = Phenyl, Biphenyl oder Naphthyl, die außer durch Nitro auch durch $C_1$-$C_4$-Alkyl, $C_1$-$C_4$-Alkoxy, Chlor, Brom, Cyano, Carbamoyl oder Hydroxy substituiert sein können, oder Thienyl,

$X^1$ für $X^{1}$ = -CO-, $-CH_2-CO-$, $SO_2$- oder eine Einfachbindung,

$Y^1$ für $Y^{1'}$ = -N(R)- oder eine Einfachbindung,

$Y^2$ für $Y^{2'}$ = -N(R)- oder eine Einfachbindung,

$\overset{\oplus}{E}$ für $\overset{\oplus}{E}{}'$ = $-\overset{\oplus}{N}(R^1R^2R^2)-$ oder 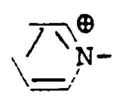 ,

W für W' = r-bindiger Benzol-, Naphthalin-, Cyclohexyl-, Thiophen(2,5)- oder s-Triazin(2,4,6)-Rest oder Rest der Formeln

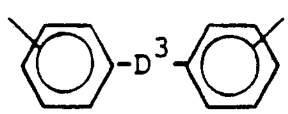 oder 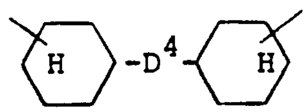 ,

mit

$D^3$ = $-CH_2-$, $-CH(CH_3)-$, $-C(CH_3)_2-$, -CH=CH-, $-CH(C_6H_5)-$, $-CH(C_6H_4-)-$, -CO-NH-, -NH-CO-NH-, -N($C_1$-$C_2$-Alkyl)-, $-N(C_6H_5)-$, -O-, -S-, $-SO_2-$, 1,1-Cyclohexylen, p-Phenylen, Thiophen(2,5), 1,3,4-Oxadiazol-(2,5), 1 ,3,4-Thiadiazol(2,5) , Oxazol(2,5) , Thiazol(2,5) eine direkte Bindung oder

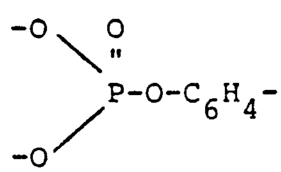

$D^4$ = $-CH_2-$, $-CH(CH_3)-$, $-C(CH_3)_2-$ $-CH(C_6H_{11})-$, $-CH(C_6H_{10}-)-$, -CO-NH-, -NH-CO-NH-, -O-, 1,1-Cyclohexylen oder eine direkte Bindung, wobei die unter W', $D^3$ und $D^4$ genannten Ringe durch $CH_3$, $CH_3O$, Cl oder $NO_2$ substituiert sein können,

stehen und worin die übrigen Symbole die oben angegebene Bedeutung besitzen.

Von besonderem technischen Wert sind Verbindungen der Formel

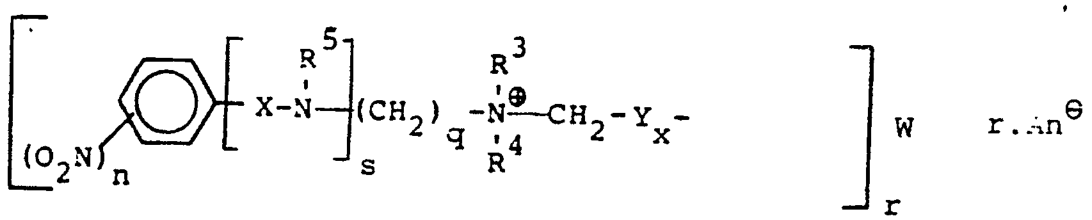

(VI)

und

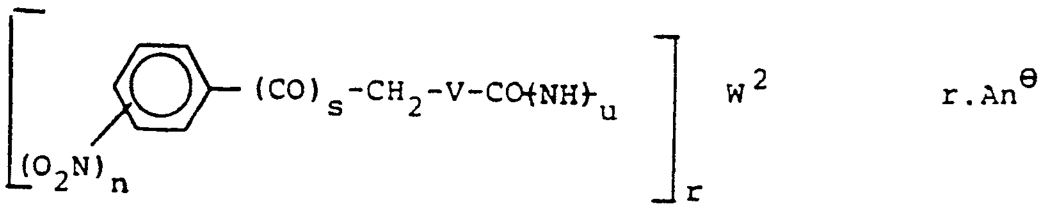

(VII)

worin

| | |
|---|---|
| X | für -CO- oder $-SO_2-$, |
| Y | für -CO-NH-, -CO-NH-CO-NH- oder -CO-, |
| $R^3$ und | $R^4$ für $C_1$-$C_4$-Alkyl , das durch Hydroxy substituiert sein kann, und |
| $R^4$ | auch für einen Phenyl-$C_1$-$C_2$-alkyl- oder Benzoylmethylrest, der durch Nitro substituiert sein kann, stehen, oder |
| $R^3$ und | $R^4$ ringgeschlossen sind und dann zusammen mit dem Stickstoffatom einen gegebenenfalls durch oder 2 Methylgruppen substituierten Pyrrolidin-, Piperidin-, Morpholin- oder Piperazin-Ring bilden, |
| $R^5$ | für Wasserstoff oder zusammen mit $R^3$ auch für die restlichen Glieder eines Piperazinringes, |
| q | für 2 oder 3 und, wenn s = 0 ist, auch für 1, |
| u | für 0 oder 1, |
| $W^2$ | für W oder im Falle u = 1 auch für eine Einfachbindung, |
| s | für 0 oder 1, |
| x | für 0 oder 1, |

wobei x für 1 steht, wenn X für CO und W für Phenylen oder Cyclohexylen stehen,

V für 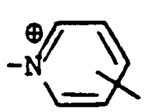 oder 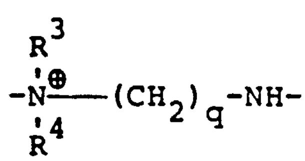 stehen, und

$An^{\ominus}$, W, n und r die in Formel (I) angegebene Bedeutung haben.

Eine weitere bevorzugte Gruppe von Nitroderivaten sind Verbindungen der Formel

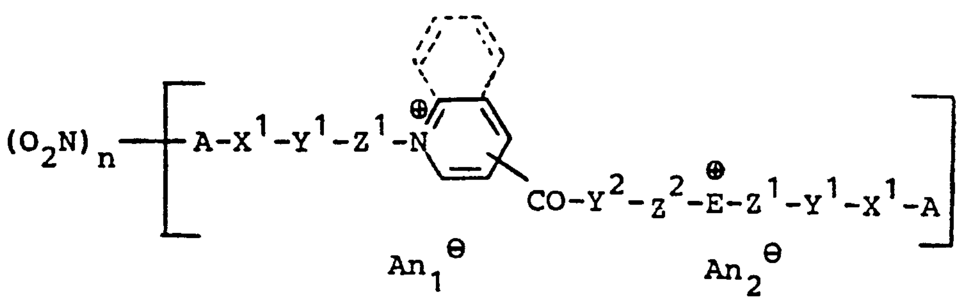 (VIII)

worin

n,A,$X^1$ ,$Y^1$ ,$Y^2$ ,$Z^1$ ,$Z^2$ und $\overset{\oplus}{E}$ die oben angegebene Bedeutung haben und

$An_1^{\ominus}$ und $An_2^{\ominus}$ für gleiche oder verschiedene farblose Anionen stehen.

Beispiele für die Reste $(O_2N)_nA$ sind 4-Nitrophenyl, 3-Nitrophenyl, 3,5-Dinitrophenyl, 2-Nitrophenyl, 3-Nitro-4-methylphenyl, 3-Nitro-4-methoxyphenyl, 3-Nitro-4-chlorphenyl, 3-Methyl-4-nitrophenyl, 4'-Nitro-biphenylyl, 2',4'-Dinitro-biphenylyl, 3-Nitro-5-dimethylaminophe nyl, 5-Nitro-naphthyl-(1), 5-Nitro-naphthyl-(2), 5,7-Di-nitro-naphthyl-(1), 5-Nitrofuryl-(2), 5-Nitro-thienyl-(2), 6-Nitro-benzo[ b ]furyl-(2), 5-Nitro-indolyl-(3), 5-Nitro-thiazolyl-(2), 6-Nitro-benzthiazolyl-(2), 5-Nitro-1,3,4-thiadiazolyl-(2) und 6-Nitro-chinolyl-(4).

Wenn R in $Y^1$ für CO oder $SO_2$ steht, kann es mit A in der o-Stellung zu $X^1$ oder - im Fall eines kondensierten Ringsystems - mit der peri-Stellung verknüpft sein und dann zusammen mit $(O_2N)n$-A-$X^1$-N- z.B. die Reste

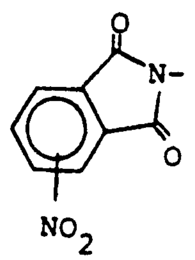

oder

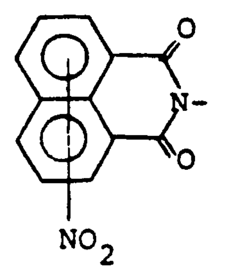

bilden.

Die Verbindungen der Formel (I) sind nach verschiedenen Verfahren zugänglich

Verbindungen der Formel

$$(O_2N)_nA-X^1-Y^1-Z^1-E \qquad (IX)$$

worin

n,A,$X_1$,$Y^1$ und $Z^1$ die oben angegebene Bedeutung besitzen und

E für die noch nicht kationische Vorstufe von $E^{\oplus}$ steht, können im Molverhältnis r:1 mit Verbindungen der Formel

$$W(-G-X^2-Y^2-Z^2-L)_r \qquad (X)$$

worin

W, G, $X^2$, $Y^2$, $Z^2$, L und r die oben angegebene Bedeutung besitzen,

umgesetzt werden.

Die Umsetzung wird zweckmäßig in einem inerten organischen Lösungsmittel im Temperaturbereich von 40 - 160°C durchgeführt, wobei eine Reaktionszeit von etwa 2 - 50 Stunden erforderlich ist.

Die Reaktionsprodukte (I) fallen in der Regel als kristalline Substanzen an. Werden technisch anwendbare Lösungen oder Dispersionen von (I) gewünscht, kann die Umsetzung vorteilhaft in dem betreffenden Lösungsmittel durchgeführt werden, so daß die Isolierung als Substanz entfällt.

Geeignete inerte Lösungsmittel sind beispielsweise gegebenenfalls durch Chloratome, eine Nitrogruppe oder Niederalkoxy substituierte Kohlenwasserstoffe wie Benzol, Toluol, Xylol, Mesitylen, Chlorbenzol, o-Dichlorbenzol, 1,2,4-Trichlorbenzol, Chlortoluole, Dichlortoluole, Nitrobenzol, Anisol; Dichlormethan, Chloroform, Tetrachlormethan, 1,2-Dichlorethan, 1,2-Dichlorpropan, 1,1,2,2-Tetrachlorethan, 2-Nitropropan; Ether wie Ethylenglykoldimethylether, Dioxan, Tetrahydrofuran; dipolar aprotische Lösungsmittel wie Dimethylformamid, N-Methylpyrrolidon und Dimethylsulfoxid; Ester wie Caprolacton; Nitrile wie Acetonitril; polare OH-Gruppen enthaltende Lösungsmittel wie Ethylenglykol, Diethylenglykol, Polyglykole, 2-Cyanoethanol und Wasser.

Geeignete Ausgangsverbindungen der Formel (X) sind beispielsweise 1,4-Bischlormethylbenzol, 1,4-Bisbrommethylbenzol, 2,4-Bischlormethyl-1,5-dimethylbenzol, 1,3,5-Trischlormethylbenzol, 4,4'-Bischlormethylbiphenyl, 1,4-Bischlormethylnaphthalin, 2,6-Bischlormethylnaphthalin, 4,4'-Bischlormethyl-benzophenon, -diphenylsulfon, -diphenylamin, -N-methyl-diphenylamin, -triphenylamin, -diphenylether, -diphenylsulfid, -diphenylmethan, -triphenylmethan, -diphenylpropan, Tris-(4-chlormethyl-phenyl)-methan, 2,5-Bischlormethylthiophen, -1,3,4-oxadiazol, -1 ,3,4-thiadiazol, N,N',N"-Trischlormethyl-s-triazin-2,4,6-trion, N,N',N"-Trismethylol-cyanursäure-p-toluolsulfonsäure-triester, 4,4'-Bischloracetamido-benzoesäureanilid, 4,4'-Bis-(chloracetamidomethyl)-biphenyl (US-PS 4 370 486), 4,4'-Bis-(chloracetamido)-triphenylmethan, -diphenylmethan oder -3,3'-dimethoxy-biphenyl, 4,4',4"-Tris-(chlor-acetamido)-triphenylmethan, 4,4',4"-Tris-(chloracetamido)-triphenylphosphonat, Bis-(4-chloracetamidocyclo hexyl)-methan, 2,2-Bis-(4-chloroacetamidocyclohexyl)-propan, 4,4'-Bis-chloracetyl-diphenylether, 4,4'-Bis-chloracetyldiphenylsulfid, 4,4'-Bis-(chloracetureido)-diphenylmethan und 4,4',4"-Tris-(chloracetureido)-triphenylmethan (aus 4,4',4"-Triaminotriphenylmethan und 3 Äquivalenten Chloracetylisocyanat).

Verbindungen der Formel (IX) , worin $Y^1$ für -O-, -S-, -N(R)- oder -N(R)NH- stehen, wobei R die oben angegebene Bedeutung besitzt, können durch Umsetzung von Nitroverbindungen der Formel

$$(O_2N-)_nA-X^1-Hal \qquad (XI)$$

worin
A und $x^1$ die oben angegebene Bedeutung besitzen und
Hal für ein als Anion abspaltbares Halogenatom wie Chlor, Brom oder Jod steht,
mit einer Verbindung der Formel

$$H-Y^1-Z^1-E \qquad (XII)$$

worin
$Y^1$, $Z^1$ und E die oben angegebene Bedeutung besitzen,
hergestellt werden.

Die Reaktion wird zweckmäßig in Wasser und/oder einem inerten organischen Lösungsmittel wie einem gegebenenfalls durch Halogenatome oder eine Nitrogruppe substituierten aromatischen oder aliphatischen Kohlenwasserstoff der oben angegebenen Art und/oder einem Acylgruppen übertragenden, zu intermediären N-Acylierungen fähigen Reaktionsmedium wie Pyridin oder Picolingemischen; Acetonitril, Dimethylformamid, Dimethylsulfoxid, im Temperaturbereich von etwa 0 - 110° C durchgeführt.

Als Protonenfänger kann das in (XII) vorhandene, an $Z^1$, $R^1$ und gegebenenfalls $R^2$ gebundene Stickstoffatom oder eine zusätzlich angewendete Base wie Natronlauge, Kalilauge, Soda, Kaliumcarbonatlösung, Alkalibicarbonatlösung, Magnesiumoxid, Triethylamin, Triisopropanolamin, Dimethylanilin, Pyridin oder Picolingemische dienen.

Geeignete Ausgangsverbindungen der Fomel (XI) sind beispielsweise 4-Nitrobenzolsulfochlorid, 4-Nitrobenzoylchlorid, 3-Nitrobenzolsulfochlorid, 3-Nitrobenzoylchlorid, 3,5-Dinitrobenzoylchlorid, 4-Chlor-3-nitrobenzoylchlorid, 4-Chlor-3-nitrobenzolsulfochlorid, 3,5-Dinitro-4-hydroxybenzoyl-chlorid, 3-Carbamoyl-5-nitrobenzoyl-chlorid, 3-Methyl-4-nitrobenzoylchlorid, 4-Methyl-3-nitrobenzoylchlorid, 4-Methyl-3-nitrobenzolsulfochlorid, 4-Methoxy-3-nitrobenzoylchlorid, 2-Nitrobenzolsulfochlorid, 2,4-Dinitrobenzolsulfochlorid, 4-p-Nitrophenylbenzoylchlorid, 4-p-Nitrophenylbenzolsulfochlorid, p-(2,4-Dinitrophenyl)-benzoylchlorid, p-(2,4-Dinitrophenyl)-benzolsulfochlorid, 5-Nitronaphthalin-1-sulfochlorid, 5-Nitronaphthalin-2-sulfochlorid, 4-Nitro-phenylacetylchlorid, 2,4-Dinitrophenoxyacetylchlorid, 2,4-Dinitrophenylacetylchlorid, 4-Nitro-zimtsäurechlorid, 5-Nitro-thienylchlorid, 1,2-Dimethyl-5-nitroindol-3-carbonsäurechlorid.

Im Falle, daß in Verbindung (I) $Y^1$ = -N(R)- bedeutet und R eine mit einer o- oder peri-Stellung zur A-$X^1$-Bindung mit A verknüpfte -CO- oder $-SO_2$-Gruppe darstellt, geht man statt von (XI) zweckmäßig von dem Anhydrid der Formel

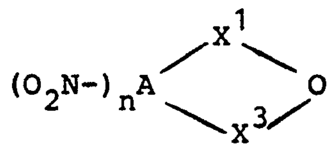

(XIII)

worin

$X^3$ für -CO- oder $SO_2$- steht und

$X^1$, A und n die oben angegebene Bedeutung haben,

aus und setzt dieses mit (XII) um. Man arbeitet in einem der oben empfohlenen Lösungsmittel bei Reaktionstemperaturen von etwa 50 - 170 °C.

Beispiele für (XIII) sind 3-Nitrophthalsäureanhydrid, 4-Nitrophthalsäureanhydrid, 4-Nitronaphthalsäureanhydrid und 4-Nitro-2-sulfo-benzoesäureanhydrid.

Geeignete Ausgangsverbindungen der Formel (XII) sind beispielsweise 1-Amino-3-dimethylamino-n-propan, 1-Amino-3-diethylamino-n-propan, 1-Amino-3-methylamino-n-propan, 1-Amino-3-cyclohexylamino-n-propan, 1-Amino-2-diethylaminoethan, 1-Amino-2,2-dimethyl-3-dimethylamino-n-propan, N,N',N"-Trimethyldiethylentriamin, Bis(3-amino-n-propyl)-methylamin, Bis-(3-amino-n-propyl)-amin, Diethylentriamin, 4-Amino-1-diethylamino-n-pentan, Glycin-cholinester, Cholin-ß-aminoethylether, 2-/ (3-Amino-n-propyl)-methylamino / -ethanol, 2-(2-Amino-ethylamino)-ethanol, 2-Dimethylaminoethanol, 2-(2-Dime-thylaminoethoxy)-ethanol, 2-Diethylaminoethanol, 2-Dime-thylamino-2-propanol, 1-Diethylamino-2-propanol, Bis(2-hydroxy-n-propyl)-methylamin, 3-Dimethylamino-1-propanol, 2-Diethylaminoethanthiol, N-(3-Amino-n-propyl)-pyrrolidin, N-Methyl-N'-(2-aminoethyl)-piperazin, N-Methylpiperazin, N-ß-Hydroxyethyl-piperazin, 1-(3-Amino-n-propyl)-1,2,4-triazol, 1-(3-Amino-n-propyl)-1,2,3-triazol, 1-(3-Amino-n-propyl)-imidazol, 4-Amino-N,N-di-methylbenzylamin, 4-Aminomethyl-N,N-dimethyl-benzylamin und 4-Aminomethylpyridin.

Eine besonders rationelle Verfahrensvariante besteht darin, daß man die aus (XI) und (XII) oder (XII) und (XIII) hergestellten Verbindungen der Formel (IX) ohne Zwischenisolierung im gleichen Reaktionsmedium nach der Überführung in die freie Base mit (X) umsetzt.

Ein weiteres Verfahren zur Herstellung von Zwischenverbindungen der Formel (IX), welches sich besonders für solche Verbindungen eignet, in denen

x' -CO-,

Y' eine direkte Bindung,

Z' eine -CH(Q)-$CH_2$-Gruppe ,

Q Wasserstoff oder Methyl bedeuten,

ist dadurch gekennzeichnet, daß man eine Nitroverbindung der Formel

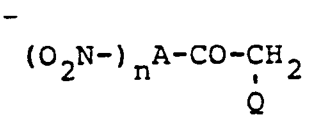

(XIV)

worin n, A und Q die oben angegebene Bedeutung besitzen,

mit Formaldehyd und einem gegebenenfalls cyclischen, sekundären Amin der Formel

H - E (XV)

worin E die oben angegebene Bedeutung besitzt,

unter den üblichen Bedingungen der "Mannich-Reaktion" umsetzt.

Man arbeitet zweckmäßig in Wasser und/oder in einem organischen Lösungsmittel der oben angegebenen Art im Temperaturbereich von 20 - 130 °C in Gegenwart eines Äquivalents einer starken Säure wie Salzsäure oder p-Toluolsulfonsäure.

Typische Ausgangsverbindungen der Formel (XIV) sind beispielsweise 3-Nitroacetophenon, 3-Nitropropiophenon, 4-Nitroacetophenon, 4-p-Nitrophenyl-acetophenon, 3,5-Di-nitro-acetophenon, p-2,4-Dinitrophenyl-acetophenon, 1-Acetyl-5-nitro-naphthalin, 2-Acetyl-5-nitro-furan, 2-Acetyl-5-nitro-thiophen, 2-Acetyl-5-nitro-1,3,4-thiadiazol, 2-Acetyl-5-nitro-thiazol, 2-Acetyl-6-nitro-benzo-/ b /furan, 2-Acetyl-5-nitrobenzo/ b /thiophen, 2-Acetyl-5-nitro-benzthiazol, 2-Acetyl-6-nitro-benzthiazol.

Geeignete sekundäre Amine der Formel (XV) sind beispielsweise Dimethylamin, Diethylamin, Di-n-butylamin, 2-Methylaminoethanol, Diethanolamin, Pyrrolidin, Piperidin, Morpholin, N-Methyl-piperazin, N-ß-Hydroxyethyl-piperazin, 2-Methylindolin, Tetrahydrochinolin, N-Methylanilin, 1,2,3- oder 1,2,4-Triazol oder Imidazol.

Auch hier besteht eine rationelle Verfahrensvariante darin, die so gewonnene Verbindung der Formel (IX) ohne Zwischenisolierung nach der Überführung in die Base mit (X) weiter zu (I) umzusetzen.

Ein weiteres Verfahren zur Herstellung von Zwischenverbindungen der Formel (IX), welches sich besonders für solche Verbindungen eignet, in denen

$Y^1$ eine direkte Bindung,

$Z^1$ eine $-CH_2-CH(Q)-V^1$-Gruppe,

$V^1$ eine direkte Bindung oder eine $-O-CH_2-CH_2$-Gruppe,

Q Wasserstoff oder Methyl bedeuten,

ist dadurch gekennzeichnet, daß man an eine Nitroverbindung der Formel

$$(O_2N-)_n A-X^1-CH=\underset{Q}{\underset{|}{C}}H \qquad (XVI)$$

worin n, A, $X^1$ und Q die oben angegebene Bedeutung besitzen,

eine Verbindung

$$H - V^1 - E \qquad (XVII)$$

worin $V^1$ und E die oben angegebene Bedeutung besitzen,

addiert.

Die Addition wird beispielsweise nach den in DE-A-25 34 180 und DE-A- 27 00 996 beschriebenen Methoden durchgeführt.

Ein weiteres Verfahren zur Herstellung von Verbindungen der Formel (I) besteht darin, daß man Verbindungen der allgemeinen Formel worin

$$\underset{(An^{\ominus})_x}{(\overset{\oplus}{K}{}^1-Z^2-Y^2-X^2-G-)_r W} \qquad (XVIII)$$

$\overset{\oplus}{K}{}^1$, $X^2$, $Y^2$, $Z^2$, G, W, $An^{\ominus}$, x und r die oben angegebene Bedeutung haben,

mit nitrierend wirkenden Mitteln, insbesondere mit Salpetersäure oder Salpetersäure-Schwefelsäure-Gemischen, umsetzt.

Je nach Substituenten in A und W und Nitrierbedingungen treten 1 - 3 Nitrogruppen in $\overset{\oplus}{K}{}^1$ und oder W ein. Man arbeitet zweckmäßig im Temperaturbereich von -5 bis 90° C.

In analoger Weise lassen sich auch die Zwischenverbindungen der Formel (IX) durch Nitrierung nitrogruppenfreier Vorstufen herstellen.

Ein weiteres Verfahren zur Herstellung von Verbindungen der Formel (I) besteht darin, daß man Verbindungen der Formel

$$(E-Z^2-Y^2-X^2-G-)_r W \qquad (XIX)$$

worin

E, $X^2$, $Y^2$, $Z^2$, G, W und r die oben angegebene Bedeutung besitzen,

im Molverhältnis 1:r mit Verbindungen der Formel

$$(O_2N-)_nA-X^1-Y^1-Z^1-L \qquad (XX)$$

worin
n,A,$X^1$,$Y^1$,$Z^1$ und L die oben angegebene Bedeutung besitzen,
umsetzt.

Geeignete Lösungsmittel und Reaktionsbedingungen sind die gleichen wie für die Umsetzung von (IX) und (X).

Ein Verfahren zur Herstellung von Verbindungen der Formel (VIII) ist dadurch gekennzeichnet, daß man Verbindungen der Formel

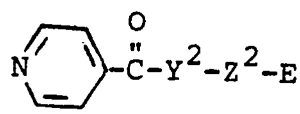

(XXI)

worin
$y^2$,$Z^2$ und E die oben angegebene Bedeutung haben, im Molverhältnis 1:2
mit Verbindungen der Formel (XX) umsetzt.

Geeignete Lösungsmittel und Reaktionsbedingungen sind die gleichen wie für die Umsetzung von (IX) mit (X).

N -Î@ O -Verbindungen werden dadurch hergestellt, daß man tertiäre acyclische oder cyclische ( >N-)- oder ( ≥ N)-Verbindungen mit Wasserstoffperoxid oder Peressigsäure behandelt. Man arbeitet zweckmäßig in einem inerten organischen Lösungsmittel wie Methylethylketon oder Eisessig im Temperaturbereich von 10 - 60° C, vorzugsweise 20 - 40° C.

Verbindungen der Formel (II) können beispielsweise dadurch hergestellt werden, daß man Cyanurchlorid in beliebiger Reihenfolge mit 1 Äquivalent einer Verbindung der Formel

$$(O_2N)_nA-X^1-Y^1-Z^1-Y'-H \qquad (XXII)$$

worin
n, A, $X^1$, $Y^1$, $Z^1$ und Y' die gleich Bedeutung wie in Formel (II) besitzen,
und 2 Äquivalenten einer Verbindung der Formel

$$H-Y^2-Z^2-\overset{\oplus}{E}{}^1\ (An^{\ominus})_x \qquad (XXIII)$$

worin
$Y^2$, $Z^2$, $E^{\oplus 1}$, $An^{\ominus}$ und x die gleiche Bedeutung wie in Formel (II) besitzen,

umsetzt.

Die Umsetzung kann zweckmäßig in wäßrigem Medium, bevorzugt in einer Mischung aus einem niederen aliphatischen Keton wie Methylethylketon und Wasser in Gegenwart von Kalium- oder Natriumbicarbonat als Säurefänger in erster Reaktionsstufe bei pH 4 - 4,5 und 10 - 20° C, in zweiter Reaktionsstufe pH 4 - 6 und 40° C und in dritter Reaktionsstufe (Ersatz des 3. Chloratoms des Cyanurchlorids) bei pH 6 - 7 und 60 - 100° C, beispielsweise unter Rückflußsieden durchgeführt werden.

In anderen Fällen setzt man zunächst eine Verbindung der Formel

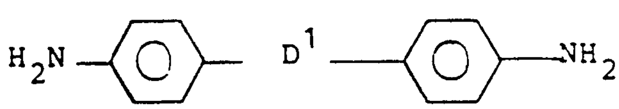

(XXIV)

oder

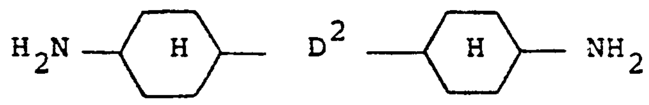

(XXV)

worin $D^1$ und $D^2$ die gleiche Bedeutung wie in Formel (I) besitzen,

mit 2 Äquivalenten Cyanurchlorid und dann nacheinander mit 2 Äquivalenten (XXII) und 2 Äquivalenten (XXIII) um.

Auch hier wählt man zweckmäßig die oben angegebenen Reaktionsbedingungen.

Zwischenprodukte XIX der speziellen Formel

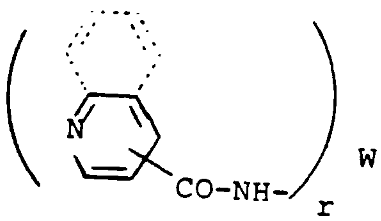

(XXVI)

worin

r und W die gleiche Bedeutung wie in Formel (I) besitzen,

werden vorteilhaft in der Weise hergestellt, daß man eine Pyridin- oder Chinclincarbonsäure im Molverhältnis r : 1 mit einem Di- oder Triisocyanat der Formel $(O=C=N)_r W$ in einem inerten Lösungsmittel wie Toluol oder Chlorbenzol im Temperaturbereich von 90-140 °C umsetzt, wobei Kohlendioxid abgespalten wird.

Herstellungsbeispiele

Beispiel 1

11,8 g 3,5-Dinitro-benzoesäure-3'-dimethylamino-n-propyl-amid und 3,5 g α,α'-Dichlor-p-xylol werden in 150 ml Dimethylformamid 48 Stunden unter Rühren auf 100 °C erhitzt und abgekühlt. Der kristalline Niederschlag wird abgesaugt, mit Isopropanol gewaschen und im Vakuumexsiccator getrocknet. Man erhält 13,2 g Verbindung der Formel

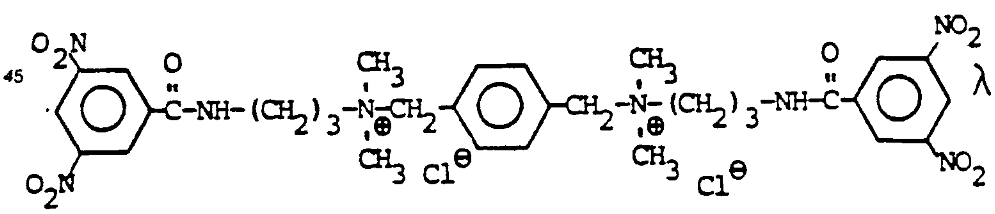

$\lambda_{max}$ = 259 nm

Das eingesetzte 3,5-Dinitro-benzoesäure-3'-dimethyl-amino-n-propylamid wird wie folgt hergestellt:

Zu einer Suspension von 46,1 g 3,5-Dinitro-benzoylchlorid in 400 ml wasserfreiem Acetonitril tropft man unter Kühlung und Rühren 20,4 g 3-Dimethylamino-n-propylamin hinzu, wobei man die Temperatur zwischen 20 und 30 °C hält. Anschließend läßt man die Mischung noch 4 h rühren. Der kristalline Niederschlag wird abgesaugt, mit Isopropanol gewaschen und bei 40 °C im Vakuum getrocknet.

Man erhält 62,1 g 3,5-Dinitro-benzoesäure-3'-dimethyl-amino-n-propylamid-hydrochlorid in Form farbloser Kri stalle (Zers. über 300 °C). Zur Überführung in die freie Base wird die Gesamtmenge des Hydrochlorids in 450 ml Wasser suspendiert, mit 20 g 45 %iger Natronlauge alkalisch gestellt und 1 h

verrührt. Der farblose kristalline Niederschlag wird abgesaugt, mit Wasser gewaschen und im Vakuumexsiccator getrocknet. Man erhält 54,5 g 3,5-Dinitro-benzoesäure-3'-dimethylamino-n-pro-pylamid (m/e = 296 (M+)).

In analoger Weise werden die folgenden Verbindungen hergestellt:

| Beispiel | |
|---|---|
| 2 | 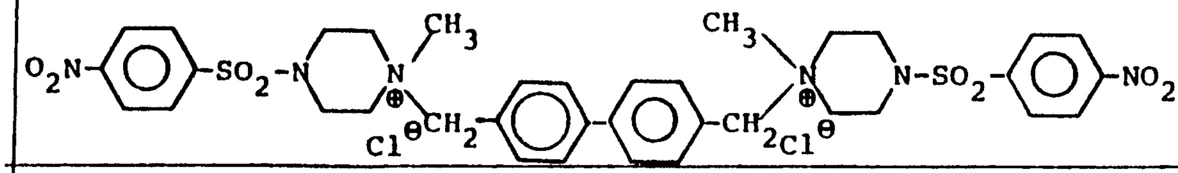 |
| 3 | 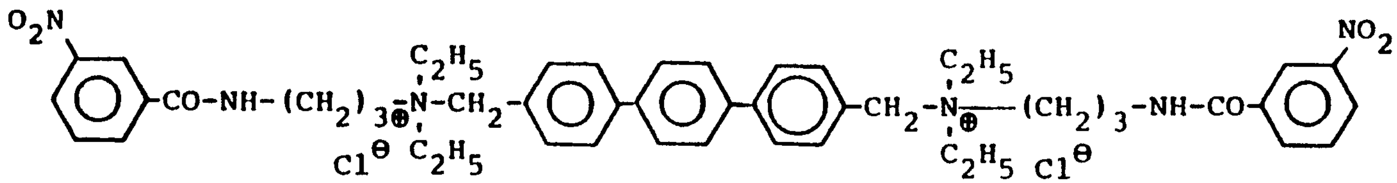 |
| 4 | 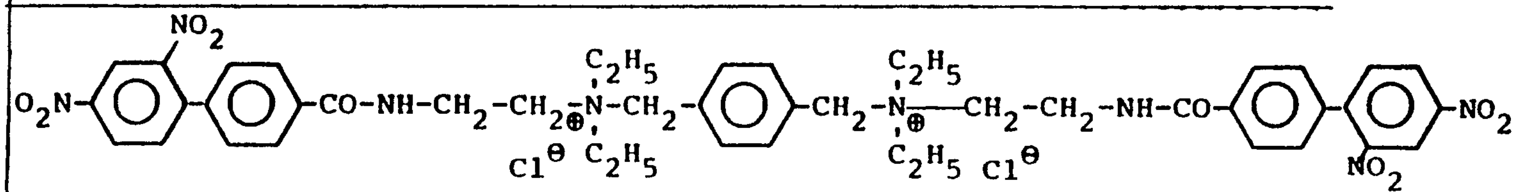 |
| 5 | 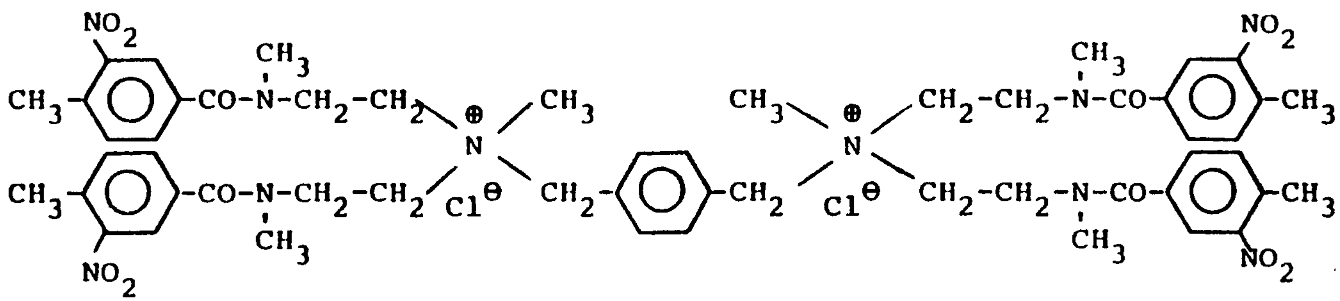 |

| Beispiel | |
|---|---|
| 6 | 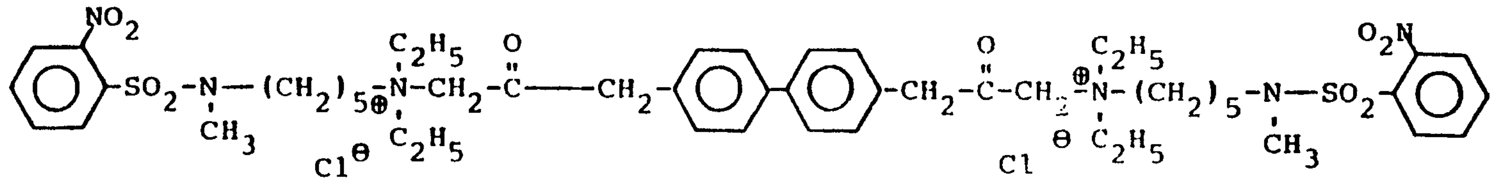 |
| 7 | 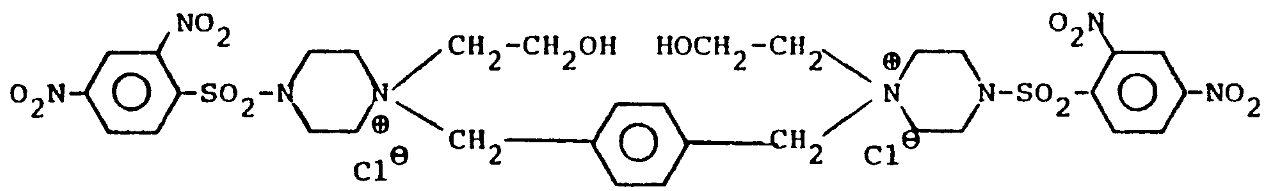 |
| 8 | 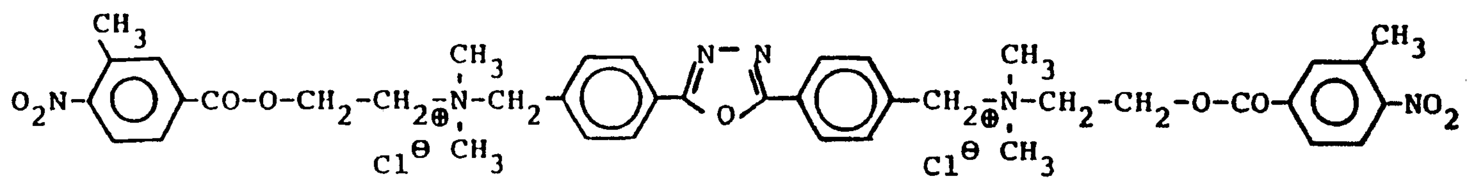 |
| 9 | 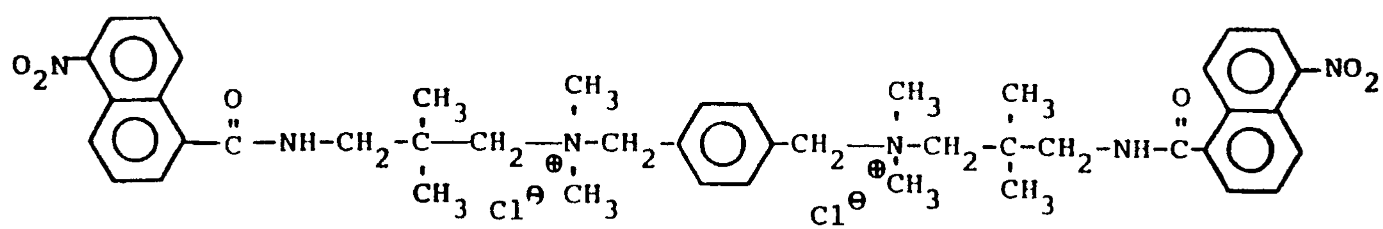 |

| Beispiel | |
|---|---|
| 10 | 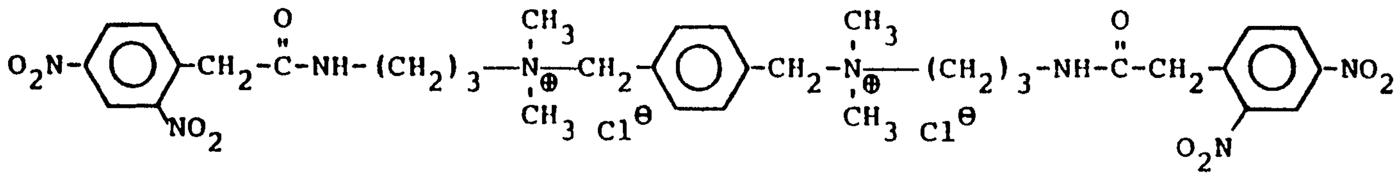 |
| 11 | 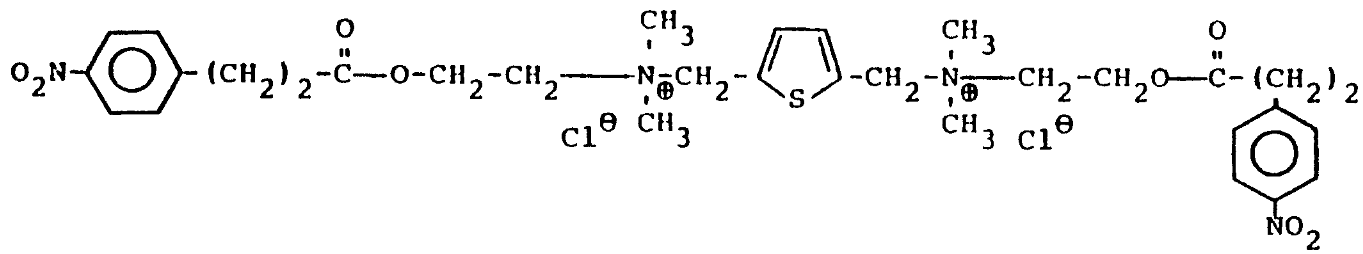 |
| 12 | 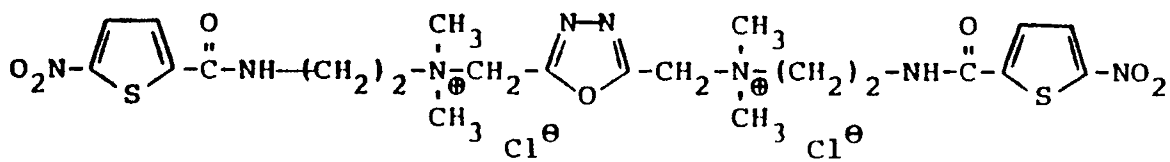 |
| 13 | 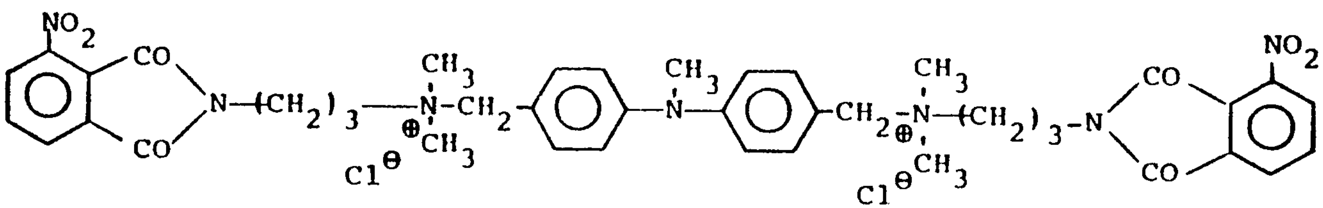 |

| Beispiel | |
|---|---|
| 14 | 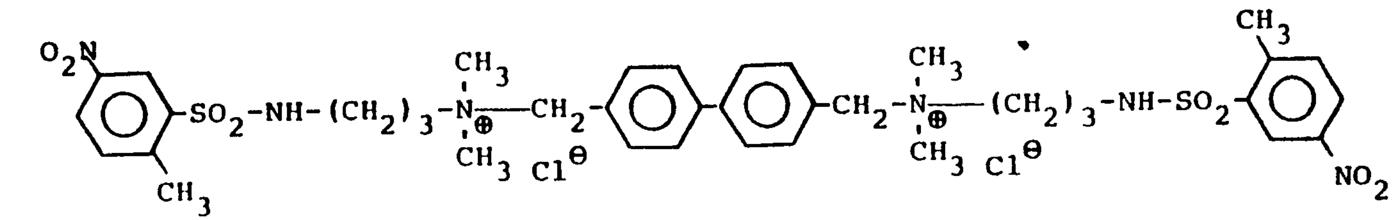 |
| 15 | 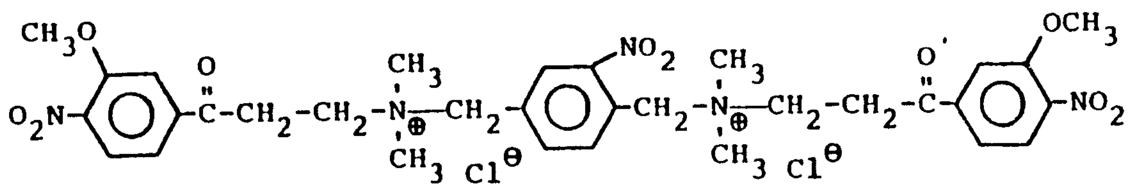 |
| 16 | 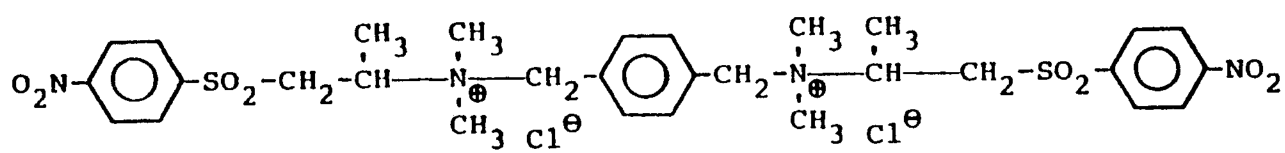 |
| 17 | 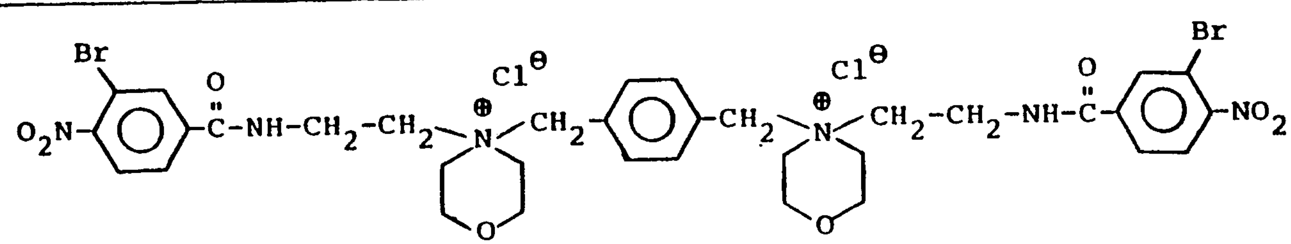 |

| Beispiel | |
|---|---|
| 18 | 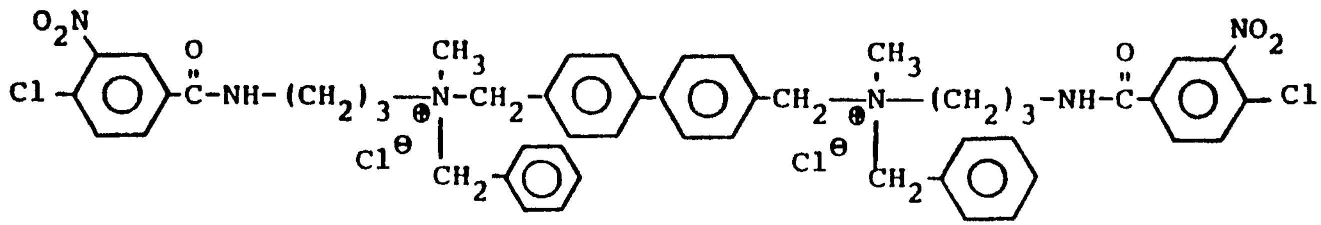 |
| 19 | 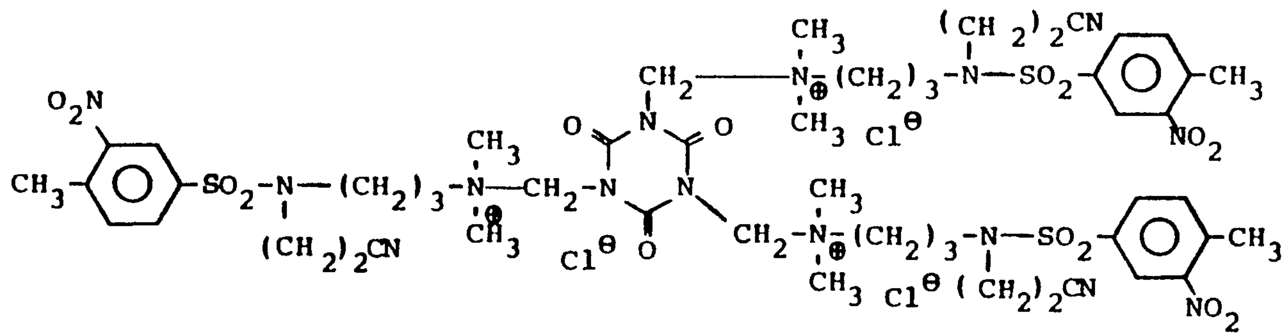 |
| 20 | 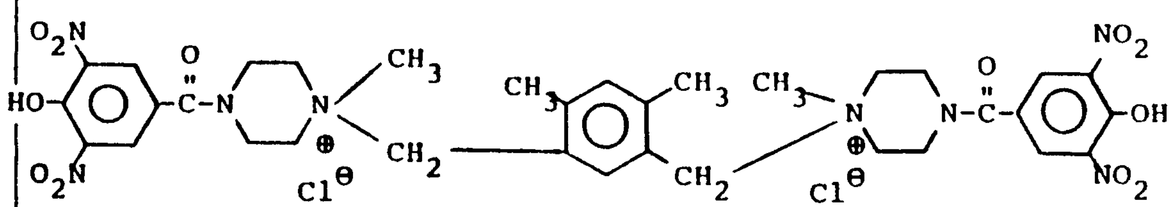 |

Beispiel

21

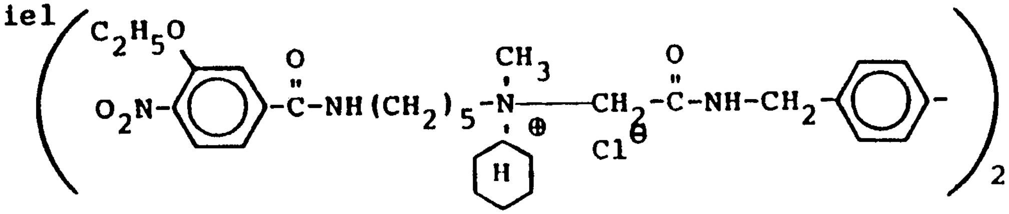

22

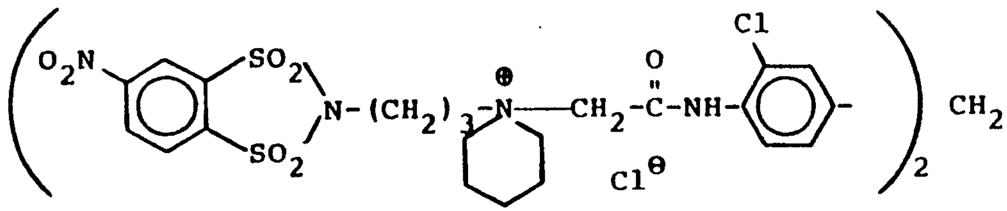

23

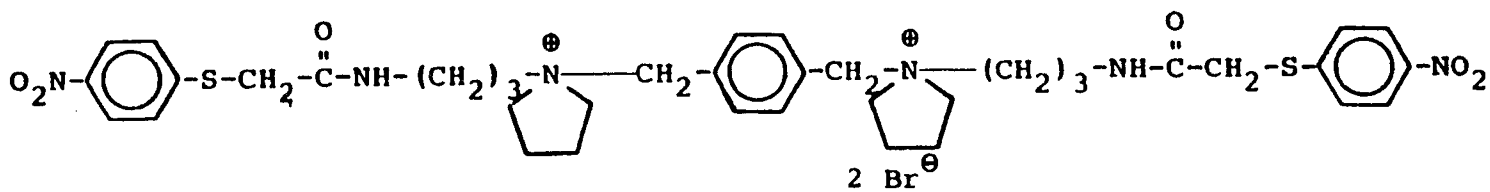

24

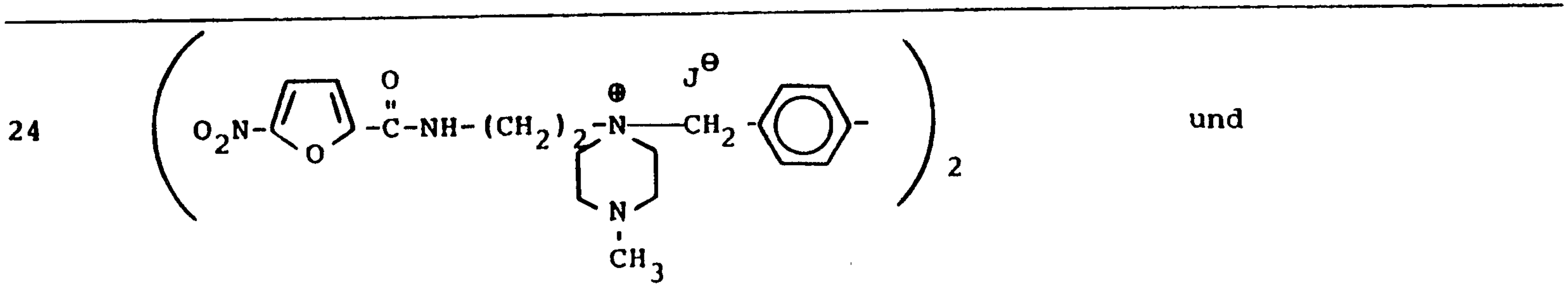

und

Beispiel 25

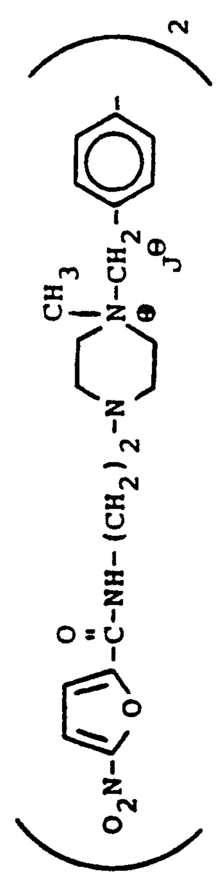

Beispiel 26

74,2 g 4-Nitrobenzoylchlorid werden in 400 ml Chlorbenzol suspendiert, unter Kühlung und Rühren bei 20 - 30 °C mit 40,8 g 3-Dimethylamino-n-propylamin versetzt und 10 h bei Raumtemperatur verrührt. Nachdem man sich dünnschichtchromatographisch von der Vollständigkeit der Umsetzung überzeugt hat, tropft man 72 g 30 %ige Natriummethylatlösung hinzu, fügt 35 g α ,α '-Dichlor-p-xylol hinzu, erwärmt die Mischung unter Abdestillieren von Methanol auf 100 °C und verrührt sie 20 h bei dieser Temperatur. Nach dem Ab-kühlen wird der kristalline Niederschlag abgesaugt, mit 500 ml Isopropanol gewaschen und bei 40 °C im Vakuum getrocknet. Man erhält 143,5 g farblose Kristalle, die etwa 20 g NaCl enthalten und im übrigen aus reiner Verbindung der Formel

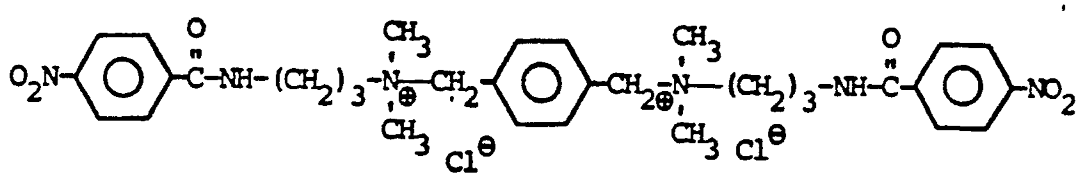

26

bestehen.

Die gleiche Verbindung 26 kann auch nach den Angaben der DE-A 3 313 965, Beispiel 46, hergestellt werden.

Beispiel 27

18,2 g 4,4'-Bis-(chloracetamidocyclohexyl)-methan und 25,6 g 4-Nitrobenzoesäure-3'-dimethylamino-n-propyl-amid werden in einer Mischung aus 75 g Octaethylenglykol (Homologen-Gemisch) sowie 7,5 g Caprolactam 6 h auf 90° C erhitzt und bei 20 - 30° C mit 120 g Aceton verdünnt. Der farblose kristalline Niederschlag wird abgesaugt, mit Aceton gewaschen und bei 40° C im Vakuum getrocknet. Man erhält 38,2 g Verbindung der Formel

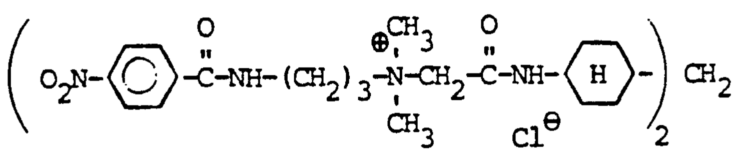

. $\lambda_{max}$ = 269 nm

27

Das eingesetzte 4,4'-Bis-(chloracetamidocyclohexyl)-methan wird wie folgt dargestellt:

105 g 4,4'-Diaminodicyclohexylmethan werden in 1000 g wasserfreiem Toluol gelöst, bei 20 bis 40° C tropfenweise mit 116,5 g Chloracetylchlorid versetzt, 8 h zum Rückflußsieden erwärmt und abgekühlt. Der farblose kristalline Niederschlag wird abgesaugt, mit Toluol gewaschen und bei 50° C im Vakuum getrocknet. Man erhält 173 g 4,4'-Bis-(chloracetamidocyclohexyl)-methan in Form farbloser Kristalle.

Beispiel 28

In eine Mischung von 20 ml Salpetersäure (D 1.5) und 50 ml konzentrierter Schwefelsäure werden bei 20° C unter Rühren 15,4 g α,α'-xylylen-bis-[(3-phenyl-acetamidopropyl)-dimethylammoniumchlorid] eingetragen. Die Mischung wird 4 h bei 20 bis 50° C verrührt, auf 300 g Eis ausgetragen und durch Zugabe von ca. 200 ml 20 %iger Kochsalzlösung ausgesalzen. Der kristalline Niederschlag wird abgesaugt und mit 12 %iger Kochsalzlösung gewaschen. Man erhält 32 g feuchte (Trockengehalt: 18 g, davon ca. 1,7 g NaCl) Verbindungen der Formel

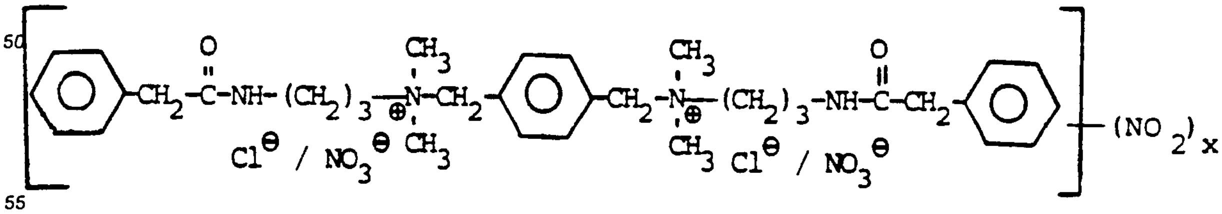

28

X = 3, 4, 5 und 6.

Beispiel 29

15,1 g 4-Nitro-benzoesäure-3'-dimethylamino-n-propyl-amid und 10,5 g 4,4'-Bis-(chloracetamidophenyl)-methan werden in 82 ml Wasser 4 h auf 90° C erhitzt und dann bei 50° C mit 19 g Harnstoff versetzt. Man erhält eine wäßrige Lösung mit einem Gehalt von 20,2 % an Verbindung der Formel

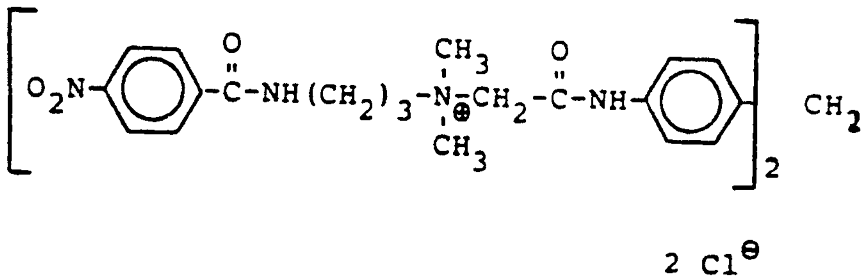

$\lambda_{max}$ 257 nm

29

und 15 % Harnstoff. Setzt man anstelle von Harnstoff 12,7 g Caprolactam und weitere 6,3 ml Wasser ein, so erhält man eine etwa gleich starke stabile wäBrige Lösung von 28 , die etwa 10 % Caprolactam enthält.

Ersetzt man 4,4'-Bis-(chloracetamidophenyl)-methan durch eine äquivalente Menge (10,8 g) 4,4'-Bis-(chloracetamidocyclohexyl)-methan, so erhält man in 82 ml Wasser bei 90° C (4 h) anschließend nach Zusatz von 19 g Harnstoff eine wäßrige Lösung mit einem Gehalt von etwa 20,4 % an Verbindung der Formel 27 und etwa 15 % Harnstoff ($\lambda_{max}$ = 269 nm).

Alle diese Lösungen sind vollkommen lagerstabil. Sie werden in dieser Form vorteilhaft zur Löschung der Fluoreszenz anionischer Weißtöner verwendet.

Wiederholt man die ursprüngliche Vorschrift mit 150 ml siedendem Acetonitril anstelle von Wasser, so erhält man Verbindung 29 in Form farbloser Kristalle in einer Ausbeute von 21,5 g.

4,4'-Bis-(chloracetamidophenyl)-methan ist folgendermaßen erhältlich:

79,2 g 4,4'-Diaminodiphenylmethan werden in 1100 ml Chloroform suspendiert, mit 153 g Triisopropanolamin und dann tropfenweise unter Rühren bei 20 bis 25° C unter Kühlung mit 90,4 g Chloracetylchlorid versetzt und 12 h verrührt. Der farblose kristalline Niederschlag wird abgesaugt, mit 400 ml Methanol, dann in 7 1 Wasser gewaschen und bei 50° C im Vakuum getrocknet. Ausbeute 128 g (91 % der Theorie); m/e = 350 ($M^{\bullet}$, 2Cl)

Analog 29 werden die folgenden Verbindungen der Formel

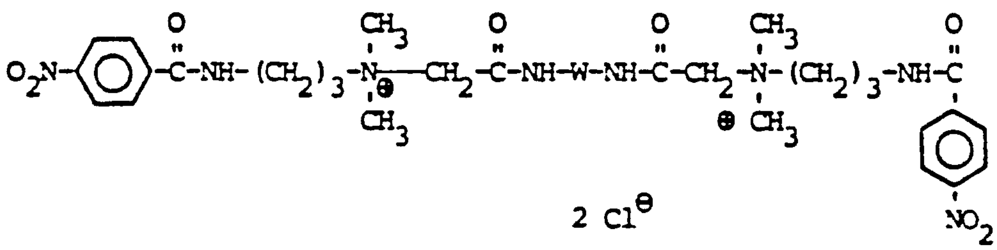

hergestellt.

| Beispiel | W |
| --- | --- |
| 30 | 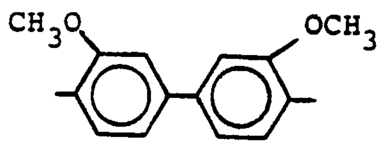 |
| 31 | 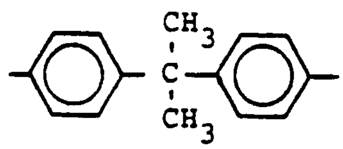 |
| 32 | 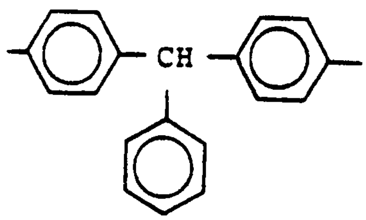 |
| 33 | 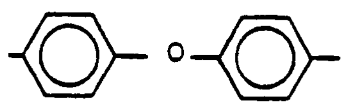 |
| 34 | 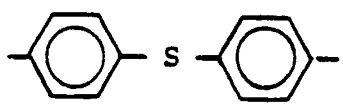 |
| 35 | 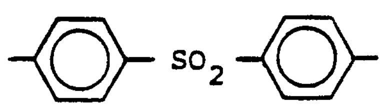 |
| 36 | 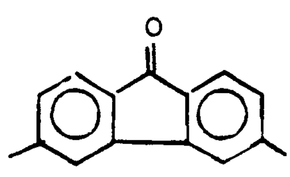 |

| Beispiel | W |
|---|---|
| 37 | 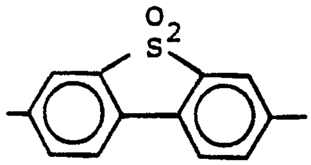 |
| 38 | 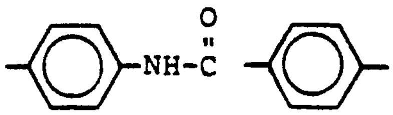 |
| 39 | 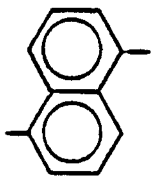 |
| 40 | 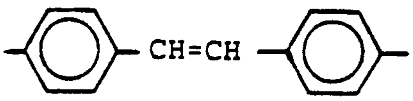 |
| 41 | 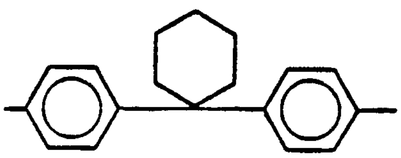 |
| 42 | 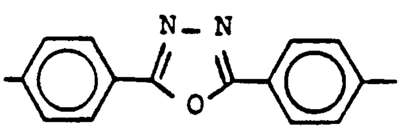 |

| Beispiel | W |
|---|---|
| 43 | 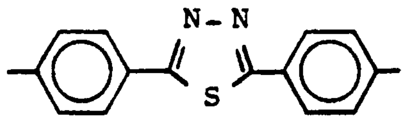 |
| 44 | 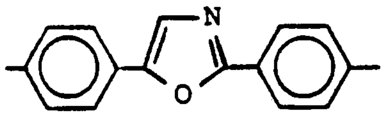 |
| 45 | 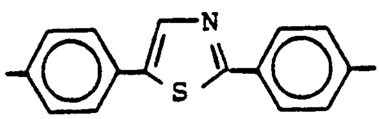 |
| 46 | 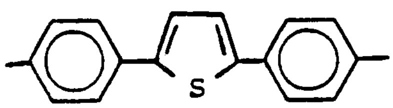 |
| 47 | 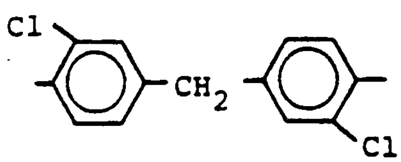 |
| 48 | 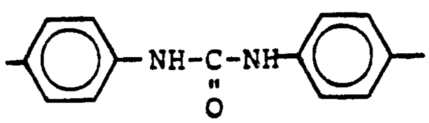 |

| Beispiel | W |
|---|---|
| 49 | 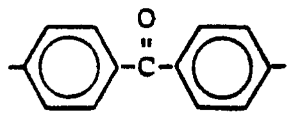 |
| 50 | 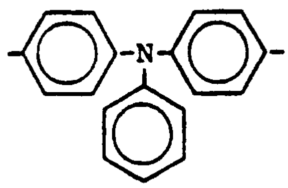 |
| 51 | 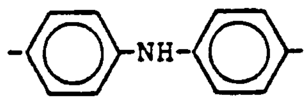 |
| 52 | 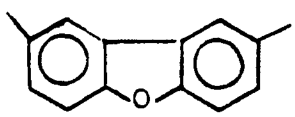 |
| 53 | 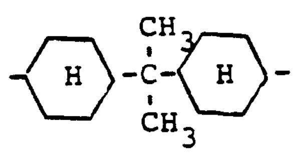 |
| 54 | 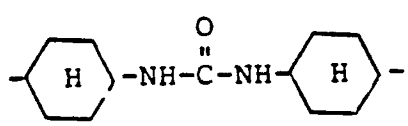 |

| Beispiel | W |
|---|---|
| 55 | 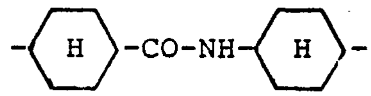 |
| 56 | 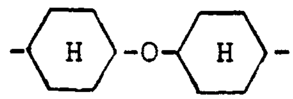 |
| 57 | 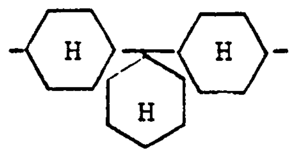 |
| 58 | 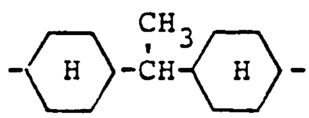 |
| 59 | 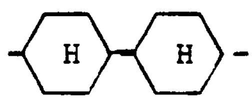 |
| 60 | 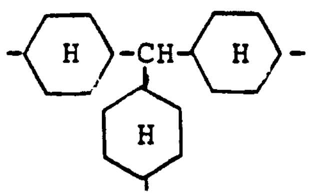 |
| 61 | 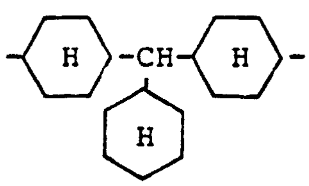 |

| Beispiel | W |
|---|---|
| 62 | 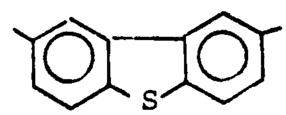 |
| 63 | 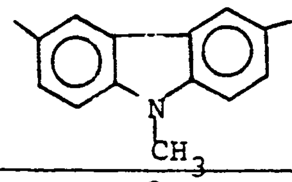 |
| 64 | 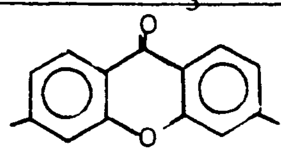 |
| 65 | 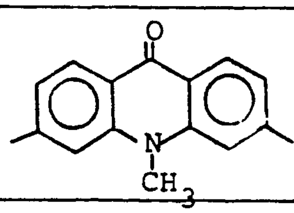 |
| 66 | 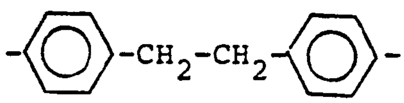 |
| 67 | 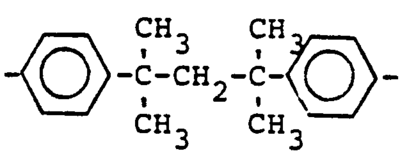 |
| 68 | 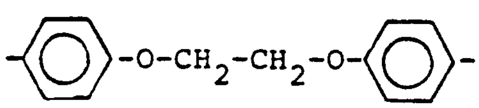 |

| Beispiel | W |
|---|---|
| 69 | 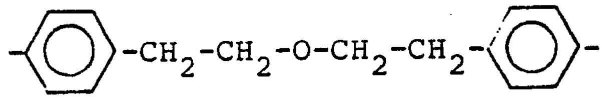 |
| 70 | 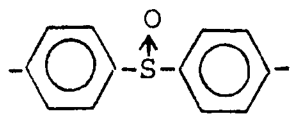 |
| 71 | 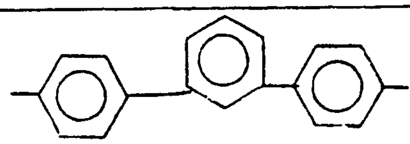 |
| 72 | 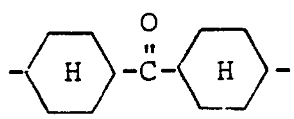 |
| 73 | 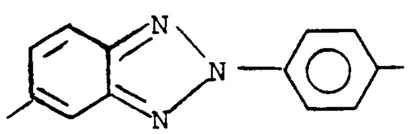 |
| 74 | 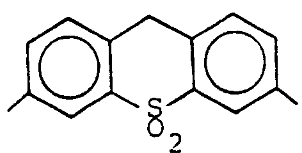 |
| 75 | 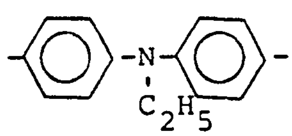 |

| Beispiel | W |
| --- | --- |
| 76 | 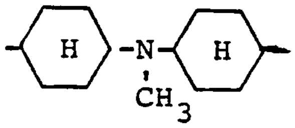 |
| 77 | 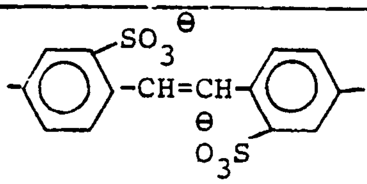 |
| 78 | 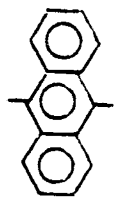 |
| 79 | 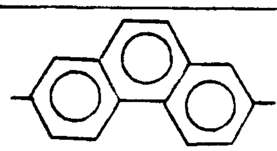 |
| 80 | 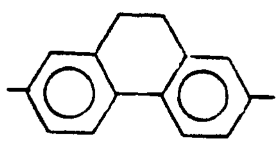 |
| 81 | 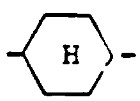 |
| 82 | 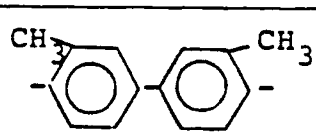 |

| Beispiel | W |
|---|---|
| 83 | 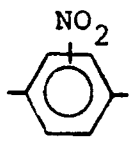 |
| 84 | 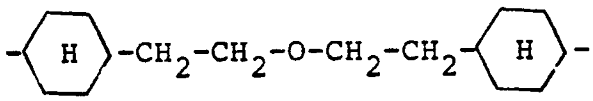 |
| 85 | 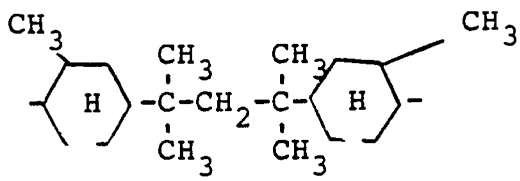 |
| 86 | 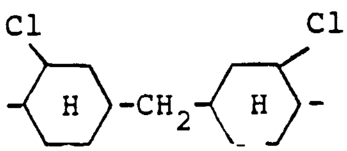 |

Ebenfalls in analoger Weise werden die folgenden, ähnlich wirksamen Verbindungen hergestellt:
Beispiel

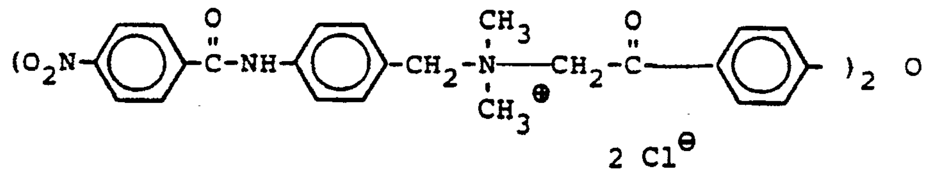

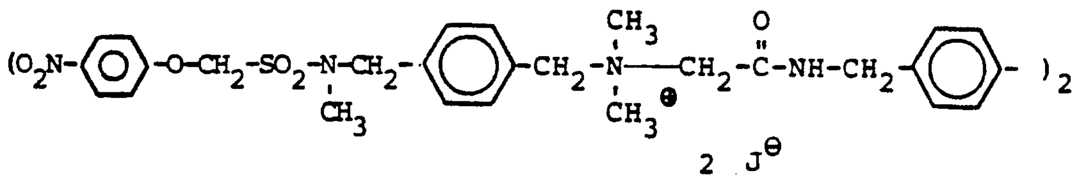

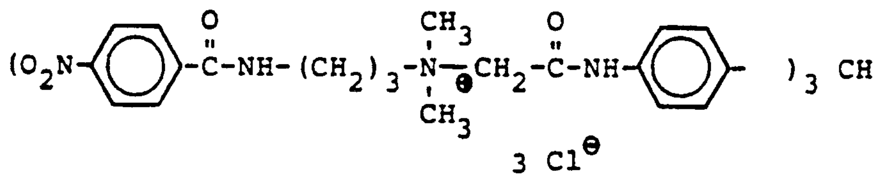

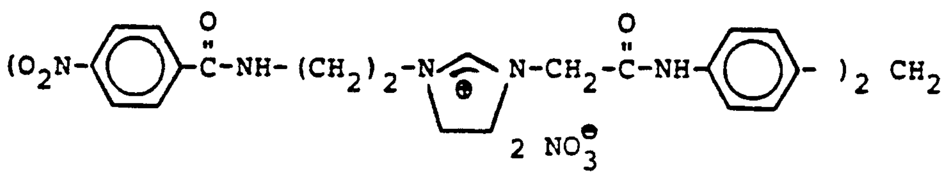

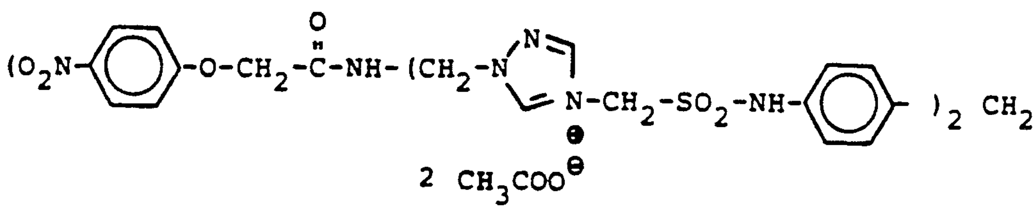

**Beispiel**

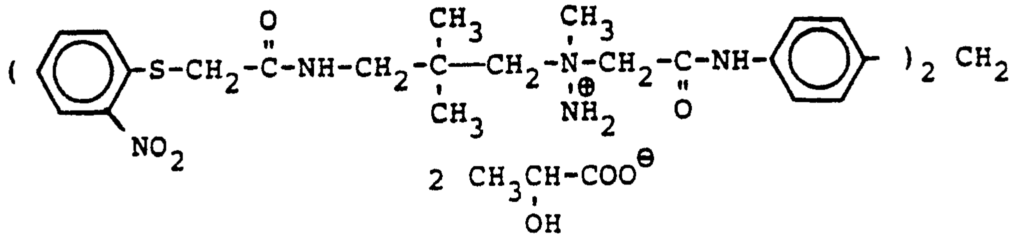

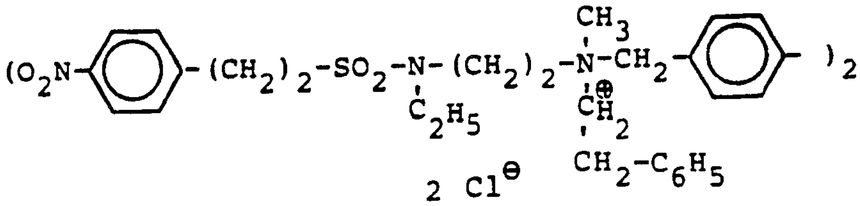

2 $Cl^{\ominus}$

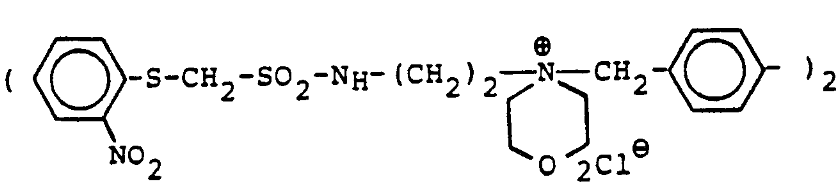

2 $Cl^{\ominus}$

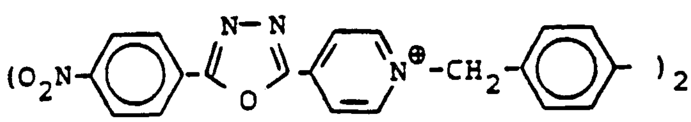

2 $Cl^{\ominus}$

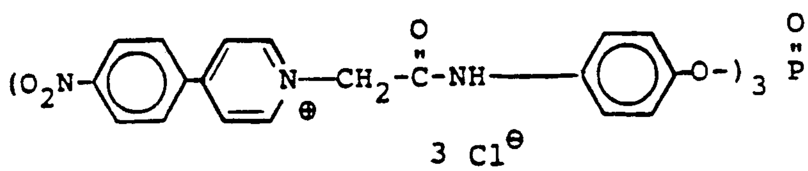

3 $Cl^{\ominus}$

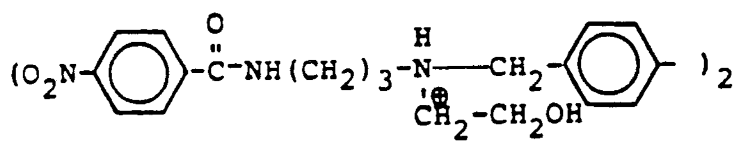

2 $CH_3-SO_3^{\ominus}$

Beispiel

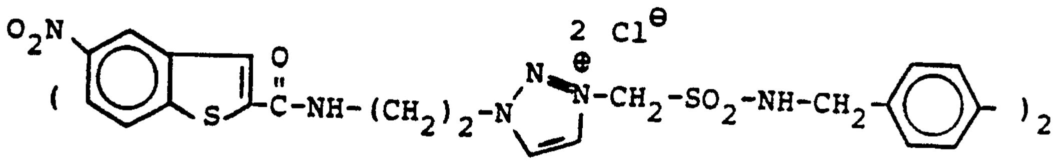

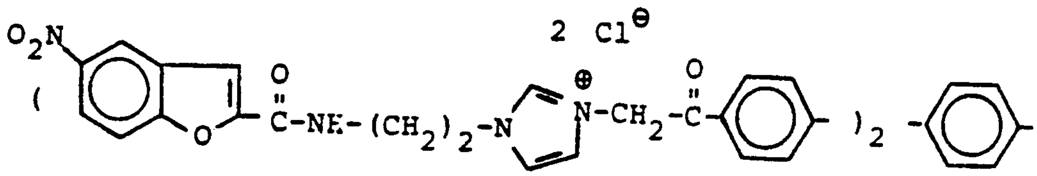

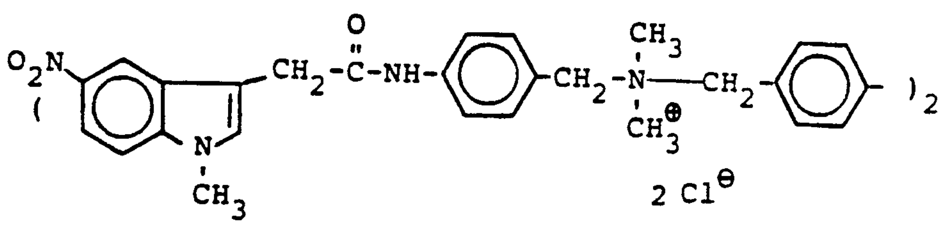

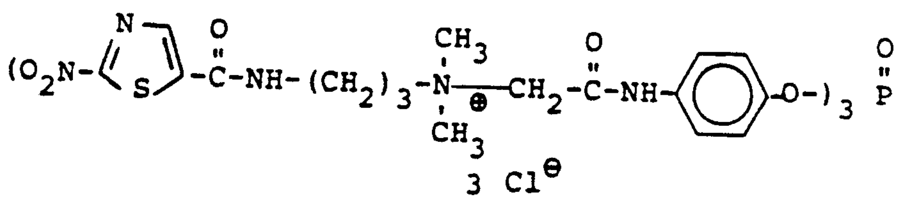

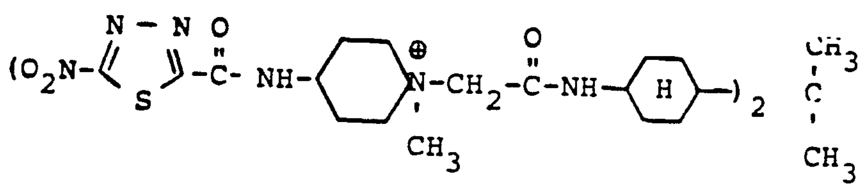

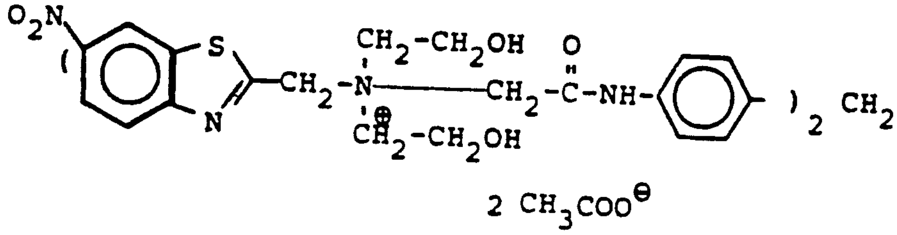

Beispiel 104

50 g 3-Nitrophenacylbromid und 40 g Bis-(isonicotinoylamidophenyl)-methan werden in 250 ml Dimethylformamid 30 h auf 100 °C erhitzt und abgekühlt. Der farblose kristalline Niederschlag wird abgesaugt, mit Isopropanol gewaschen und bei 40 °C im Vakuum getrocknet. Man erhält 85 g Verbindung der Formel

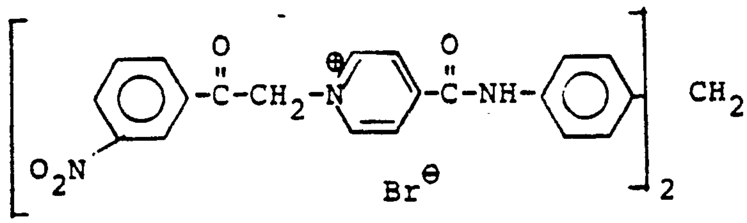

104

Eine 10 %ige Lösung von 104 in Milchsäure ist mit Wasser in jedem Verhältnis mischbar.

Bis-(isonicotinoylamidophenyl)-methan ist analog 4,4'-Bis-(chloracetamidophenyl)-methan (Beispiel 29) aus 4,4'-Diaminodiphenylmethan und 2 Äquivalenten Isonicotinoylchlorid zugänglich. 3-Nitrophenacylbromid kann analog Org. Synth., Coll. Vol. II, 420-481 aus 3-Nitroacetophenon und Brom hergestellt werden.

Analog Verbindung 104 werden die folgenden ähnlich wirksamen Verbindungen hergestellt:

Beispiel

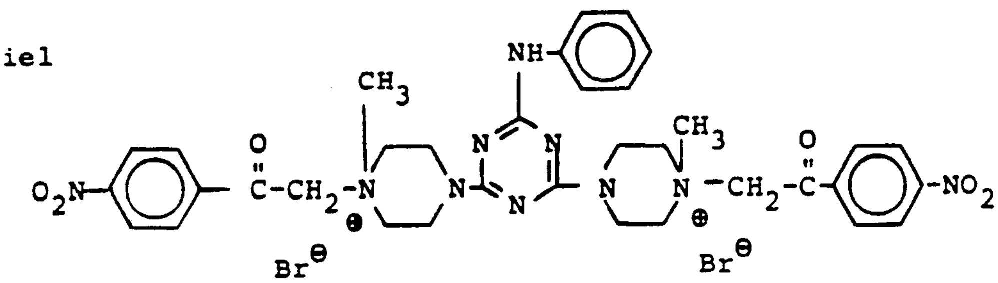

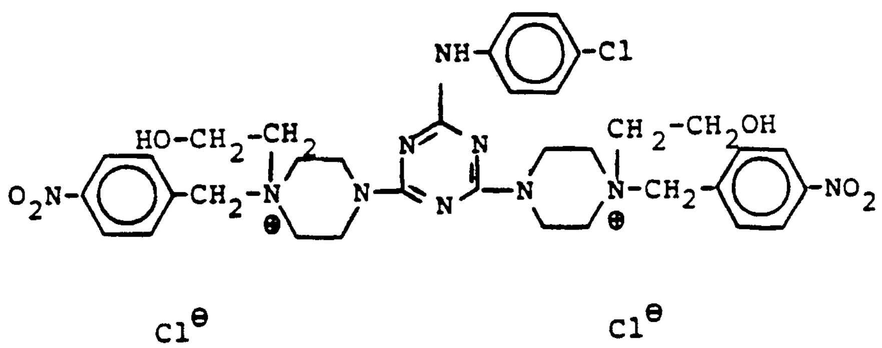

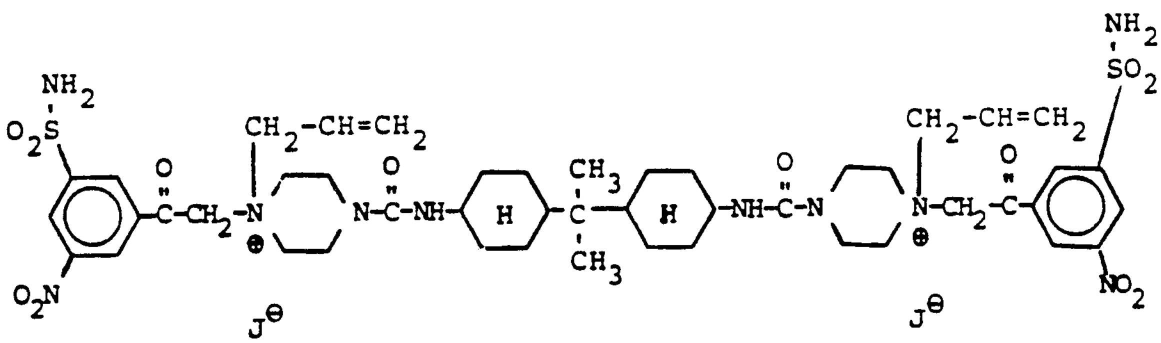

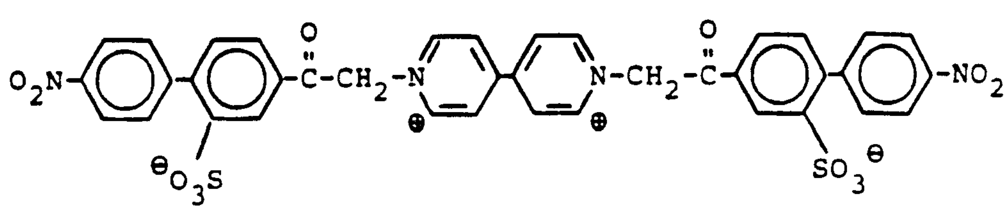

Beispiel

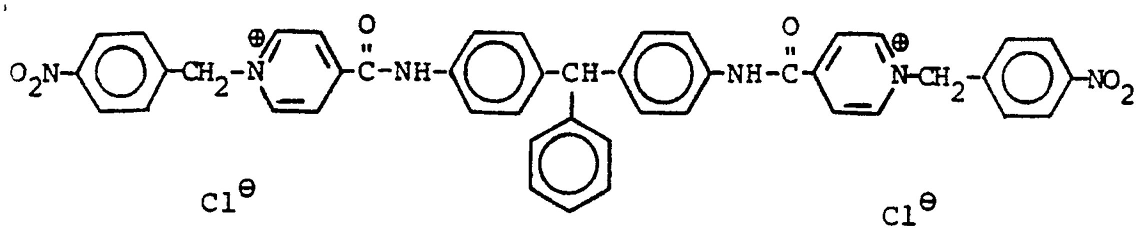

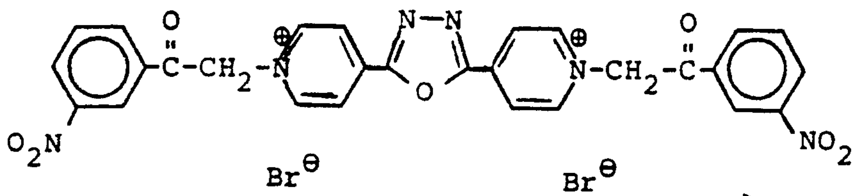

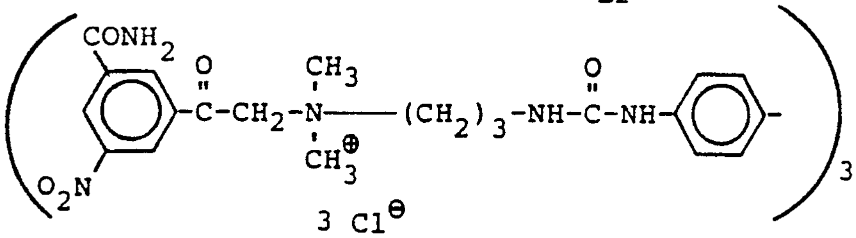

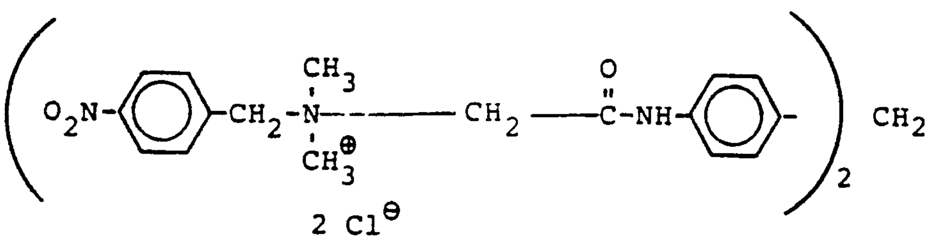

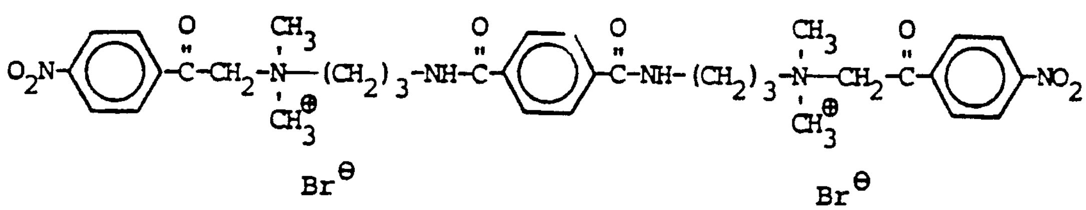

Beispiel

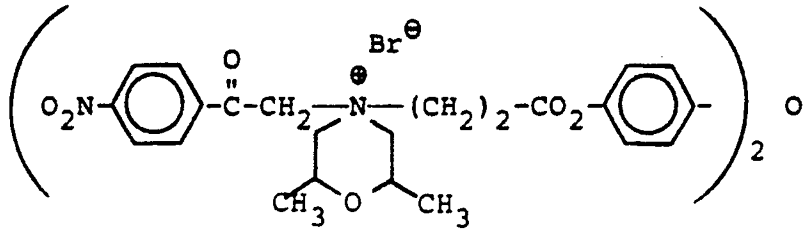

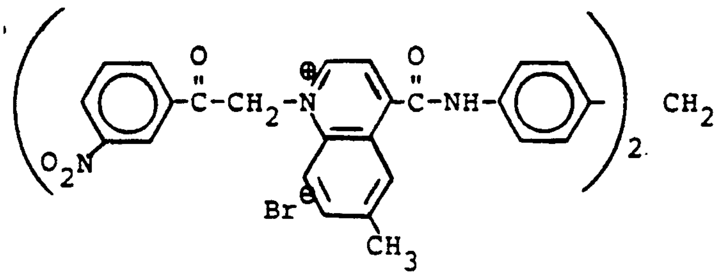

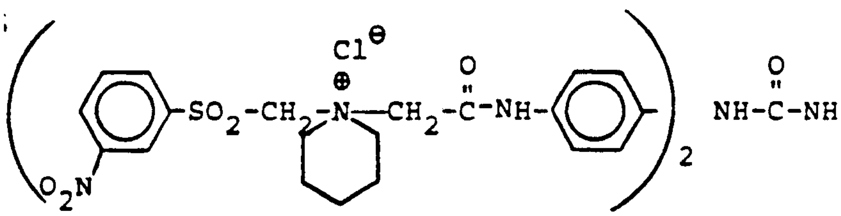

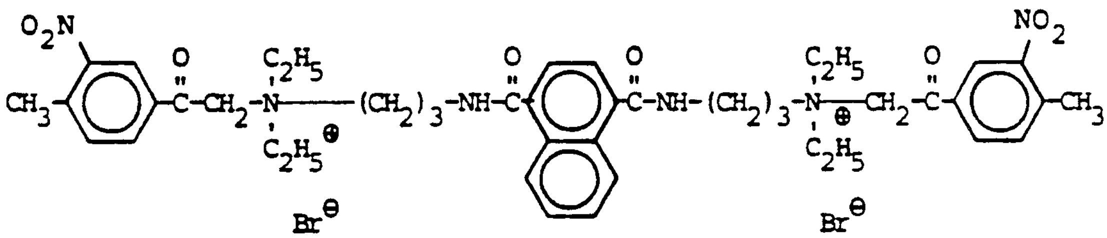

Beispiel

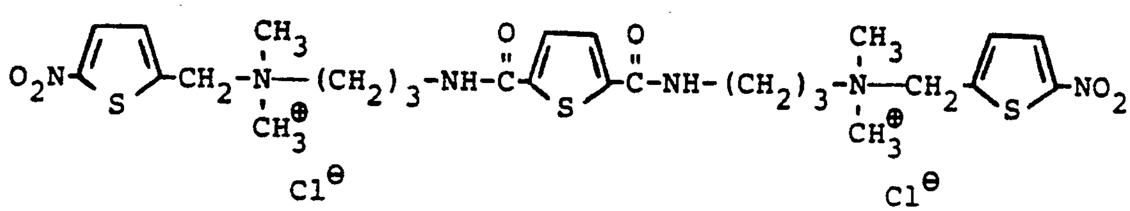

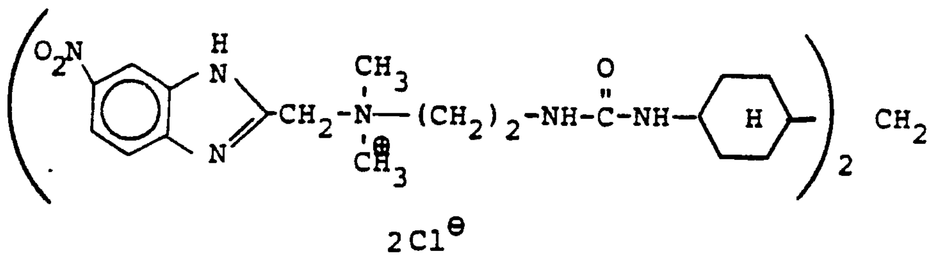

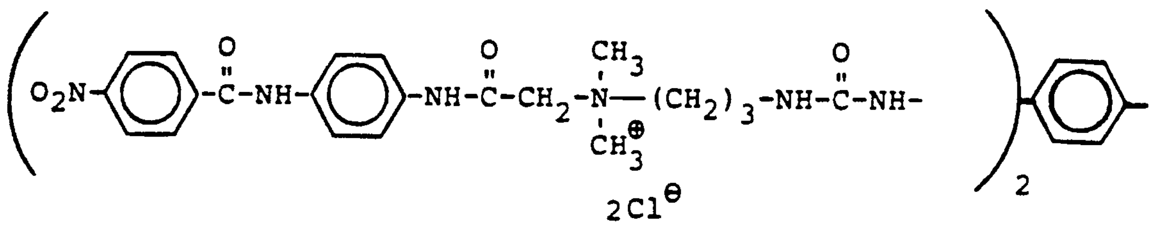

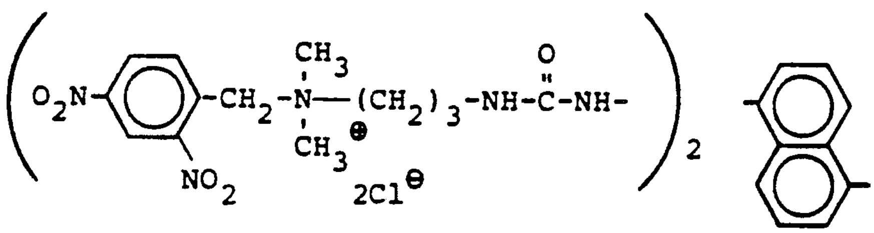

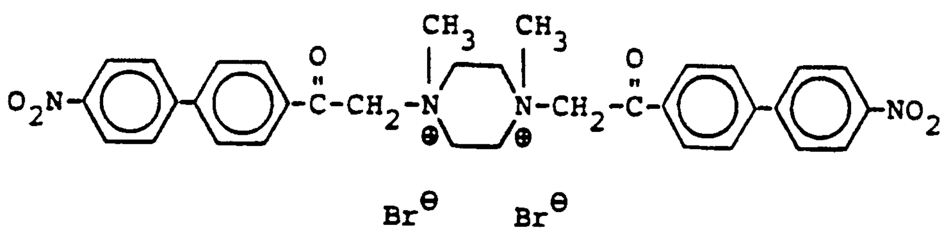

Beispiel

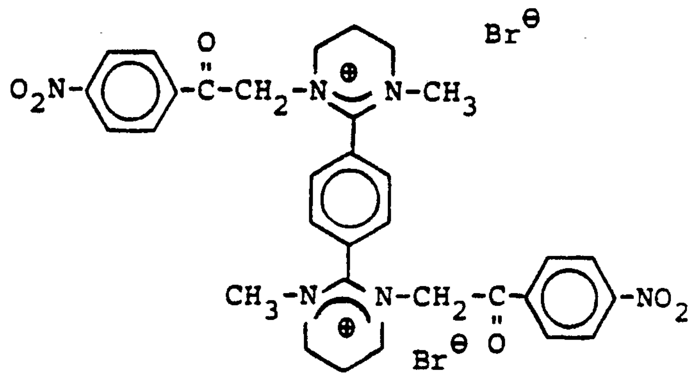

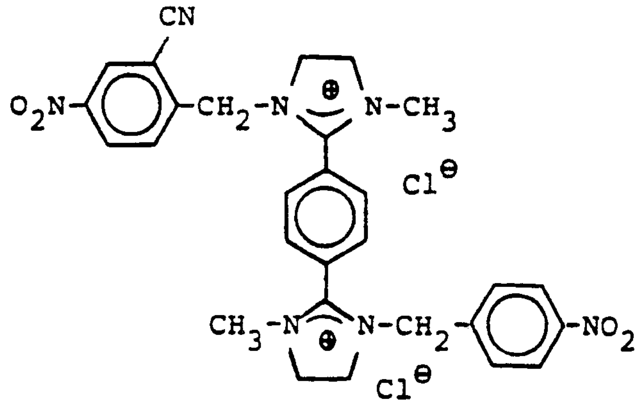

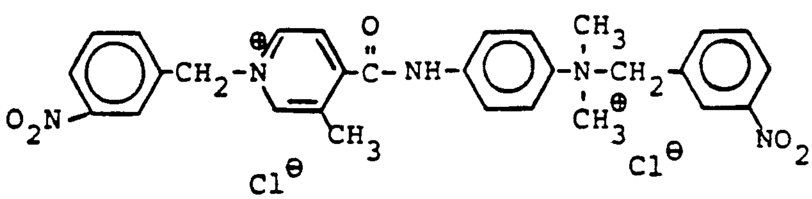

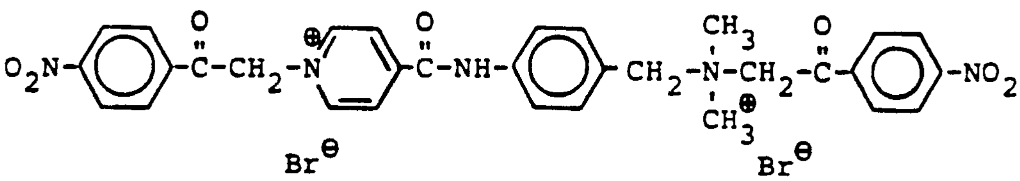

Beispiel

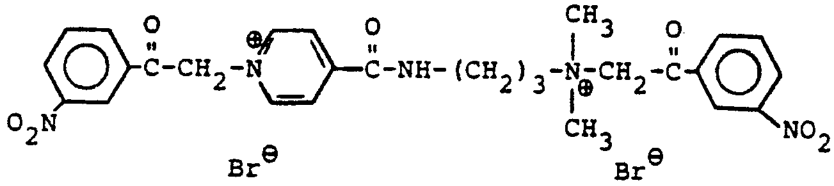

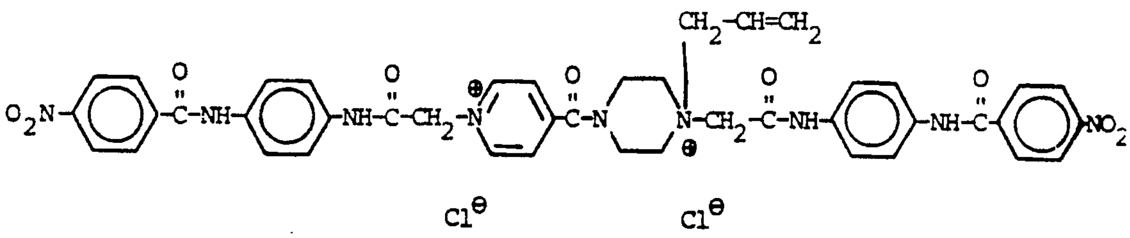

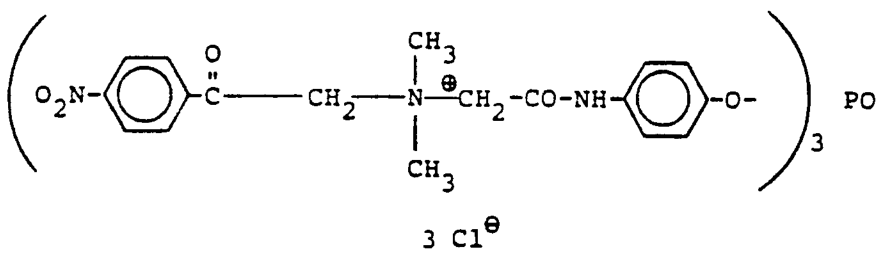

Mischung aus

(a) 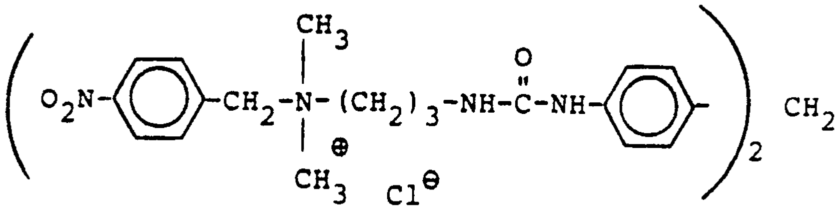 130

(b) 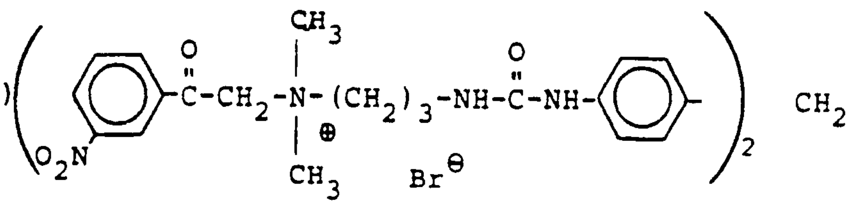 und

(c) 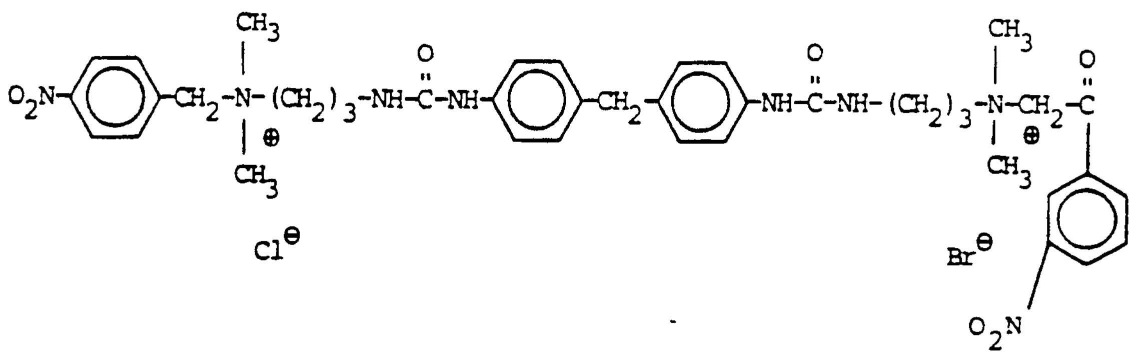

Beispiel

131

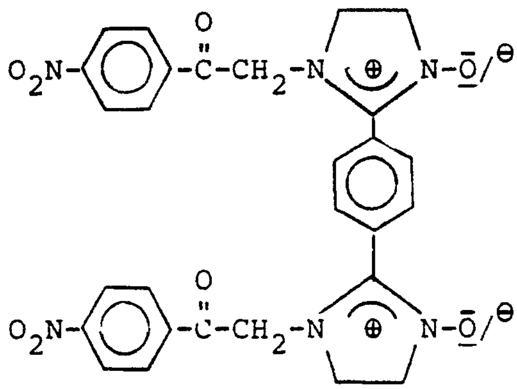

132

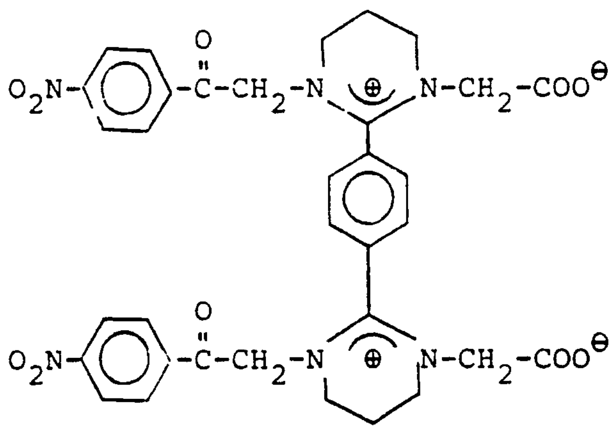

133

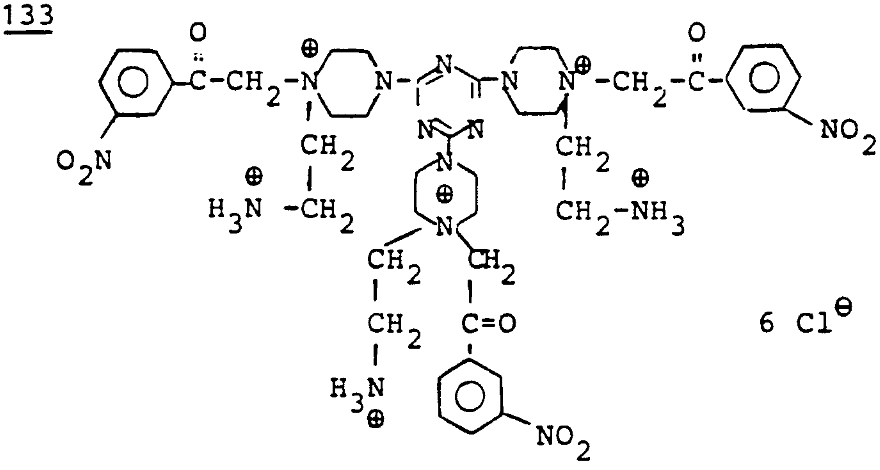

Beispiel 134

Aus 166 g Isophthalsäure und 103 g Diethylentriamin werden in Wasser und Triethylenglykol nach den Angaben der DE-A-1 912 647, Beispiel D, 1 Mol 50 %ige Polyimidazolin-Verbindung der Formel

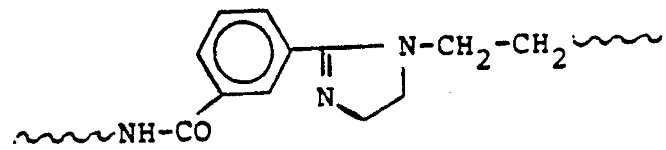

hergestellt.

43,5 g hiervon (0,1 Äquivalent, bezogen auf die Struktureinheit) werden mit 16 g 4-Nitrobenzylchlorid 16 h auf 110 °C erhitzt. Nach dem Abkühlen wird das Gemisch mit 3,5 ml Wasser verdünnt. Man erhält eine hellbraune, viskose Lösung, welche die Verbindung

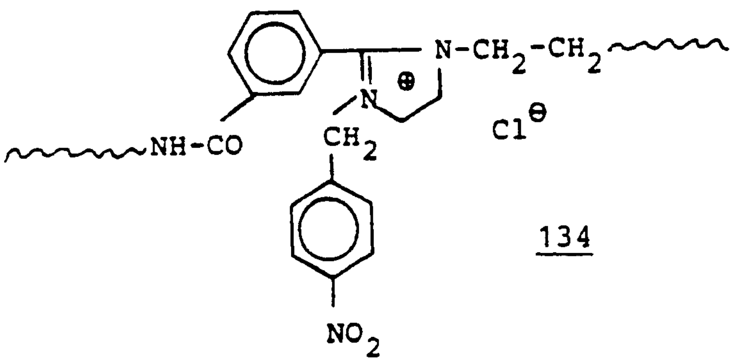

134

in Triethylenglykol/Wasser enthält.

Ein ähnlich stark wirksamer Fluoreszenzlöscher für anionische optische Aufheller wie 134 wird erhalten, wenn man 4-Nitrobenzylchlorid durch eine äquivalente Menge 3-Ni-trophenacylchlorid ersetzt.

Beispiel 135

227 g 5-Nitroisophthalsäure werden analog DE-A-1 912 647, Beispiel D und F bei 20 bis 90 °C unter exothermer Reaktion in 103 g Diethylentriamin und 150 ml Wasser unter Rühren eingetragen. Unter Stickstoffschutz erhitzt man das Gemisch innerhalb von 3 h auf 190 °C, wobei man Wasser über eine Kolonne abdestilliert. Man kondensiert weiter bei 190 bis 200 °C/30 Torr, gibt nach 2 Stunden 120 g Triethylenglykol hinzu und kondensiert weiter bei 190 bis 200 °C/40 bis 50 Torr. Nach 20 h wird das Vakuum mit Hilfe von Stickstoff aufgehoben und das Reaktionsgemisch bei 130 °C langsam mit 100 g trockenem Triethylenglykol verdünnt. Nach dem Abkühlen wird eine braune viskose Lösung erhalten, die rohes Polyimidazolin-Derivat der Formel

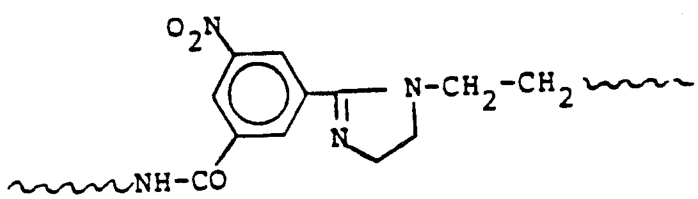

enthält.

Der 10. Teil der erhaltenen Lösung wird mit 8,5 g Chloracetamid 3 h auf 110 °C erhitzt und anschließend mit 10 ml Wasser verdünnt. Nach dem Abkühlen wird eine hellbraune viskose Lösung erhalten, welche die Verbindung

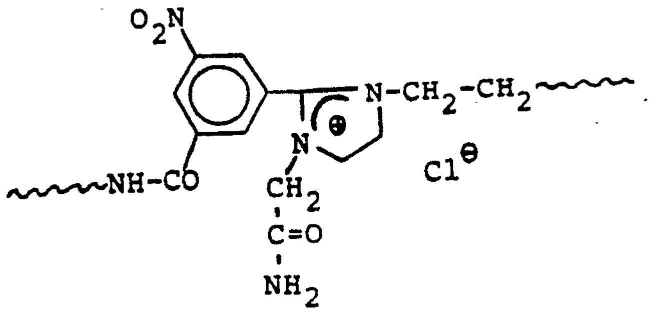

135

in Triethylenglykol/Wasser enthält.

Beispiel 136

9,3 g 4-Nitrobenzoylchlorid und 31 g Verbindung der Formel

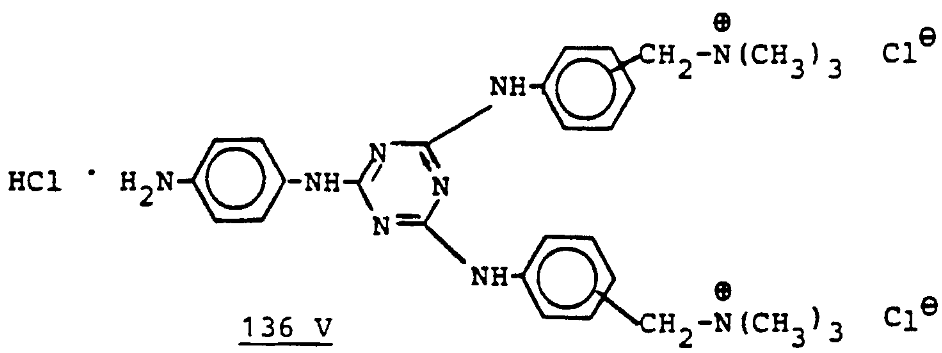

136 V

werden in 200 ml Dimethylformamid in Gegenwart von 19,1 g Triisopropanolamin, anfangs unter Kühlung, 20 h bei 20 bis 25° C verrührt und im Rotationsverdampfer im Vakuum bei 40° C Wasserbadtemperatur zur Trockne eingedampft. Der Rückstand wird 2 h mit 200 ml Ethanol verrührt, abgesaugt und mit Ethanol gewaschen. Man erhält 35 g Verbindung der Formel

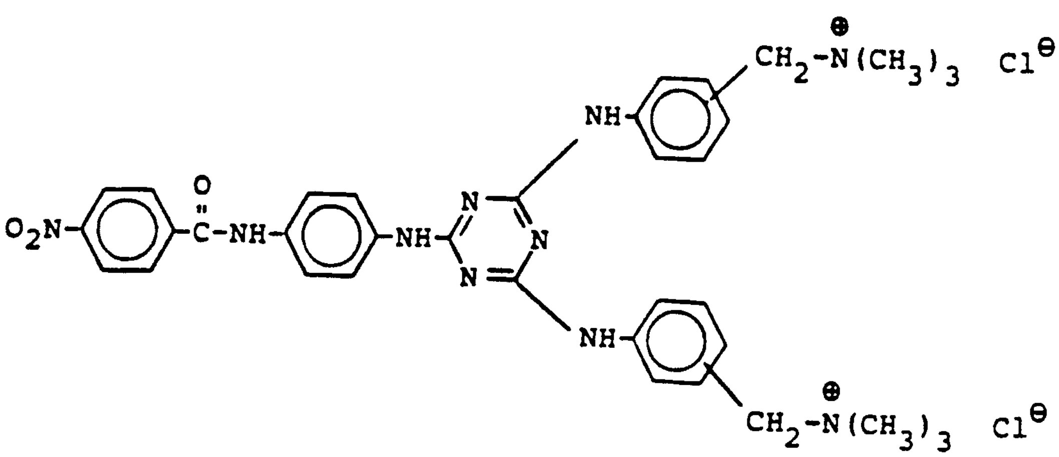

136

Das Zwischenprodukt 136 V ist auf folgendem Wege zugänglich:
43,5 g 4-Aminoacetanilid werden in einer Mischung aus
120 ml Wasser sowie

100 g Eis suspendiert und anschließend durch Zugabe von
1,7 ml 10 %iger Salzsäure auf pH 5,5 gestellt.
Diese Lösung wird vereinigt mit einer mit einem Rührwerk bei etwa 10.000 Umdrehungen homogenisierten,
0 bis 5° C kalten Suspension aus
53,6 g Cyanurchlorid,
120 ml Wasser,
48 g Eis und
1,2 g eines Polyethers aus Laurylalkohol und 5 Mol Ethylenoxid.
Die so erhaltene Suspension wird 3 h bei 5° C verrührt, wobei der pH-Wert durch Zutropfen von etwa
96 ml 10 %iger Natronlauge bei 4 gehalten wird. Anschließend fügt man eine mit etwa
30 g Natriumcarbonat auf pH 5 gestellte Lösung aus
166 g einer Mischung aus 2/3 4- und 1/3 3-Aminobenzyltrimethylammoniummethosulfat,
160 ml Wasser hinzu, erwärmt auf 95° C, stellt mit etwa
120 g Natriumcarbonat pH 8 ein und verrührt 3 h bei 95° C und pH 8.

Anschließend wird die Mischung mit
180 ml konzentrierter Salzsäure 2 h unter Rückfluß verrührt, von einer geringen Trübung filtriert, das Filtrat am Rotationsverdampfer stark eingeengt, der Rückstand mit
1 1 Methanol extrahiert und der Extrakt zur Trockene eingedampft.
Ausbeute: 153 g 136 V.

Das eingesetzte Amino-benzyltrimethylammoniummethosulfat wurde folgendermaßen hergestellt: 75 g einer Mischung aus 2/3 4- und 1/3 3-Amino-benzyl-dimethylamin werden in 200 ml Aceton bei 20 bis 25° C tropfenweise mit 47,5 ml Dimethylsulfat versetzt, wobei ein farbloses Kristallisat ausfällt. Man verrührt die Mischung 3 h bei Raumtemperatur. Der kristalline Niederschlag wird abgesaugt, mit 250 ml Isopropanol verrührt, abgesaugt, mit 100 ml Isopropanol gewaschen und bei 50° C im Vakuum getrocknet. Ausbeute 129 g. Analog Verbindung 136 werden die folgenden Verbindungen hergestellt:

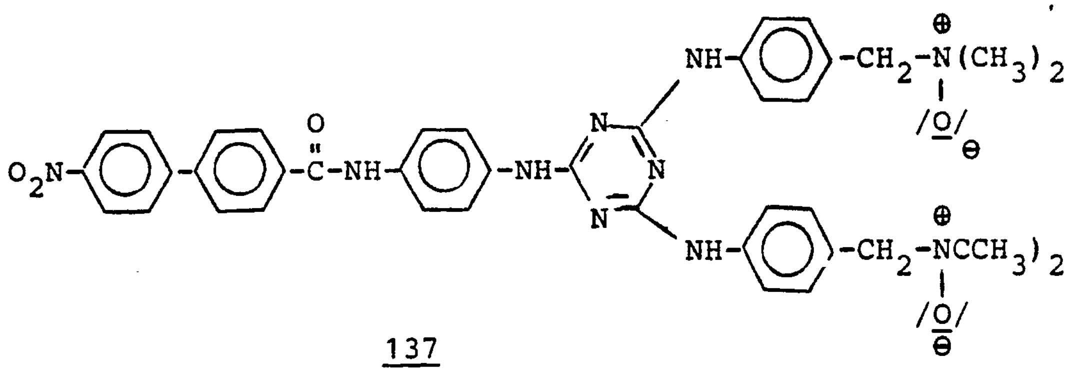

137

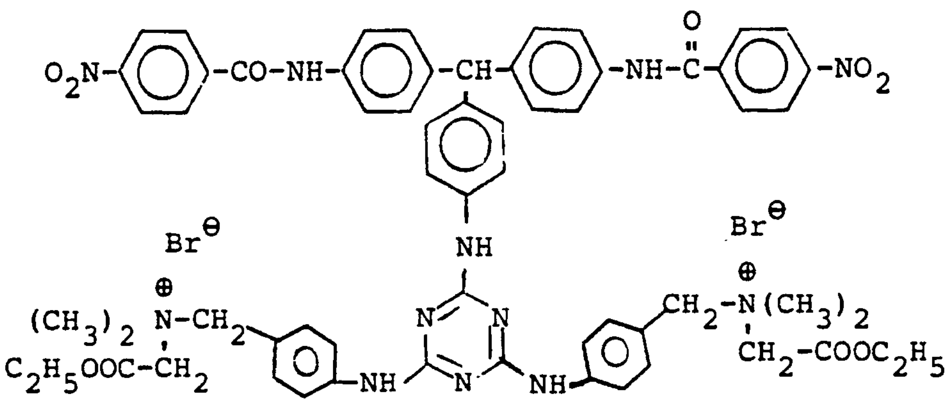

138

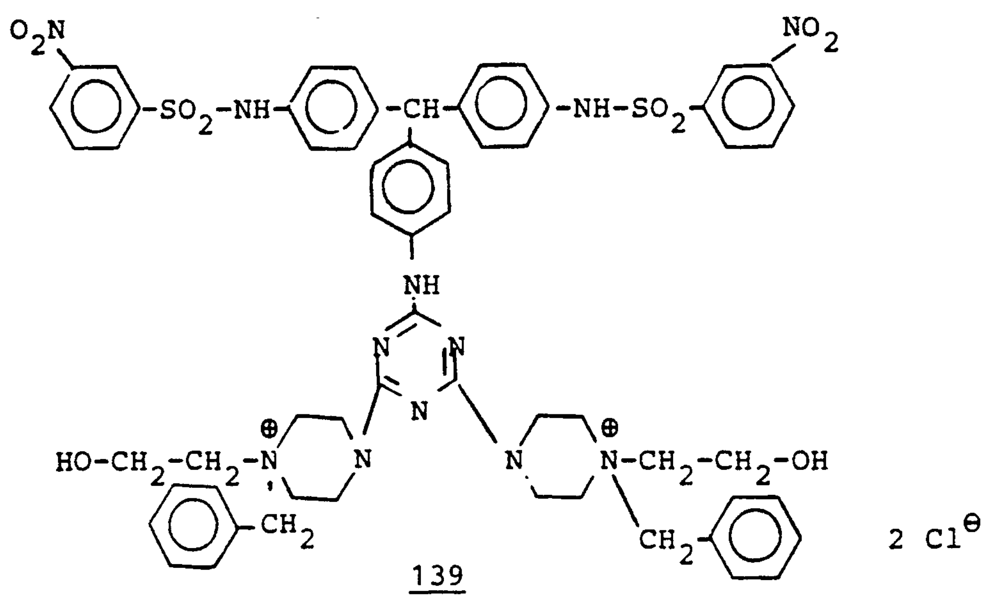

139

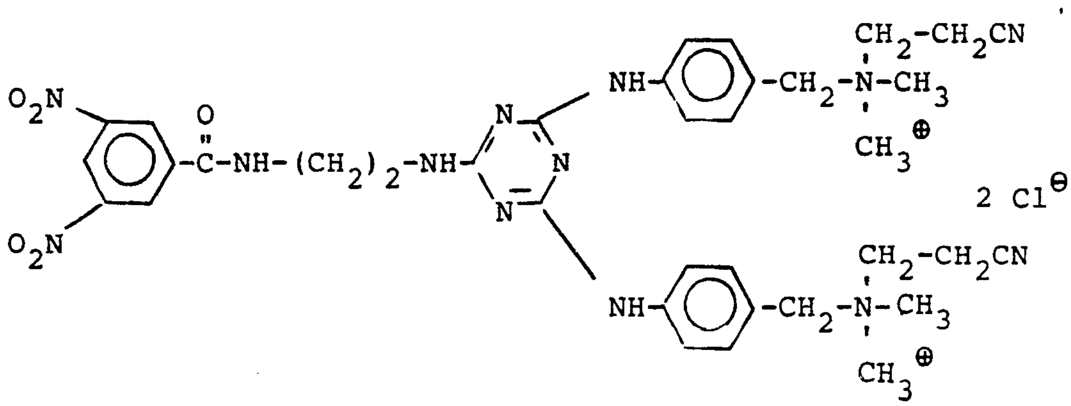

140

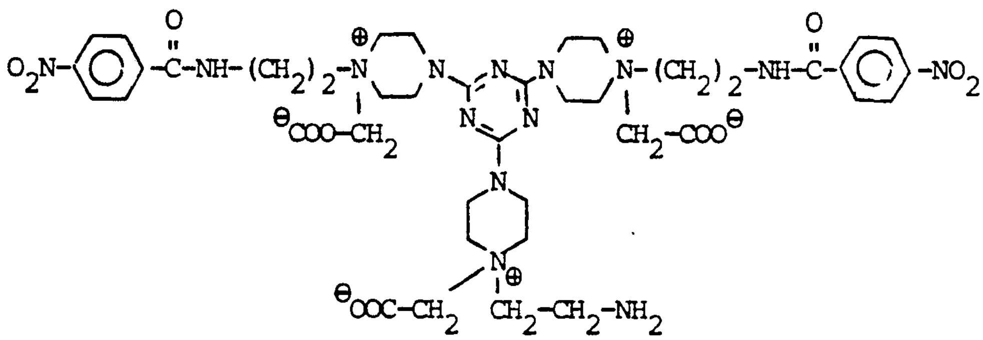

141

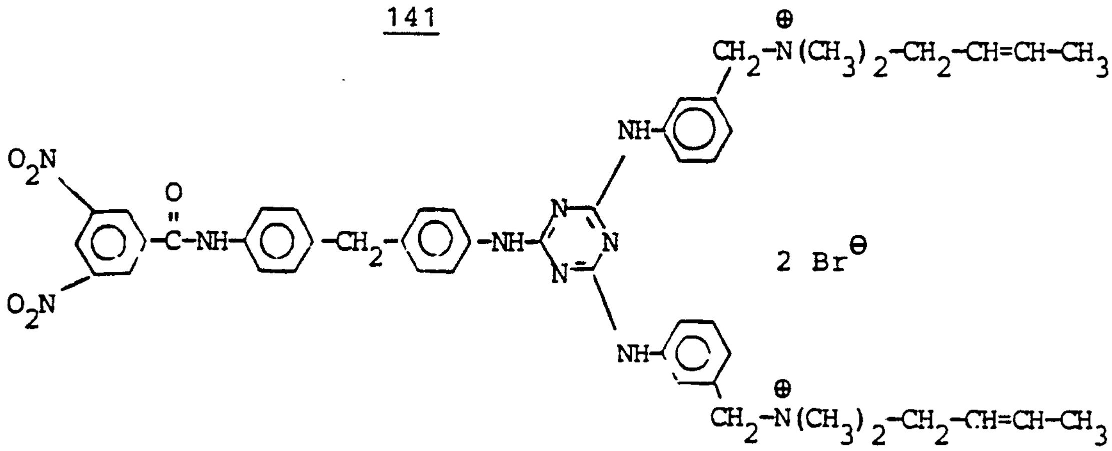

142

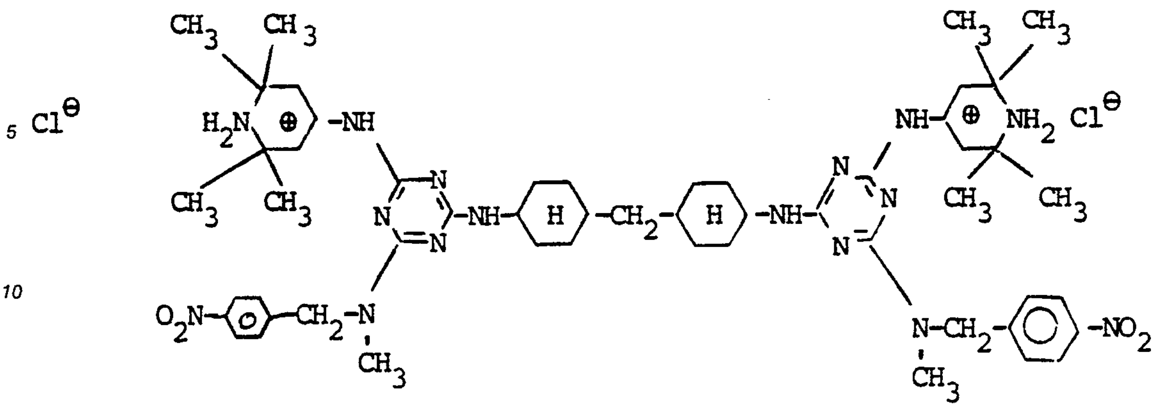

143

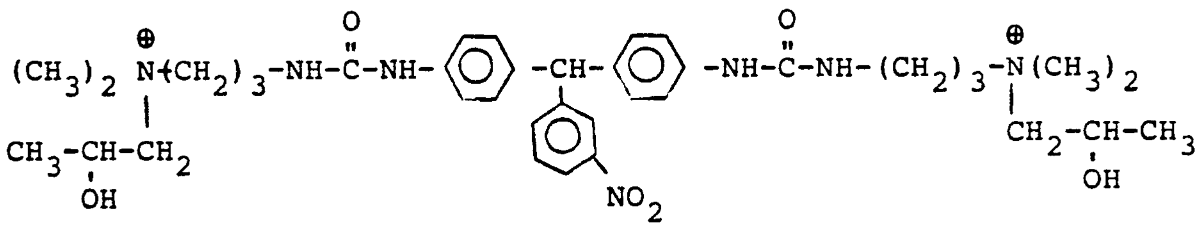

$2\ CH_3COO^{\ominus}$

144

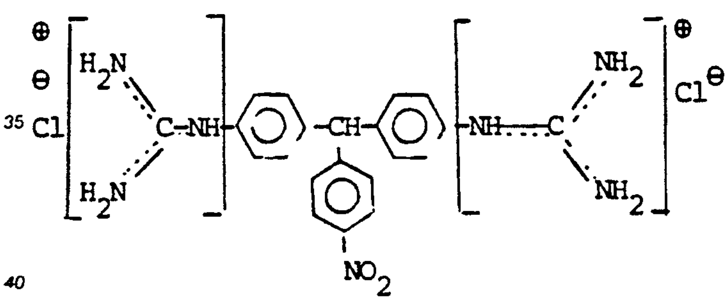

145

Die Verbindungen sind ebenfalls gut wirksame Fluoreszenzlöscher.

Beispiel 146

Eine Lösung von 70 g Maleinsäureanhydrid in 70 g Ethylacetat wird gemäß den Verfahren der DE-AS 1 495 850 und 1 469 727 im Laufe von 12 h bei 80 °C tropfenweise mit einer Lösung von 30 g Styrol und 6 g Benzoylperoxid in 100 g Ethylacetat versetzt; anschließend wird das Lösungsmittel im Vakuum abgedampft. Das erhaltene Mischpolymerisat, das ein Molgewicht von etwa 2000 besitzt, wird in 100 g Toluol suspendiert. Die Suspension wird innerhalb von 2 h bei 25 - 75 °C tropfenweise mit 75 g 3-Dimethyl-aminopropylamin versetzt und noch 15 h gerührt. Der kristalline Niederschlag wird abgesaugt, mit Toluol gewaschen und getrocknet. Die so hergestellte polymere Verbindung enthält Struktureinheiten der Formel

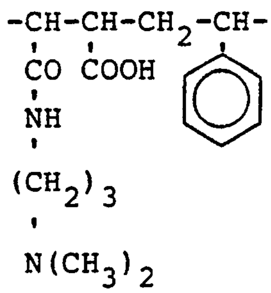

146V

0,1 Äquivalent hiervon (bezogen auf die Struktureinheit) und 16 g 4-Nitrobenzylchlorid werden in 100 ml Triethylenglykol 15 h auf 120 °C erhitzt, abgekühlt und mit 10 ml Wasser versetzt. Man erhält eine hellbraune viskose Lösung, welche eine polymere Verbindung mit Struktureinheiten der Formel

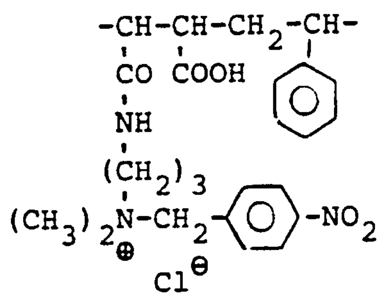

146

enthält. Die Verbindung zeigt unter den Bedingungen des Anwendungsbeispiels B starke fluoreszenzlöschende Wirkung.

Ein ähnlich stark wirksamer Fluoreszenzlöscher wird erhalten, wenn man 4-Nitrobenzylchlorid durch eine äquivalente Menge 3-Nitrophenacylchlorid ersetzt.

In analoger Weise werden polymere Fluoreszenzlöscher, die folgende Struktureinheiten enthalten, hergestellt:

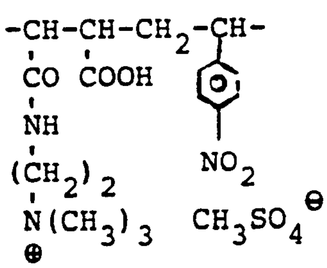

147

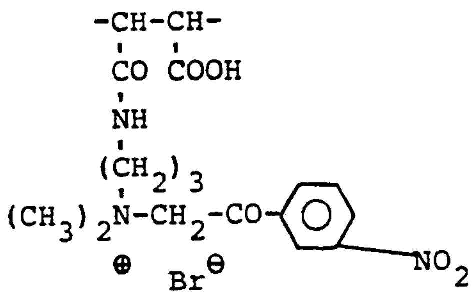

148

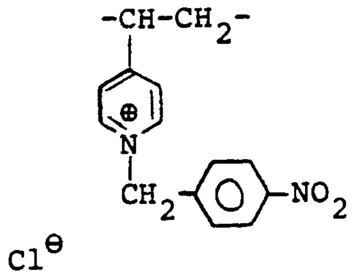

149

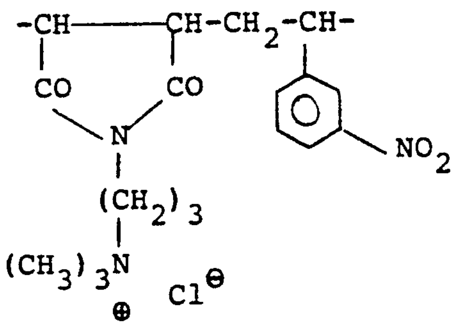

150

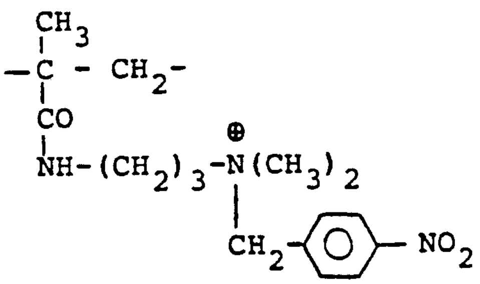

151

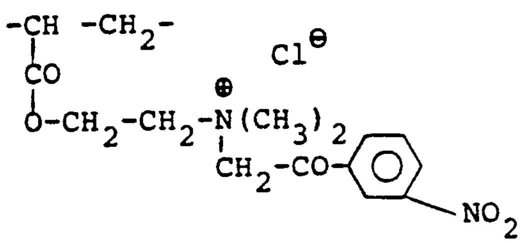

152

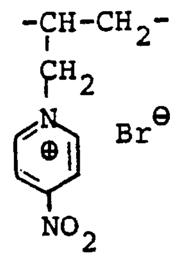

153

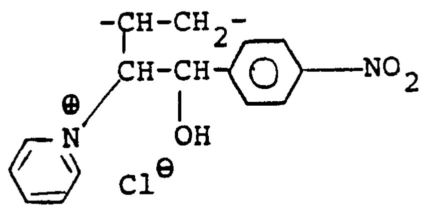

154

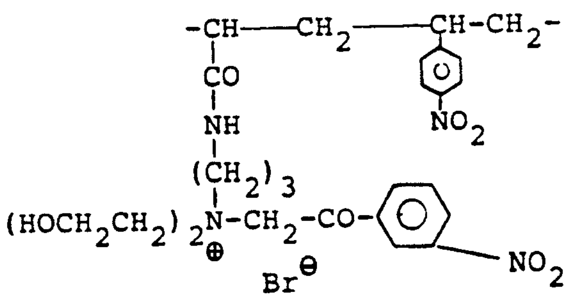

155

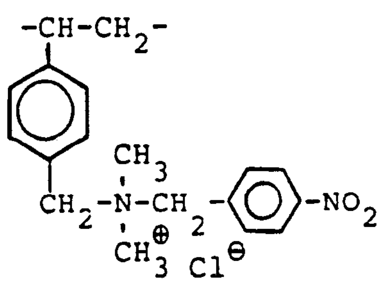

156

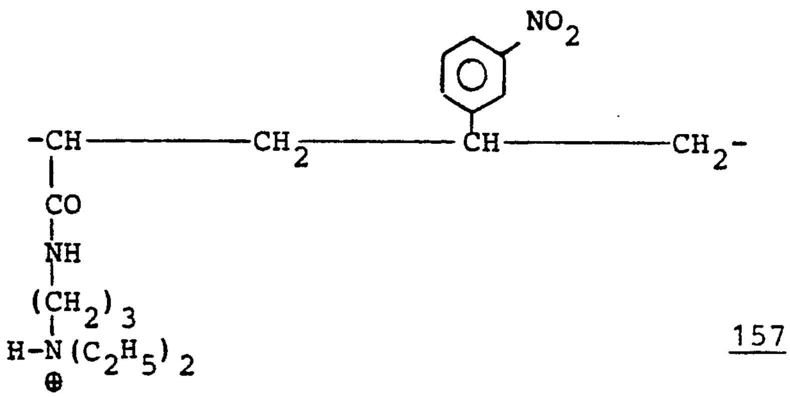

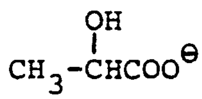

157

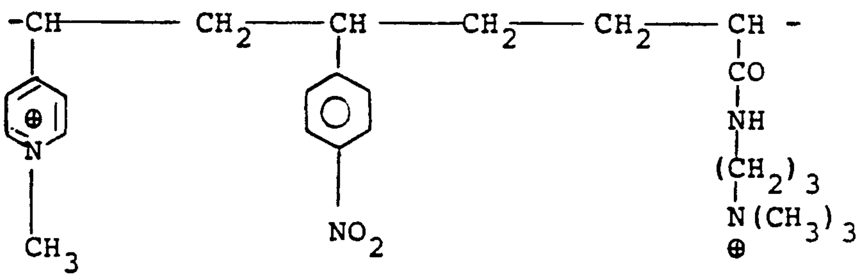

$2\ Br^{\ominus}$

158

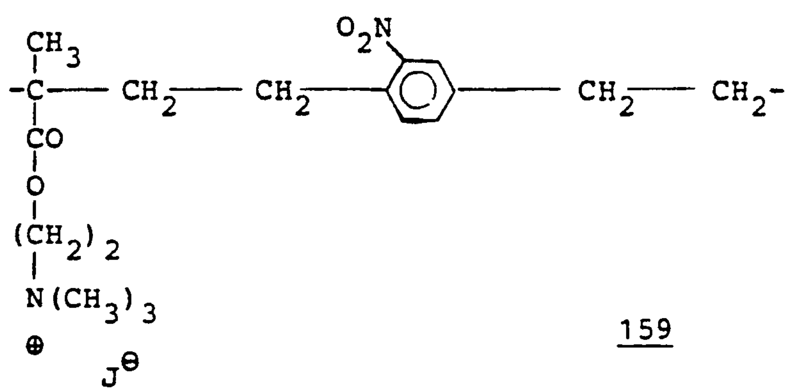

$J^{\ominus}$

159

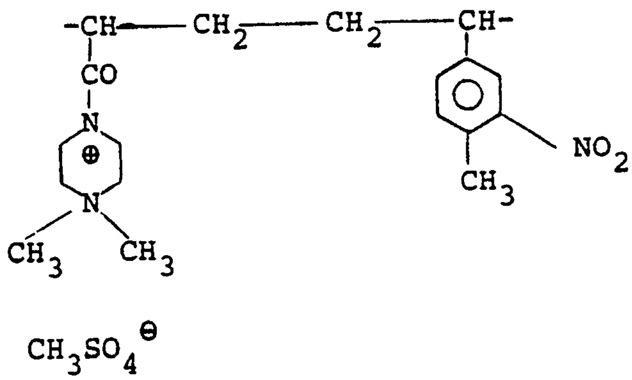

160

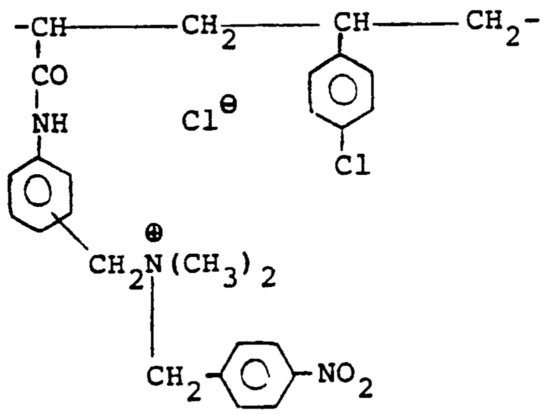

161

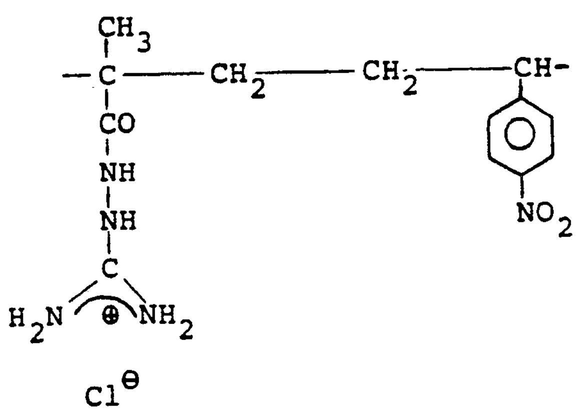

162

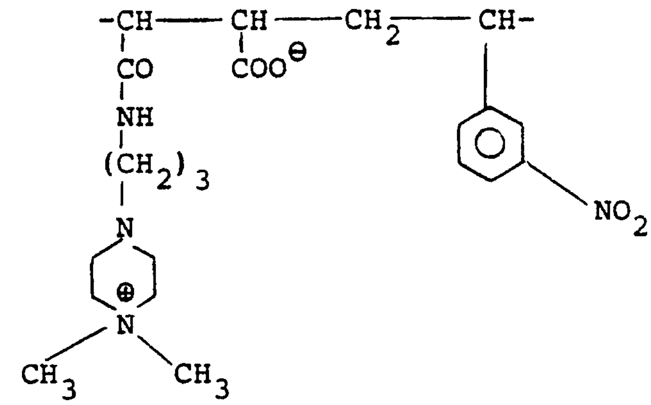

163

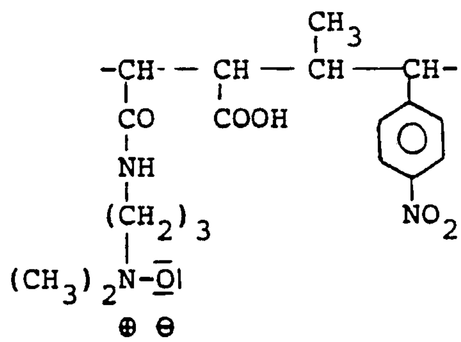

164

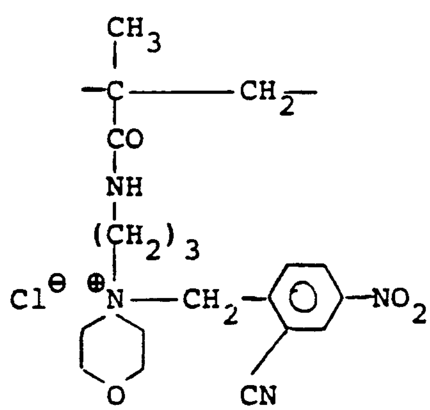

165

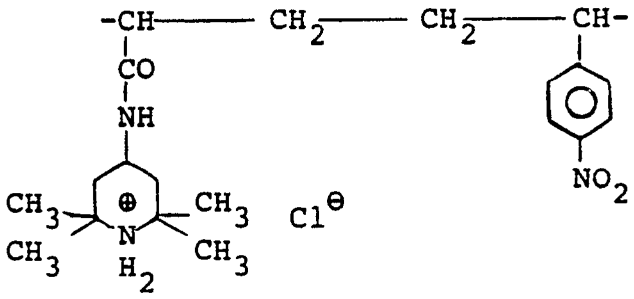

166

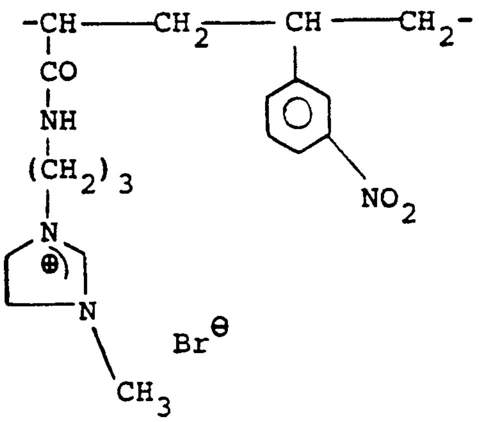

167

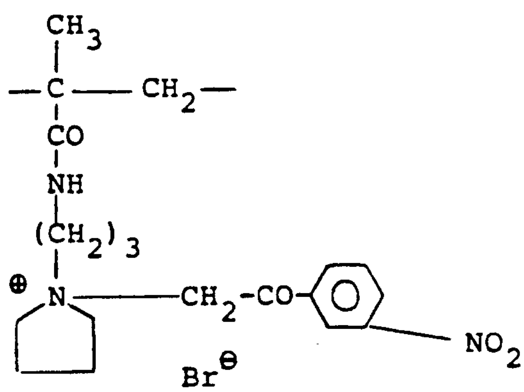

168

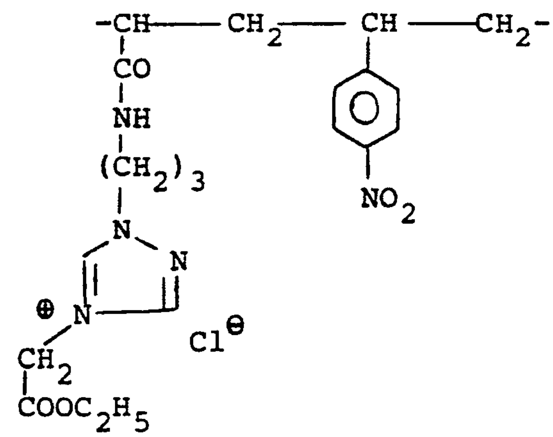

169

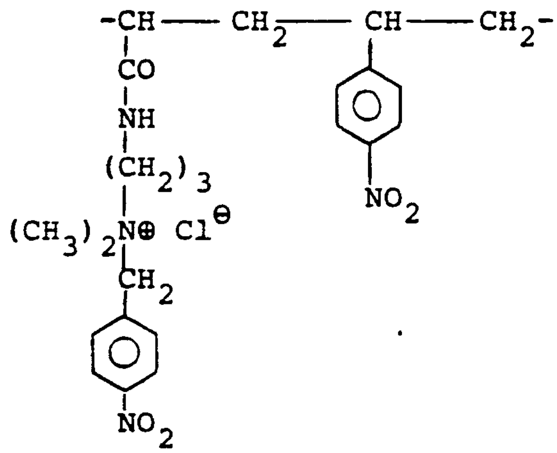

170

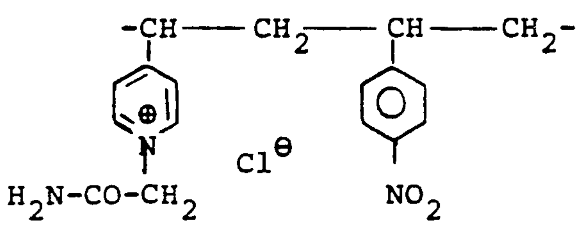

171

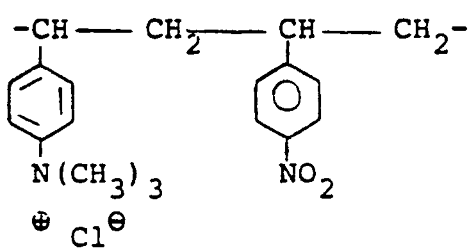

172

Beispiel 173

35 g 4,4-Bis-(chloracetamidophenyl)-methan und 27 g 4-Nitropyridin werden in 150 ml Dimethylformamid unter Stickstoffschutz 4 h auf 120 °C erhitzt. Nach dem Abkühlen wird der kristalline Niederschlag abgesaugt, mit Isopropanol gewaschen und im Vakuum getrocknet. Man erhält 52 g Verbindung der Formel

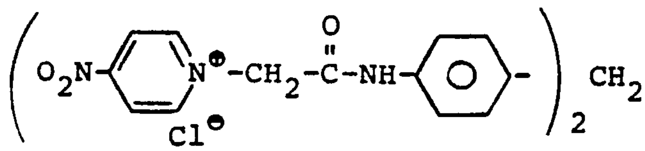

173

Unter den Bedingungen des Anwendungsbeispiels B zeigt 173 starke fluroeszenzlöschende Wirkung. In analoger Weise werden die folgenden, ähnlich stark fluoreszenzlöschenden Verbindungen hergestellt:

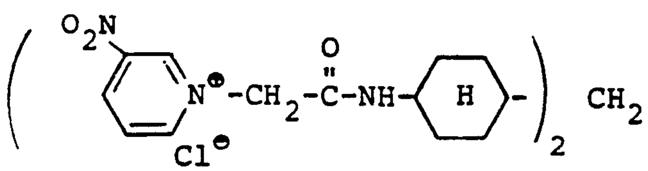

174

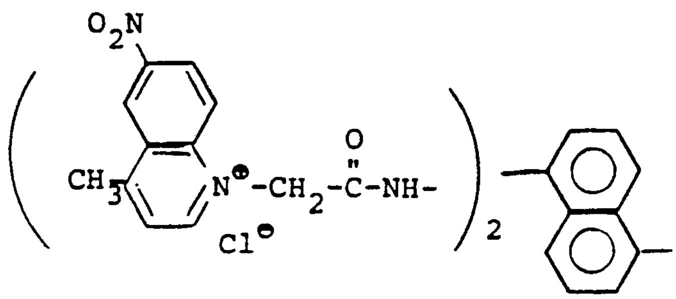

175

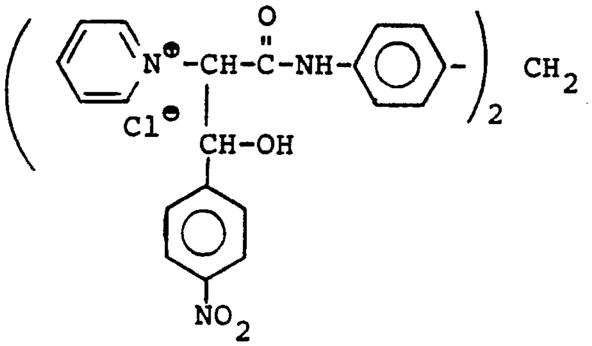

176

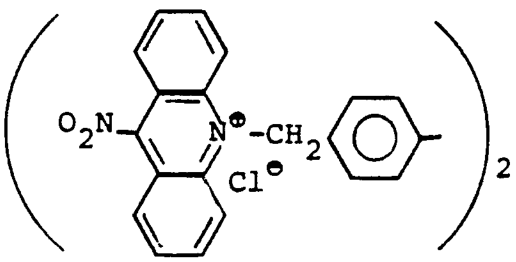

177

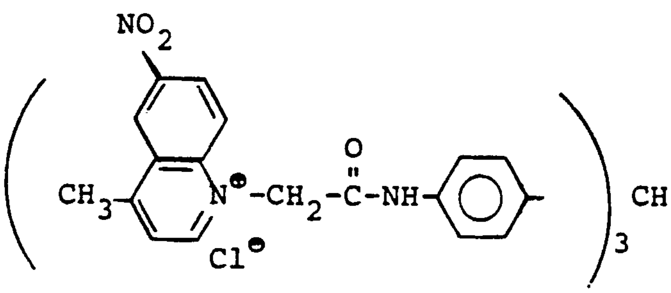

178

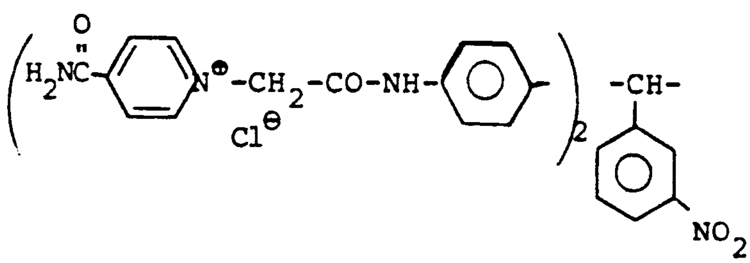

179

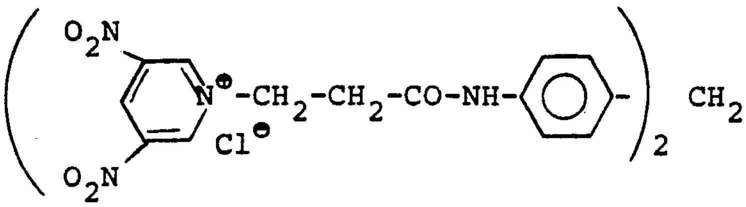

180

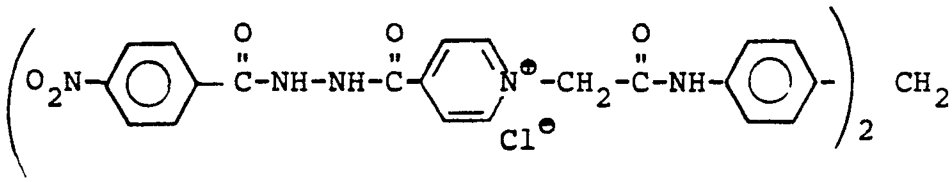

181

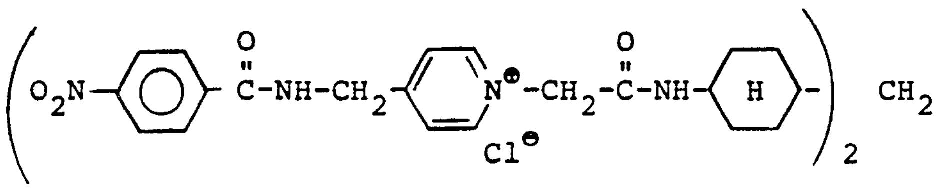

182

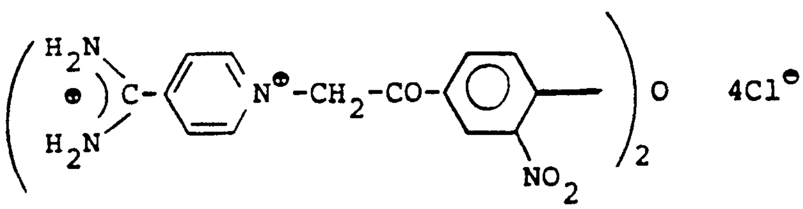

183

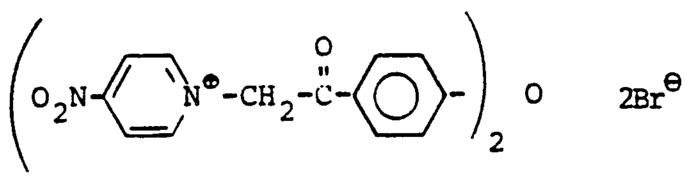

184

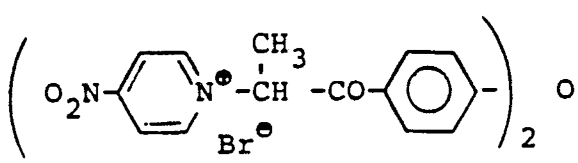

185

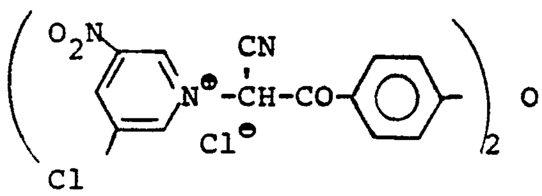

186

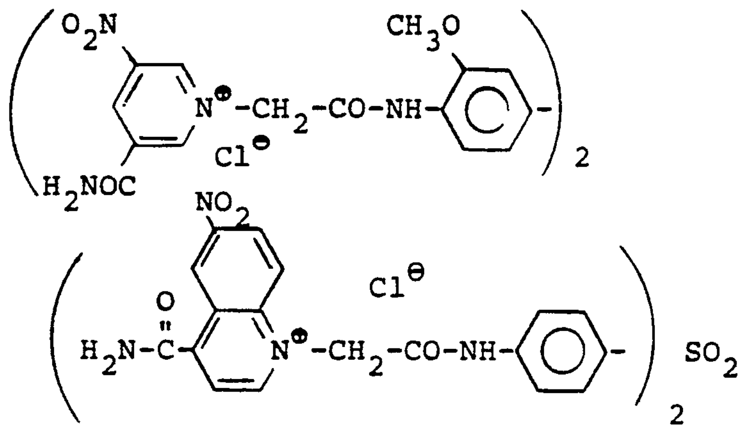

187

188

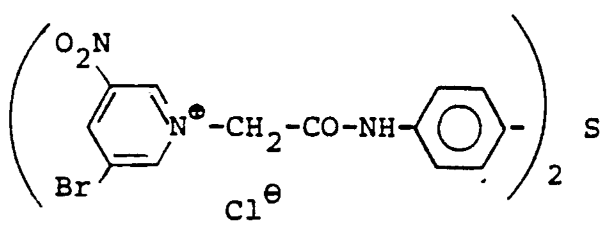

189

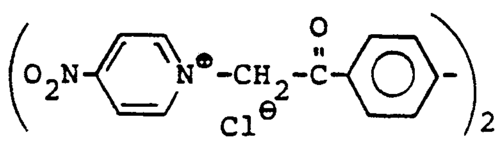

190

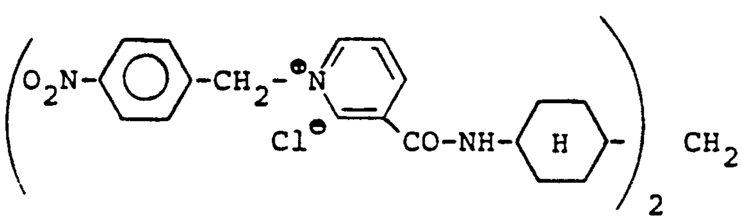

191

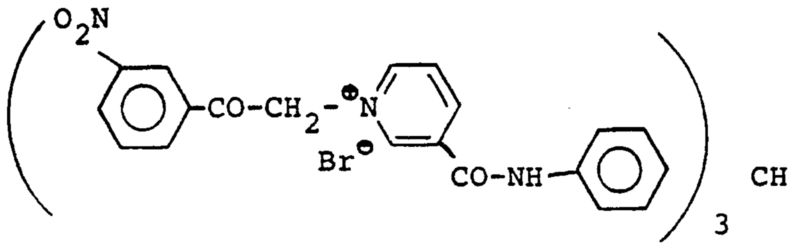

192

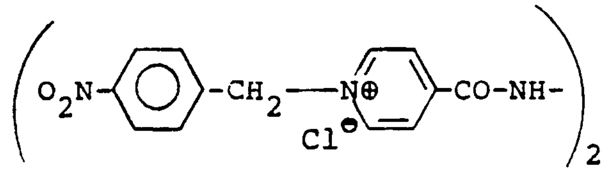

193

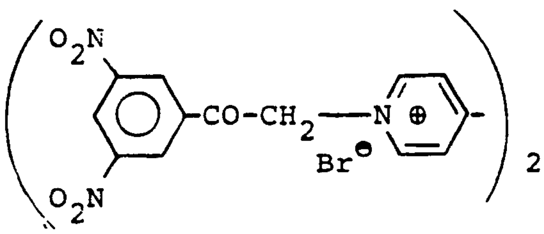

194

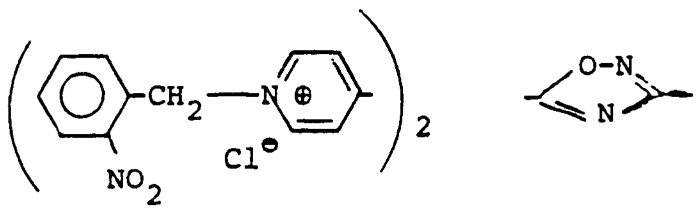

195

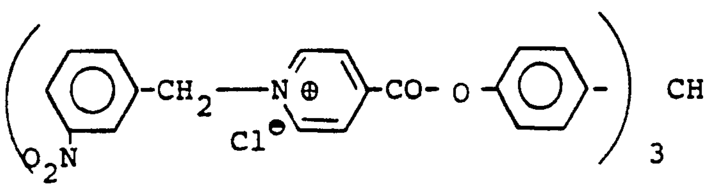

196

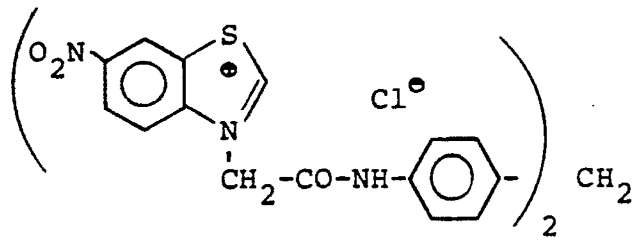

197

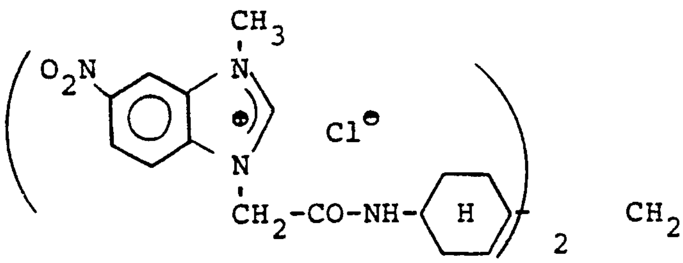

198

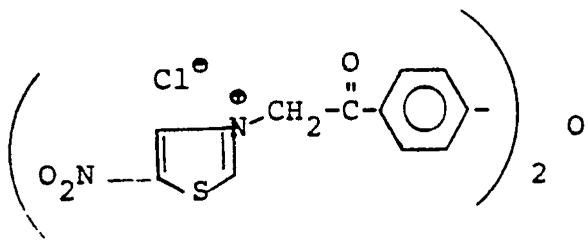

199

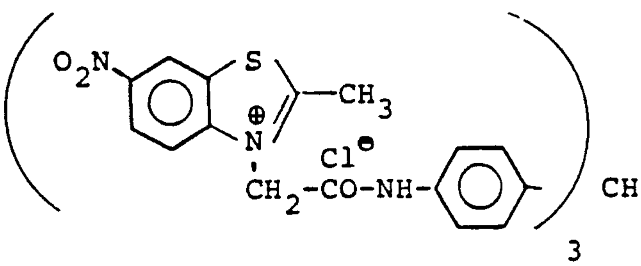

200

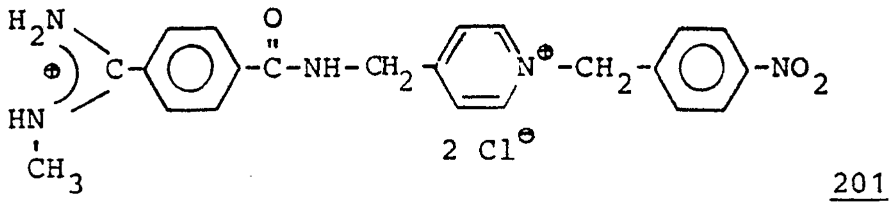

201

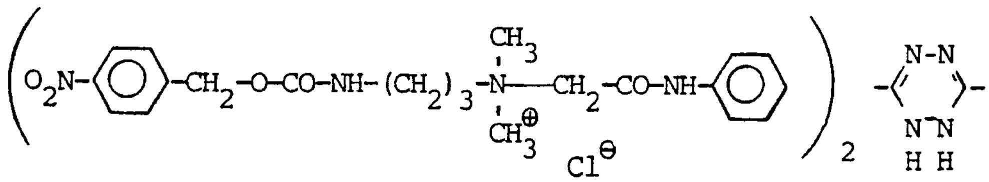

202

(Herstellung analog Beispiel 27)

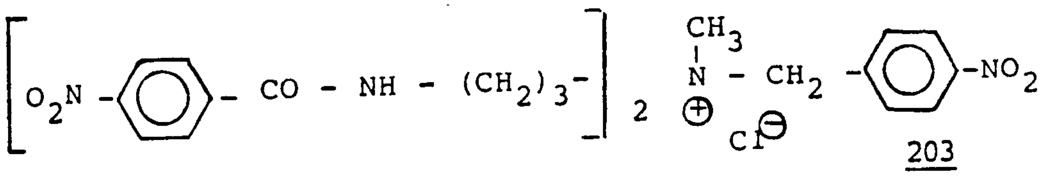

203

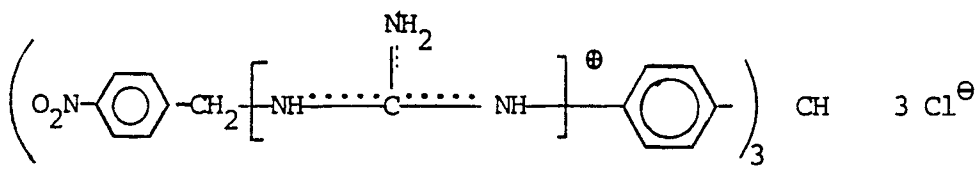

204

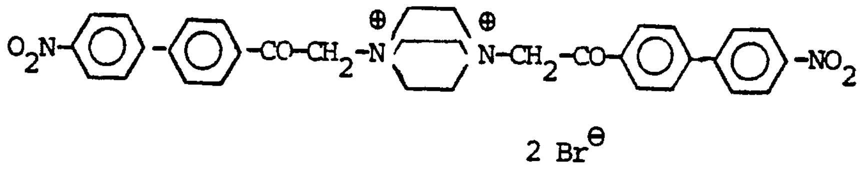

205

(Herstellung analog Beispiel 104)

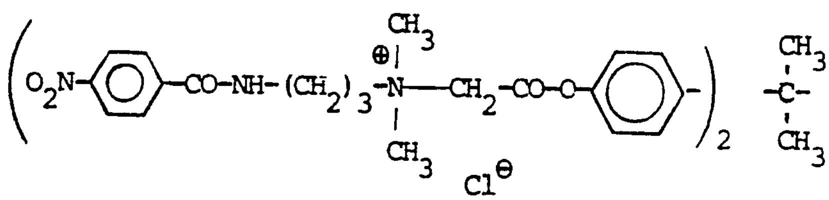

206

(Herstellung analog Beispiel 27)

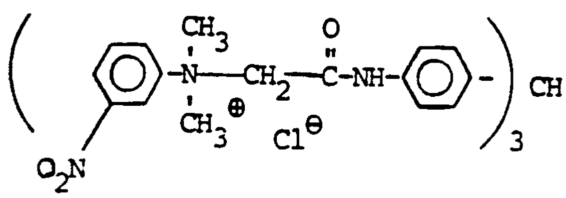

207

(Herstellung analog Beispiel 27)

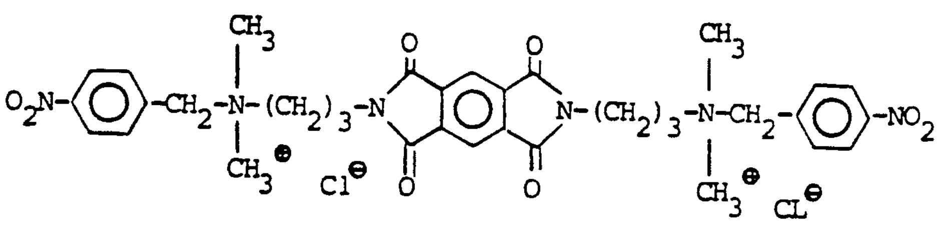

208

(Herstellung analog Beispiel 104)

Anwendungsbeispiel A

5000 Teile Papierbrei aus gebleichter Sulfitcellulose mit einem Mahlgrad von 40° Schopper-Riegler und einem Trockenstoffgehalt von 2 % (entsprechend 100 Teilen Sulfitcellulose) werden unter Rühren mit Natronlauge auf pH 7,5 gestellt, mit einer Lösung von 0,2 Teilen des optischen Aufhellers der Formel

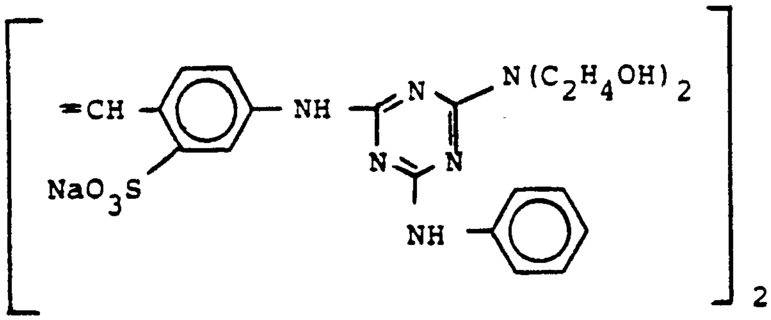

in 100 Teilen Wasser versetzt und 5 Minuten verrührt. Anschließend setzt man 100 Teile einer wäßrigen Lösung, die 0,4 Teile des im Herstellungsbeispiel 27 beschriebenen Fluoreszenzlöschers enthält zu, verrührt 1 Minute und bildet das Papierblatt. Nach dem Trocknen wird ein Papier erhalten, das sich von nichtaufgehelltem Papier praktisch nicht unterscheidet. Setzt man dagegen die gleiche Menge eines nach DE-A- 1 912 647, Beispiel A oder B oder eines nach DE-A- 24 48 293, Herstellungsbeispiel 1 hergestellten Fluoreszenzlöschers ein, wird ein Papier mit deutlich wahrnehmbarem Weißgrad erhalten. Wiederholt man die Vergleichsversuche im pH-Bereich von 9, ist der Unterschied noch größer.

Anwendungsbeispiel B

5000 Teile Papierbrei aus gebleichter Sulfitcellulose mit einem Mahlgrad von 40° Schopper-Riegler und einem Trockenstoffgehalt von 2 % werden einige Minuten mit 3 Teilen kristallisiertem Aluminiumsulfat verrührt, mit einer Lösung von 0,1 Teilen des optischen Aufhellers der Formel

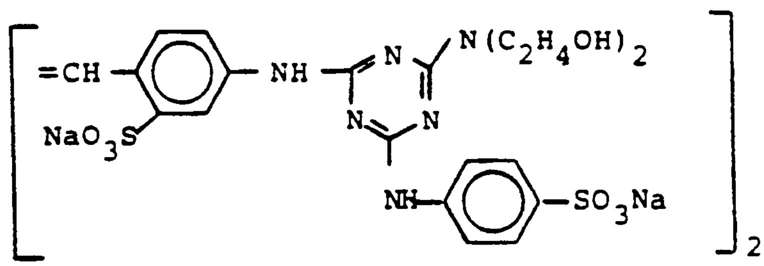

in 100 Teilen Wasser versetzt. Nach 15 Minuten werden 2 Teile Harzmilch zugegeben. Nach guter Durchmischung stellt man mit Schwefelsäure auf pH 4,5. Nun fügt man 100 Teile einer wäßrigen Lösung, die 0,7 Teile des im Beispiel 89 beschriebenen Fluoreszenzlöschers enthält, hinzu, verrührt 1 Minute, verdünnt mit Wasser auf 20 000 Teile und bildet das Papierblatt. Nach dem Trocknen wird ein geleimtes Papier erhalten, das sich von nichtaufgehelltem praktisch nicht unterscheidet.

Ähnlich starke Fluoreszenzlöscheffekte werden erhalten, wenn man einen der in den anderen Herstellungsbeispielen beschriebenen Fluoreszenzlöscher einsetzt.

Anwendungsbeispiel C

Auf einer Langsiebmaschine wird unter Verwendung von aufgehelltem Altpapier als Rohstoff Papier mit einem Gewicht von 80 g/m² produziert.

Zum Löschen der Fluoreszenz der darin enthaltenen optischen Aufheller besprüht man die Papierbahn in der zweiten Hälfte der Siebpartie mit einer verdünnten wäßrigen Lösung der im Herstellungsbeispiel 129 beschriebenen Verbindungen derart, daß das trockene Papier 0,02 bis 0,1 % (je nach Konzentration des Aufhellers im Rohstoff) Fluoreszenzlöscher enthält.

Das so fabrizierte Papier entspricht in den optischen Eigenschaften einer Papierqualität, welche keinen optischen Aufheller enthält.

Mit gleichem Erfolg werden die in den übrigen Herstellungsbeispielen beschriebenen Fluoreszenzlöscher eingesetzt.

Anwendungsbeispiel D

10 g gebleichtes Nessel-Baumwollgewebe wird im Flottenverhältnis 1:20 30 Minuten bei 50 °C mit einer Lösung von 0,1 % BLANKOPHOR ® BA 267 % behandelt. Nach dem Spülen und Trocknen wird ein Weißgrad nach Berger von 150 gemessen (Grundweiß ca. 80).

Das so weißgetönte Gewebe wird anschließend im Flottenverhältnis 1:20 30 Minuten bei 50 °C mit 0,1 % des im Herstellungsbeispiel 112 beschriebenen Fluoreszenzlöschers behandelt. Nach dem Spülen und Trocknen wird wiederum der Weißgrad nach Berger bestimmt. Er liegt im Bereich von 100 bis 110. Visuell ist nur noch ein geringer Aufhelleffekt wahrnehmbar.

Mit ähnlichem Erfolg können auch andere in den Herstellungsbeispielen 1 bis 133 beschriebene Verbindungen eingesetzt werden.

Anwendungsbeispiel E

Die Möglichkeit, unerwünschte Restanteile von wäßrigen Lösungen anionischer Weißtöner in Färbeapparaturen, Färbemaschinen, Vorratsgefäßen und Zuleitungen unter Verwendung von Fluoreszenzlöschern der Beispiele 1 bis 145 unwirksam zu machen, sei an folgendem quantitativen Modellversuch gezeigt:

a) 0,03 mMol eines optischen Aufhellers der Formel

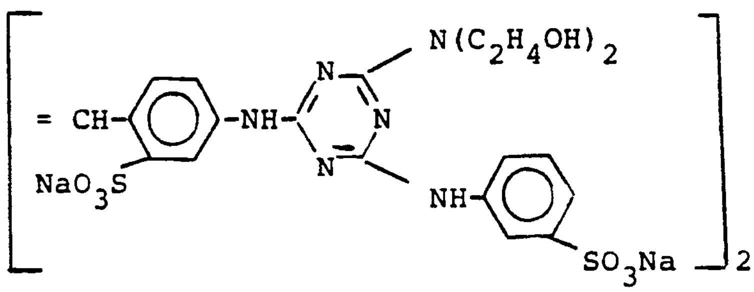

(42,2 mg 86,5 %ig = 36,5 mg 100 %ig werden in 100 ml Wasser gelöst.

b) 0,1 mMol Fluoreszenzlöscher der Formel 29 (85,4 mg) werden in 100 ml Wasser gelöst.

c) 5 ml der unter a) hergestellten Lösung werden auf 100 ml aufgefüllt.

d) 5 ml der unter a) und x ml der unter b) hergestellten Lösung werden vermischt und auf 100 ml aufgefüllt (x = 1, 2, 3, 4).

In einem Zeiss-Spektralphotometer DMR 21 mit 1 ml Quarzküvette und Fluoreszenzzusatzgerät ZF M4 (450 W Xenonlampe) mißt man nacheinander unter Fluoreszenzanregung mit UV-Licht von 345 nm die Intensität der Fluoreszenzemissionen der folgenden Lösungen bei 436 nm:

Lösung c) <1,5 .$10^{-5}$ Mol/l Aufh.) 100 % Fluoresz. Intensität
Lösung d),x = 1 ( $10^{-5}$ Mol/h 29) 59 % Fluoresz. Intensität
Lösung d), x = 2 (2,0 . $10^{-5}$ Mol/l 29 ) 27% Fluoresz. Intensität
Lösung d),x = 3 (3,0 .$10^{-5}$ Mol/l 29) 3 % Fluoresz. Intensität
Lösung d),x = 4 (4,0 . $10^{-5}$ Mol/l 29) 0 % Fluoresz. Intensität

Der Versuch zeigt, daß Fluoreszenzlöscher 29 den oben aufgeführten optischen Aufheller in verdünnter wäßriger Lösung bei Raumtemperatur bereits bei einem Molverhältnis von 2:1 weitgehend, bei einem Molverhältnis von 2,7:1 völlig unwirksam macht.

Setzt man anstelle von Verbindung 29 eine der in DE-A 1 912 647 oder 2 448 293 beschriebenen Verbindung ein, so wird eine völlige Fluoreszenzlöschung - auch bei noch höheren Anwendungskonzentrationen des Löschers - nicht erreicht.

Setzt man anstelle von Verbindung 29 den Fluoreszenzlöscher der Formel 89 ein, so wird die völlige Fluoreszenzlöschung bereits bei einem Molverhältnis von 4:3 (Löscher/opt. Aufheller) erreicht.

## Ansprüche

1. Verfahren zur Löschung der durch anionische optische Aufheller erzeugten Fluoreszenz durch Einwirkung von praktisch farblosen, wasserlöslichen kationischen oder amphoteren Verbindungen, dadurch

gekennzeichnet, daß die Verbindungen mindestens eine Ammoniumgruppe, mindestens zwei Aryl-, Arylen-, Hetaryl- und/oder Hetarylengruppen und mindestens eine Nitrogruppe im Molekül enthalten.

2. Verfahren nach Anspruch 1, dadurch gekennzeichnet, daß die Verbindungen 2 bis 6 Ammoniumgruppen, die acyclisch sind oder in gesättigte, teilgesättigte oder heteroaromatische Ringe als Ringglied eingebunden sind, 2 bis 12 ein- oder zweibindige Gruppen der Benzol-, Furan-, Thiophen-, Pyrrol-, Pyrazol-, Oxazol-, Thiazol-, 1,2,4-Oxadiazol-, 1,3,4-Oxadiazol-, 1,3,4-Thiadiazol-, Imidazol-, 1,2,4-Triazol-, Pyran-, Pyridin-, Pyrimidin-, Pyrazin- oder s-Triazin-Reihe, die durch 5- oder 6-gliedrige aromatische oder cycloaliphatische Ringe anelliert und/oder durch nicht-ionische Reste substituiert sein können, und 2 bis 9 Nitrogruppen oder - falls es sich um oligomere oder polymere Verbindungen handelt - pro wiederkehrende Einheit 1 - 2 Ammoniumgruppen der obengenannten Art, 1 - 3 Aryl- oder Arylenreste und 1 - 3 Nitrogruppen enthalten.

3. Verfahren nach Anspruch 1, dadurch gekennzeichnet, daß die Verbindungen die allgemeine Formel
$(O_2N)_n[(\overset{\oplus}{K}{}^1-Z^2-Y^2-X^2-G-)_r W]$ (r-a) . $An^{\ominus}$
besitzen, worin

$\overset{\oplus}{K}{}^1$ für $\overset{\oplus}{E}{}^1$ oder $A-X^1-Y^1-Z^1-\overset{\oplus}{E}-$
A für einen Benzol-, Biphenyl-, Naphthalin-, Furan-, Benzofuran-, Thiophen-, Benzothiophen-, Thiazol-, Benzthiazol-, Benzimidazol-, 1,3,4-Thiadiazol- oder Indol-Rest, der außer durch Nitro auch durch $C_1$-$C_4$-Alkyl , Halogen, Carbamoyl, Sulfamoyl, Cyan, Hydroxy, $C_1$-$C_4$-Alkoxy oder eine Sulfonsäuregruppe substituiert sein kann,
$X^1$ für $-(CH_2)t-CO-$, $-CH_2-Y^1$ $-CO-$, $-(CH_2)_t-SO_2-$, $-O-CH_2-CO-$, $-O-CH_2-SO_2-$, $-S-CH_2-CO-$, $-S-CH_2-SO_2-$oder eine Einfachbindung,
t für 0, 1 oder 2,
$X^2$ für -CO-, -CO-NH-CO- oder $-SO_2-$ oder eine Einfachbindung,
$Y^1$ und $Y^2$ je für -O-, -S-, -N(R)-, -N(R)-NH- oder eine Einfachbindung,
R für Wasserstoff, $C_1$-$C_2$-Alkyl oder Cyanethyl oder in $Y^1$ und $Y^2$ auch für -CO- oder $-SO_2-$, die mit der o- oder peri-Stellung von A bzw. eines Benzolringes W verbunden sein können,
$Z^1$ und $Z^2$ je für $C_1$-$C_6$-Alkylen, p-Benzylen, p-Xylylen oder eine Einfachbindung stehen.

$E^{\oplus}$ für $-\overset{\oplus}{N}(R^1R^2)-$, 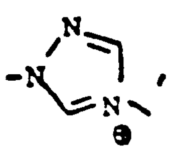, 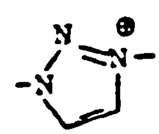; 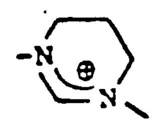,

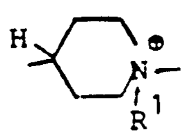 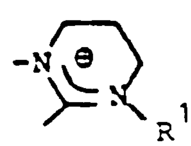, 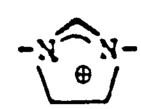, 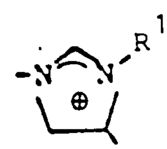

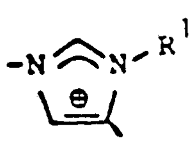 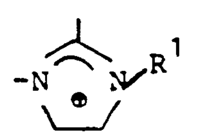, 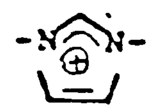 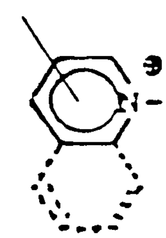

stehen, wobei diese Ringe gegebenenfalls durch 1 - 4 Methylgruppen substituiert sein können, und jede der beiden freien Valenzen an $-X^1-Y^1-Z^1-$ oder an $-Z^2-Y^2-X^2-G-$ gebunden sein kann,

$\overset{\oplus}{E}{}^1$ für $-\overset{\oplus}{N}(R^1R^2R^2)$, 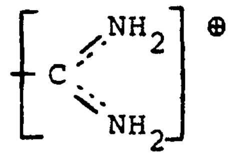 , 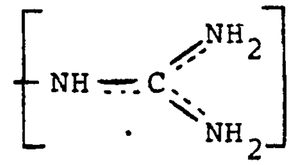

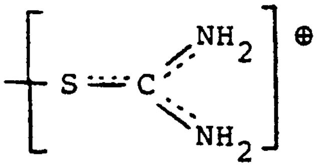 , 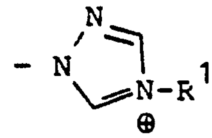 , 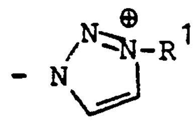 ,

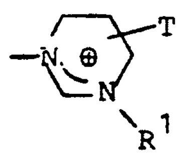 , 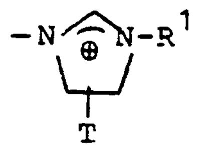 , 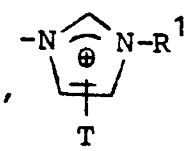 , 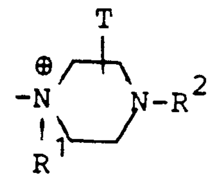 ,

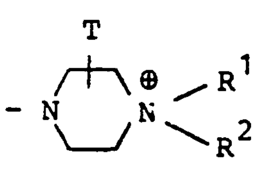 , 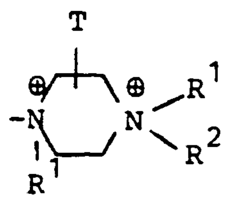 , 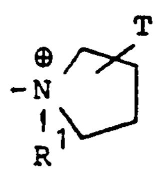 ,

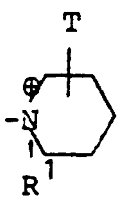 , 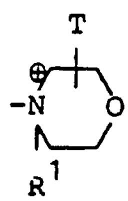 , 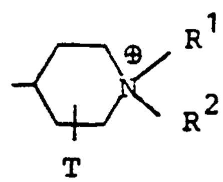 ,

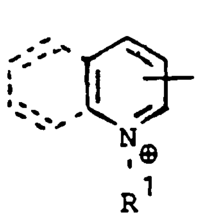 , 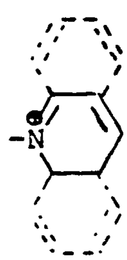 , 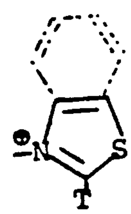 oder 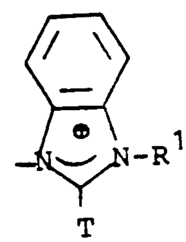

T für Wasserstoff oder - in Abhängigkeit von der Art des Ringes - für 1-4 Methylgruppen stehen, worin die aromatischen Reste außer durch 1 - 2 Nitro auch durch $C_1$-$C_4$-Alkyl, Halogen, Cyan oder Carbamoyl und die Amidinium-, Guanidinium- und Thiuronium-Reste auch durch 1-2 Reste $R^2$ substituiert sein können,

$R^1$ und $R^2$ unabhängig voneinander für Wasserstoff, $C_1$-$C_4$-Alkyl , das durch OH, $NH_2$, Halogen, $C_1$-$C_4$-Alkoxy, COOH, $CONH_2$, CN oder $C_1$-$C_4$-Alkoxy-carbonyl substituiert sein kann, $C_2$-$C_4$-Alkenyl, Cyclohexyl, Phenyl-$C_1$-$C_3$-alkyl oder Benzoylmethyl, die außer durch Nitro auch durch $C_1$-$C_4$-Alkyl, $C_1$-$C_4$-Alkoxy, Cl oder Br kernsubstituiert sein können,

$R^1$ außerdem für $-O^{\ominus}$ oder A-$X^1$-$Y^1$-$Z^1$-,

R und $R^1$ auch ringgeschlossen sind und dann zusammen mit -N-$Z^1$-N- oder -N-$Z^2$-N- einen gegebenenfalls durch 1 bis 2 Methylgruppen substiuierten Piperazin-Rest bilden, oder

$R^1$ und $R^2$ auch ringgeschlossen sind und dann zusammen mit dem Stickstoffatom, an das sie gebunden sind, einen gegebenenfalls durch 1 bis 4 Methylgruppen substituierten Pyrrolidin-, Piperidin-, Morpholin-oder Piperazin-Ring bilden, der am zweiten N-Atom durch gegebenenfalls

durch OH oder $NH_2$ substituiertes $C_1$- bis $C_3$-Alkyl substituiert sein kann,
G für -NH-, $-NH-CH_2-$, -O- oder eine Einfachbindung,
W für einen r-wertigen Rest aus der Reihe Benzol, Naphthalin, Anthracen, Phenanthren, 9,10-Dihydro-phenanthren, Cyclohexan, Fluoren-9-on(3,6), Thiophen(2,5), Dibenzofuran(3,6), Dibenzothiophen(3,6) , Dibenzothiophen-S-dioxid(2,7), 9-H-Thioxanthen-S-dioxid(3,6), Carbazol(3,6), 9-H-Xanthen-9-on(2,7) , 9-Acridon(2,7) , 1,3,4-Oxadiazol (2,5), 1,2,4-Oxadiazol-(3,5) , 1 ,3,4-Thiadiazol(2,5) , s-Triazin(2,4,6), Piperazin(1 ,4), 1 ,2-Dihydro-1,2,4,5-tetrazin(3,6) oder für einen Rest der Formeln

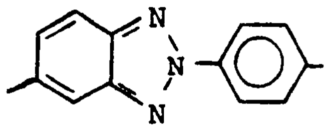 , 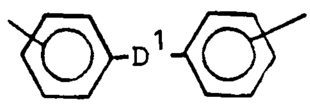 , 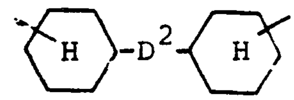 ,

oder,

für den Fall, daß $-\overset{\oplus}{E}$ - einen zweibindigen Pyridinium-oder Chinolinium-Rest bedeutet, auch für eine Einfachbindung,
$D^1$ und $D^2$ unabhängig voneinander für einen geradkettigen oder verzweigten, gegebenenfalls durch -O- unterbrochenen $-C_1-C_7$-Alkylenrest, $-O-C_2-C_4$-Alkylen-O-, -O-, -NH-, -N ($C_1-C_2$-alkyl), -CO-, -CO-NH-, -NH-CO-NH-, 1,1-Cyclohexylen oder eine direkte Bindung,
$D^1$ außerdem für $-CH(C_6H_5)-$, $-CH(C_6H_4-)-$, $-N(C_6H_5)-$, -CH=CH-, -S-, $SO_2$-, -SO-, m- oder p-Phenylen, Thiophen(2,5), 1,3,4-Oxadiazol(2,5), 1,3,4-Thiadiazol(2,5), Oxazol(2,5), Thiazol(2,5), 1,2-Dihydro-1,2,4,5-tetrazin(3,6) oder

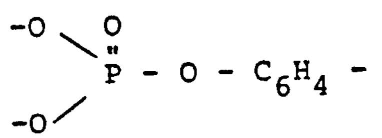 ,

$D^2$ außerdem für $-CH(C_6H_{11})-$ oder $-CH(C_6H_{10}-)-$. Wo-bei die unter W, D und $D^2$ genannten Ringe außer durch Nitro auch durch $C_1-C_4$-Alkyl , $C_1-C_4$-Alkoxy, Halogen, Nitro und/oder eine Sulfonsäuregruppe substituiert sein können,
n für eine ganze Zahl von 1 - 6,
a der Anzahl der anionischen und/oder $\overset{\oplus}{N}-\overset{\ominus}{O}$ -Gruppen entsprechend 0, 1, 2 oder 3
r für 2 oder 3, wobei r > a ist, und
$An^{\ominus}$ falls vorhanden, für ein farbloses Anion stehen, und worin die r-fach vorhandenen Ketten gleich oder verschieden sein können,
und worin im Falle r = 2 die Gruppierung $-\overset{\oplus}{E}-Z^2-Y^2-X^2-G-W-G-X^2-Y^2-Z^2-\overset{\oplus}{E}$ - insgesamt auch für

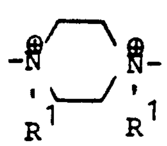 oder 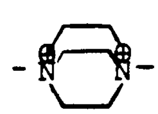 stehen kann.

4. Verfahren nach Anspruch 1, dadurch gekennzeichnet, daß die Verbindungen der allgemeinen Formel

$$(O_2N)_n - [-(A-X^1-Y^1-Z^1-Y')_e - W^1 - (G^2-Y^2-Z^2-\overset{\oplus}{E}{}^1)_f]\,(An^{\ominus})_x$$

entsprechen, worin

Y' für -O-, -S-, -NH-, $-N(CH_3)-$ oder eine Einfachbindung,

e für 1, 2 oder, wenn $W^1$ und/oder $\overset{\oplus}{E}{}^1$ insgesamt mindestens eine Nitrogruppe enthalten und $W^1 \neq W$ ist, auch für O,

f für 2 oder 3,

x je nach der Anzahl der anionischen und/oder $-\overset{\oplus}{N}-\overset{\ominus}{O}$ -Gruppen im Molekül und abhängig von f, für 0, 1, 2 oder 3

$W^1$ für 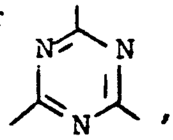 , 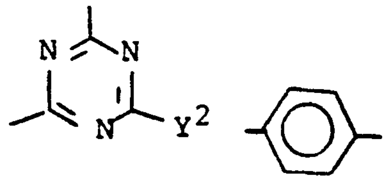 ,

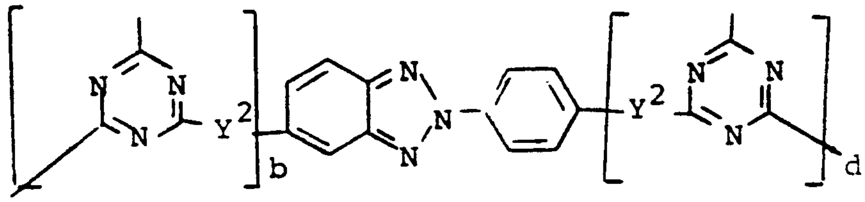 ,

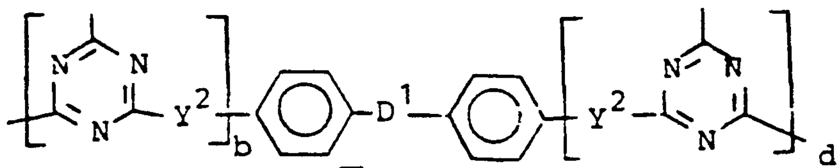 ,

oder 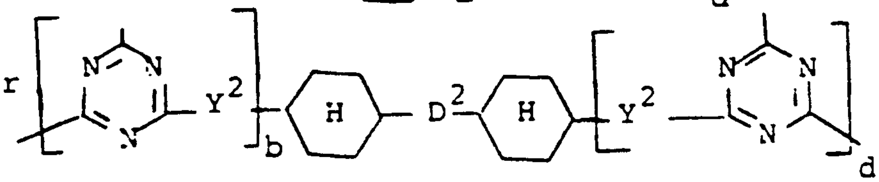

b und d für 0 oder 1,

$G^2$ für -NH-CO- oder eine Einfachbindung stehen und worin

n, A, $\overset{\oplus}{E}{}^1$, $X^1$, $Y^1$, $Y^2$, $Z^1$, $Z^2$, $R^1$, $R^2$, x und $An^{\ominus}$ die gleiche Bedeutung wie in Anspruch 3 besitzen.

**5.** Verfahren nach Anspruch 1, dadurch gekennzeichnet, daß oligomere oder polymere Verbindungen verwendet werden, welche pro wiederkehrende Einheit 1-3 Nitrogruppen und 1-2 kationische $\overset{\oplus}{E}{}^1$-Gruppen enthalten, wobei $\overset{\oplus}{E}{}^1$ die gleiche Bedeutung wie in Anspruch 4 besitzt, dadurch gekennzeichnet, daß sie erhalten werden:

a) durch Polymerisation oder Mischpolymerisation von einem oder mehreren Monomeren aus der Gruppe Maleinsäureanhydrid

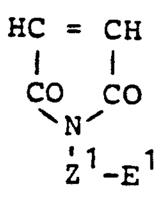 , 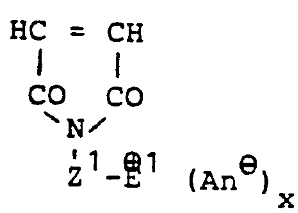

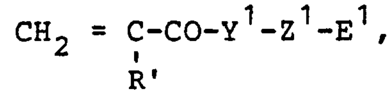

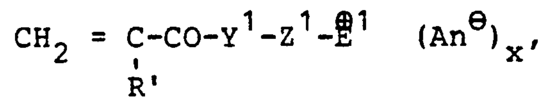

N-Allylpyridimiumhalogenid oder dessen Addukt mit einem Nitrobenzaldehyd, $E^1\text{-}CH=CH_2$ wie 2-oder 4-Vinylpyridin und gegebenenfalls zusätzlich Vinylchlorid, Vinylacetat, Styrol, ∝-Methylstyrol oder 4,4'-Divinylbenzol, die durch Methyl, Chlor, $E^1(CH_2)v$-, $\overset{\oplus}{E}{}^1(CH_2)v$- und/oder Nitro kernsubstituiert sein können,

wobei R' für Wasserstoff oder Methyl,
$E^1$ für eine durch Umsetzung mit $R^1L$ in $-\overset{\oplus}{E}{}^1$ überführbare basische Gruppe,
L für eine als Anion abspaltbare Gruppe und
v für 0 oder 1 stehen und $Y^1$. $Z^1$, $E^{\oplus 1}$, $An^{\ominus}$, X und $R^1$ die oben angegebene Bedeutung haben,
für den Fall, daß Maleinsäureanhydrid als Monomeres eingesetzt wird, durch weitere Umsetzung mit einer Verbindung der Formel
$HN(R)\text{-}Z^1\text{-}E^1$
oder
$HN(R)\text{-}Z^1\text{-}\overset{\oplus}{E}{}^1\ (An^{\ominus})_x$.
worin R, $Z^1$, $E^1$, $\overset{\oplus}{E}{}^1$, $An^{\ominus}$ und x die oben angegebene Bedeutung haben,
und falls noch nichtkationische $E^1$-Gruppen vorliegen, durch deren weitere Umsetzung mit $R^1$-L, worin $R^1$ und L die oben angegebene Bedeutung haben, sowie gegebenenfalls durch anschließende Mono- oder Dinitrierung vorhandener aromatischer Gruppen oder
b) durch Polykondensation von Di-($C_2$-$C_3$-alkylen)-tri-aminen oder Tri-($C_2$-$C_3$-alkylen)-tetraminen der Formel
$H_2N\{(CH_2)_k\text{-}E\}_w(CH_2)_k\text{-}NH_2$
worin

k für 2 oder 3,
w für 1 oder 2 und
-E- für eine durch Umsetzung mit $R^1$-L in $-\overset{\oplus}{E}$ -überführbare basische Gruppe stehen, mit Dicarbonsäuren der Formel

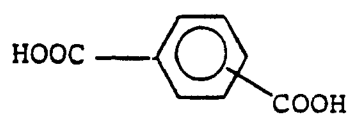

und/oder $HOOC\text{-}(CH_2)_g\text{-}COOH$

oder ihren Derivaten (wie Säurechloriden, Anhydriden, Niederalkylestern, Imino-niederalkylesterhydrochloriden onder Nitrilen),
worin g für eine ganze Zahl von 0 bis 6 steht, durch weitere Umsetzung mit $R^1$-L,
worin $R^1$ und L die oben angegebene Bedeutung haben,
sowie gegebenenfalls anschließende Mono- oder Dinitrierung vorhandener aromatischer Gruppen.

6. Verfahren nach Anspruch 5, dadurch gekennzeichnet, daß Verbindungen verwendet werden, die wiederkehrende Einheiten der Formel

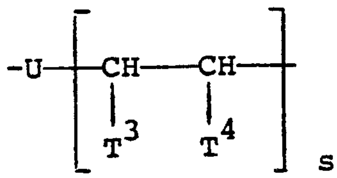 oder

$-[NH-(CH_2)_k-B^1-CO]_z-B^2-$

enthalten, worin

U für 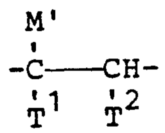 , 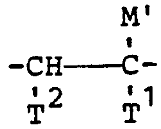 oder 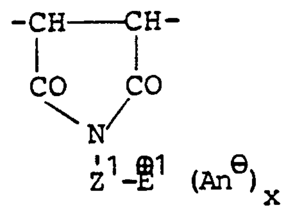

s für 0 oder 1,
M' für Wasserstoff oder Methyl,

$T^1$ für $-CO-N(R)-Z^1-E^{\oplus 1}$ $(An^{\ominus})_x$ oder

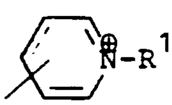 $-R^1$ $(An^{\ominus})_x$ ,

$T^2$ für $T^1$, COOH oder

- im Falle $T^1$ = 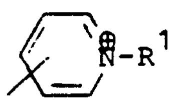 $-R^1$ $(An^{\ominus})_x$ - für Wasserstoff,

und einer der Reste $T^3$ und $T^4$ für Phenyl, Tolyl oder Chlorphenyl, die durch Nitro substituiert sein können, und der andere für Wasserstoff oder Methyl.

$B^1$ 1) für 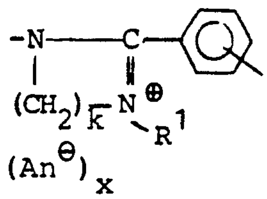 oder

2) für 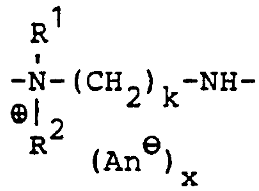

$B^2$ - falls $B^1$ die unter 1) angegebene Bedeutung hat und z = O oder 1 ist - für

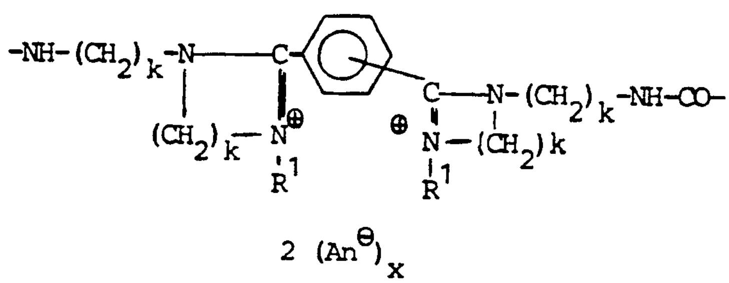 und

- falls $B^1$ die unter 2) angegebene Bedeutung hat und z = 1 ist - für 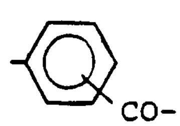 oder

$-(CH_2)_g-CO-$,

k = für 2 oder 3,
g = für eine ganze Zahl von 1 bis 6, x, $Z^1$, $E^1$, $R^1$, $R^2$ und $An^{\ominus}$ die gleiche Bedeutung wie Anspruch in 4 besitzen, die Anzahl der sauren und/ oder - $\overline{O}|^{\ominus}$ -Gruppen gleich oder kleiner ist als die der kationischen Ladungen und worin pro wiederkehrende Einheit 1-3 aromatisch gebundene Nitrogruppen vorhanden sind.

7. Nitroverbindungen der allgemeinen Formel $(O_2N)_n\{(A-X^1-Y^1-Z^1-\overset{\oplus}{E}-Z^2-Y^2-X^2-G)_r w\}$ (r-a).$An^{\ominus}$ worin

A für einen Benzol-, Biphenyl-, Naphthalin-, Furan-, Benzofuran-, Thiophen-, Benzothiophen-, Thiazol-, Benzthiazol-, Benzimidazol-, 1,3,4-Thiadiazol- oder Indol-Rest, der außer durch Nitro auch durch $C_1$-$C_4$-Alkyl , Halogen, Carbamoyl, Sulfamoyl, Cyan, Hydroxy, $C_1$-$C_4$-Alkoxy oder eine Sulfonsäuregruppe substituiert sein kann,
$x^1$ für ( $CH_2)_t$-CO-, $-CH_2-Y^1-CO-$, $(CH_2)_t-SO_2-$, $-O-CH_2-CO-$, $-O-CH_2-SO_2-$, $-S-CH_2-CO-$, $-S-CH_2-SO_2-$ oder eine Einfachbindung,
t für 0, 1 oder 2,
$X^2$ für -CO-, -CO-NH-CO- oder $-SO_2-$ oder eine Einfachbindung,
$Y^1$ und $Y^2$ je für -O-, -S-, -H(R)-, -N(R)-NH- oder eine Einfachbindung,
R für Wasserstoff, $C_1$-$C_2$-Alkyl oder Cyanethyl oder in $Y^1$ und $Y^2$ auch für -CO- oder $-SO_2-$, die mit

der o- oder peri-Stellung von A bzw. eines Benzolringes W verbunden sein können, $Z^1$ und $Z^2$ je für $C_1$-$C_6$-Alkylen, p-Benzylen, p-Xylylen oder eine Einfachbindung,

$E^{\oplus}$ für $-\overset{\oplus}{N}(R^1R^2)-$, 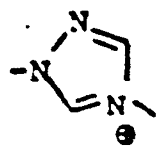, 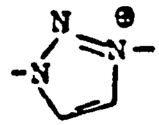, 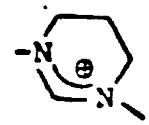,

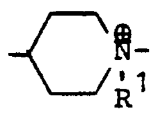, 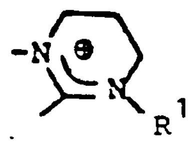, 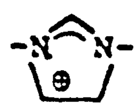, 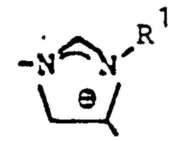,

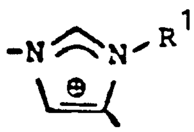 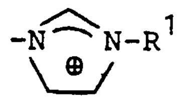, 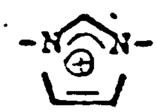 oder

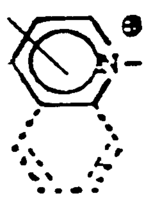

stehen, wobei diese Ringe gegebenenfalls durch 1 - 4 Methylgruppen substituiert sein können, und jede der beiden freien Valenzen an $-X^1-Y^1-Z^1-$ oder an $-Z^2-Y^2-X^2-G-$ gebunden sein kann,

$E^{\oplus 1}$ für $-\overset{\oplus}{N}(R^1R^2R^2)$, 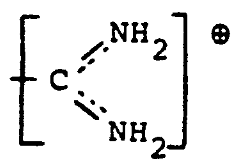, 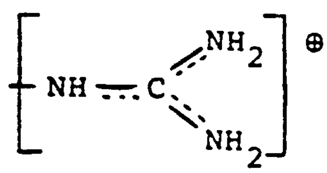,

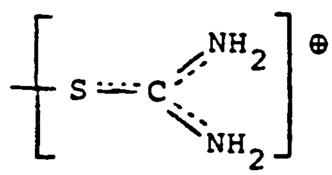, 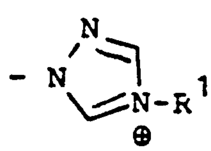, 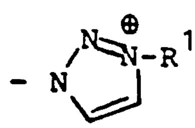,

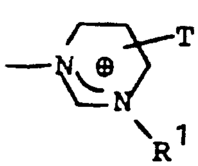, 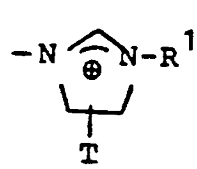, 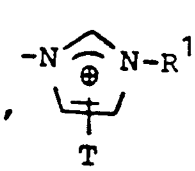, 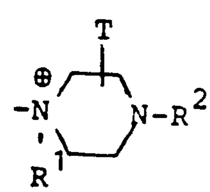,

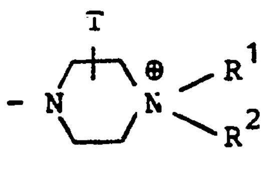, 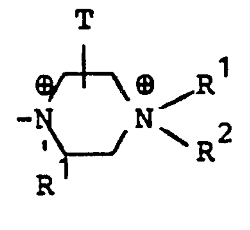, 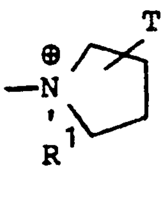,

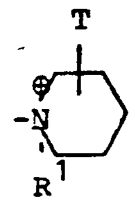, 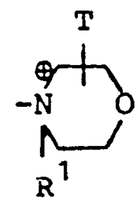, 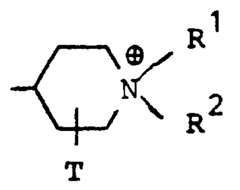,

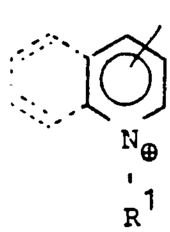, 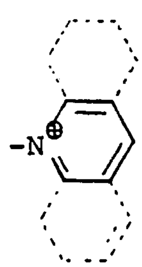, 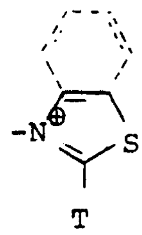, 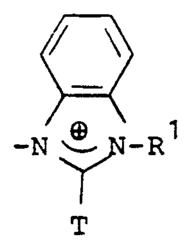

T für Wasserstoff oder - in Abhängigkeit von der Art des Ringes - für 1-4-Methylgruppen stehen worin die aromatischen Reste außer durch 1-2 Nitro auch durch $C_1$-$C_4$-Alkyl , Halogen, Cyan oder Carbamoyl und die Amidinium-, Guanidinium- und Thiuronium-Reste auch durch 1-2 Reste $R^2$ substituiert sein können,

$R^1$ und $R^2$ unabhängig voneinander für Wasserstoff, $C_1$-$C_4$-Alkyl , das durch OH, $NH_2$, Halogen, $C_1$-$C_4$-Alkoxy, COOH, $CONH_2$, CN oder $C_1$-$C_4$-Alkoxy-carbonyl substituiert sein kann, $C_2$-$C_4$-Alkenyl, Cyclohexyl , Phenyl-$C_1$-$C_3$-alkyl oder Benzoylmethyl, die außer durch Nitro, auch durch $C_1$-$C_4$-Alkyl, $C_1$-$C_4$-Alkoxy, Cl oder Br kernsubstituiert sein können,

$R^1$ außerdem für - $O^{\ominus}$ oder A-$X^1$-$Y^1$-$Z^1$-,

R und $R^1$ auch ringgeschlossen sind und dann zusammen mit -N-$Z^1$-N- oder -N-$Z^2$-N- einen gegebenenfalls durch 1 bis 2 Methylgruppen substituierten Piperazin-Rest bilden, oder

$R^1$ und $R^2$ auch ringgeschlossen sind und dann zusammen mit dem Stickstoffatom, an das sie gebunden sind, einen gegebenenfalls durch 1 bis 4 Methylgruppen substituierten Pyrrolidin-, Piperidin-, Morpholin-oder Piperazin-Ring bilden, der am zweiten N-Atom durch gegebenenfalls durch OH oder $NH_2$ substituiertes $C_1$- bis $C_3$-Alkyl substituiert sein kann,

G für -NH-, -NH-$CH_2$-, -O- oder eine Einfachbindung,

W für einen r-bindigen Rest aus der Reihe Benzol, Naphthalin, Anthracen, Phenanthren, 9,10-Dihydro-phenanthren, Cyclohexan, Fluoren-9-on(3,6), Thiophen(2,5), Dibenzofuran(3,6), Dibenzothiophen(3,6), Dibenzothiophen-S-dioxid(2,7), 9-H-Thioxanthen-S-dioxid(3,6), Carbazol(3,6), 9-H-Xanthen-9-on(2,7),9-Acridon(2,7) , 1,3,4-Oxadiazol (2,5) , 1,2,4-Oxadiazol(3,5) , 1,3,4-Thiadiazol-(2,5) , s-Triazin(2,4, 6), Piperazin(1,4), 1,2-Dihydro-1,2,4,5-tetrazin(3,6) oder für einen Rest der Formeln

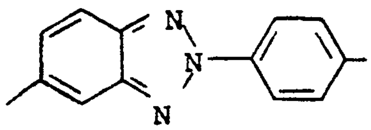 , 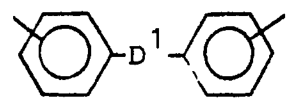 oder

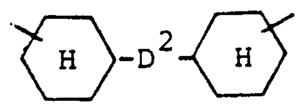 ,

oder für den Fall, daß -$\overset{\oplus}{E}$- einen zweibindigen Pyridinium-oder Chinolinium-Rest bedeutet, auch für eine Einfachbindung,
$D^1$ und $D^2$ unabhängig voneinander für einen geradkettigen oder verzweigten, gegebenenfalls durch -O- unterbrochenen $C_1$-$C_7$-Alkylenrest , -O-$C_2$-$C_4$-Alkylen-O-, -O-, -NH-, -N($C_1$-$C_2$-alkyl)-, -CO-, -CO-NH-, -NH-CO-NH-, 1,1-Cyclohexylen oder eine direkte Bindung,
$D^1$ außerdem für -CH($C_6H_5$)-, -CH($C_6H_4$)-, -N($C_6H_5$)-, -CH=CH-, -S-, -$SO_2$-, -SO-, m- oder p-Phenylen, Thiophen(2,5), 1,3,4-Oxadiazol(2,5), 1,3,4-Thiadiazol(2,5), Oxazol(2,5), Thiazol(2,5), 1,2-Dihydro-1,2,4,5-tetrazin(3,6) oder

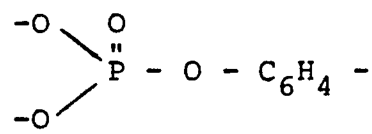 ,

$D^2$ außerdem für -CH($C_6H_{11}$)- oder -CH($C_6H_{10}$-)-, wobei die unter W, D und $D^2$ genannten Ringe außer durch Nitro auch durch $C_1$-$C_4$-Alkyl , $C_1$-$C_4$-Alkoxy , Halogen und/oder eine Sulfonsäuregruppe substituiert sein können,
n für eine ganze Zahl von 1 bis 6,
a der Anzahl der anionischen und/oder $\overset{\oplus}{N}$-$\overset{\ominus}{O}$-Gruppen entsprechend 0, 1, 2 oder 3
r für 2 oder 3, wobei r > a ist, und $An^{\ominus}$ falls vorhanden, für ein farbloses Anion stehen,
und worin die r-fach vorhandenen Ketten gleich oder
verschieden sein können,
und worin im Falle r = 2 die Gruppierung
-$\overset{\oplus}{E}$-$Z^2$-$Y^2$-$X^2$-G-W-G-$X^2$-$Y^2$-$Z^2$-$\overset{\oplus}{E}$- insgesamt auch für

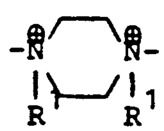 oder 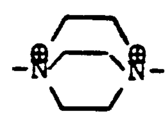 stehen kann,

mit der Einschränkung, daß Z verschieden von -$CH_2$-ist, wenn $X^2$, $Y^2$ und G für eine Einfachbindung, r für 2 und W für Phenylen oder Cyclohexylen stehen.

8. Nitroverbindungen der allgemeinen Formel $(O_2N)_n\overset{\oplus}{E}(A\text{-}X^1\text{-}Y^1\text{-}Z^1\text{-}Y')_e\ W^1\text{-}(G^2\text{-}Y^2\text{-}Z^2\text{-}\overset{\oplus}{E}{}^1)_f^{\oplus}\ (An^{\ominus})_x$
worin

Y' für -O-, -S-, -NH-, -N($CH_3$)- oder eine Einfachbindung,
e für 1, 2 oder, wenn $W^1$ und/oder $\overset{\oplus}{E}{}^1$ insgesamt mindestens eine Nitrogruppen enthalten und $W^1 \neq$ W ist, auch für 0,
f für 2 oder 3,
x je nach der Anzahl der anionischen und/oder -$\overset{\oplus}{N}$-$\overset{\ominus}{O}$-Gruppen im Molekül und abhängig von f, für 0, 1, 2 oder 3

$W^1$ für 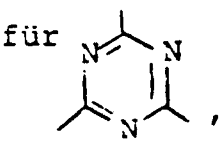 , 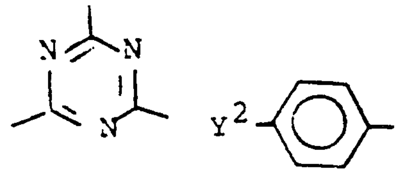 ,

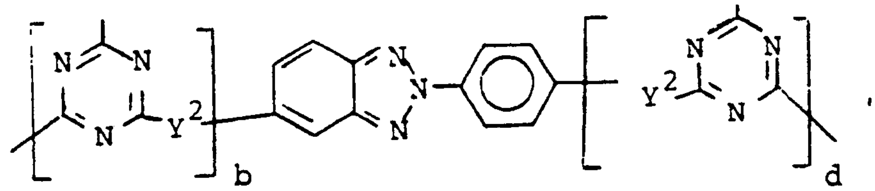 ,

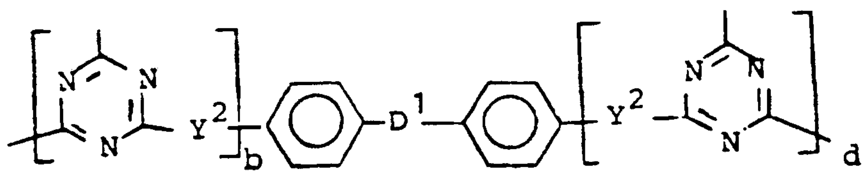 ,

oder 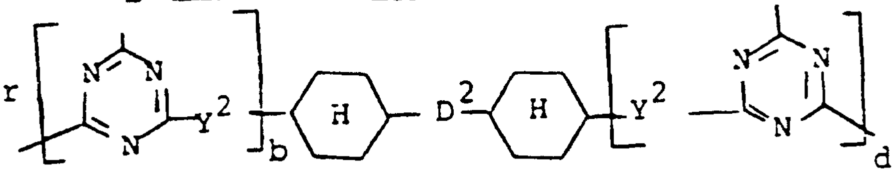

b und d für 0 oder 1, $G^2$ für -NH-CO- oder eine Einfachbindung stehen und worin n, A, $E^{\oplus 1}$, $X^1$, $Y^1$, $Y^2$, $Z^1$, $Z^2$, $R^1$, $R^2$, x und $An^{\ominus}$ die in Anspruch 4 angegebene Bedeutung haben.

9. Nitroverbindungen, die wiederkehrende Einheiten der Formel

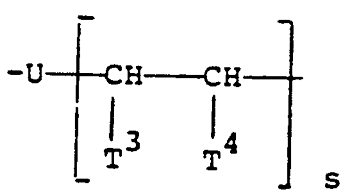

oder

$$-[-NH-(CH_2)_k-B^1-CO-]_z-B^2-$$

enthalten, worin

U für 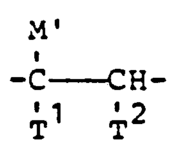 , 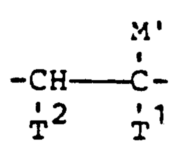 oder 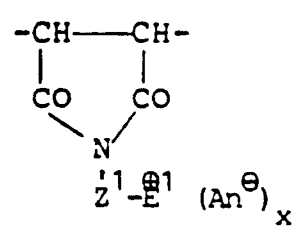

s für 0 oder 1, M' für Wasserstoff oder Methyl,

$T^1$ für $-CO-N(R)-Z^1-\overset{\oplus}{E}{}^1$ $(An^{\ominus})_x$ oder

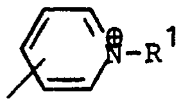 $(An^{\ominus})_x$ ,

$T^2$ für $T^1$, COOH oder

- im Falle $T^1$ = 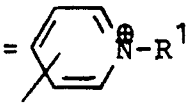 $(An^{\ominus})_x$ - für Wasserstoff,

und einer der Reste $T^3$ und $T^4$ für Phenyl, Toluyl oder Chlorphenyl, die durch Nitro substituiert sein können, und der andere für Wasserstoff oder Methyl,

$B^1$ 1) für 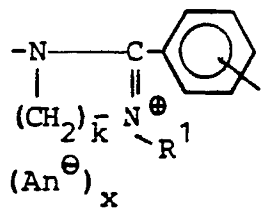 oder

2) für 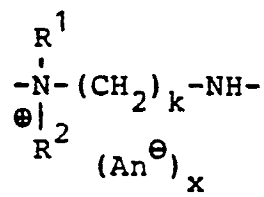

$B^2$ - falls $B^1$ die unter 1) angegebene Bedeutung hat und z = O oder 1 ist - für

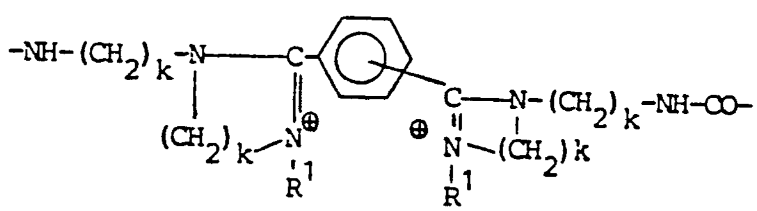 und

2 $(An^{\ominus})_x$

- falls $B^1$ die unter 2) angegebene Bedeutung hat und z = 1 ist - für 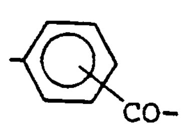 oder

$-(CH_2)_g-CO-$,

k = für 2 oder 3,

g = für eine ganze Zahl von 1 bis 6, x, $Z^1$, $E^1$, $R^1$, $R^2$ und $An^{\ominus}$ die gleiche Bedeutung wie in Anspruch 4 besitzen, die Anzahl der sauren und/ oder - $\bar{O}$ :$^{\ominus}$-Gruppen gleich oder kleiner ist als die der kationischen Ladungen und worin pro wiederkehrende Einheit 1-3 aromatisch gebundene Nitrogruppen vorhanden sind.

## Claims

1. Process for quenching the fluorescence produced by anionic fluorescent brighteners through the action of virtually colourless, water-soluble cationic or amphoteric compounds, characterized in that the compounds contain in the molecule at least one ammonium group, at least two aryl, arylene, hetaryl and/or hetarylene groups and at least one nitro group.

2. Process according to Claim 1, characterized in that the compounds contain 2 to 6 ammonium groups which are acyclic or are members of saturated, partially saturated or heteroaromatic rings, 2 to 12 monodentate or bidentate groups of the benzene, furan, thiophene, pyrrole, pyrazole, oxazole, thiazole, 1,2,4-oxadiazole, 1,3,4-oxadiazole, 1,3,4-thiadiazole, imidazole, 1,2,4-triazole, pyran, pyridine, pyrimidine, pyrazine or striazine series which can be fused with 5- or 6-membered aromatic or cycloaliphatic rings and/or substituted by non-ionic radicals, and 2 to 9 nitro groups or - if they are oligomeric or polymeric compounds - per repeat unit 1 - 2 ammonium groups of the abovementioned type, 1 - 3 aryl or arylene radicals and 1 - 3 nitro groups.

3. Process according to Claim 1, characterized in that the compounds have the general formula

$$(O_2N)_n \left[ (\overset{\oplus}{K}{}^1-Z^2-Y^2-X^2-G-)_r \; W \right] \quad (r-a) \; . \; An^{\ominus}$$

wherein

$\overset{\oplus}{K}{}^1$ represents $\overset{\oplus}{E}{}^1$- or A-$X^1$-$Y^1$-$Z^1$-$\overset{\oplus}{E}$ -.
A represents a benzene, biphenyl, napthalene, furan, benzofuran, thiophene, benzothiophene, thiazole, benzothiazole, benzimidazole, 1,3,4-thiadiazole or indole radical which, in addition to nitro, can also be substituted by $C_1$-$C_4$-alkyl , halogen, carbamoyl, sulphamoyl, cyano, hydroxyl, $C_1$-$C_4$-alkoxy or a sulpho group,
$x^1$ represents -$(CH_2)_t$-CO-, -$CH_2$-$Y^1$-CO-, -$(CH_2)_t$-$SO_2$,-, -O-$CH_2$-CO-, -O-$CH_2$-$SO_2$-, -S-$CH_2$-CO-, -S-$CH_2$-$SO_2$- or a single bond,
t represents 0, 1 or 2,
$x^2$ represents -CO-, -CO-NH-CO-, -$SO_2$,- or a single bond,
Y' and $Y^2$ each represent -0-, -S-, -N(R)-, -N(R)-NH-or a single bond,
R represents hydrogen, $C_1$-$C_2$,-alkyl or cyanoethyl or, in Y' and $Y^2$, also represents -CO- or -$SO_2$-, each of which can be bonded to the o- or periposition of A or of a benzene ring W,
Z' and $Z^2$ each represent $C_1$,-$C_6$-alkylene, p-benzylene, p-xylylene or a single bond,

$\overset{\oplus}{E}$ represents $-\overset{\oplus}{N}(R^1R^2)-$, 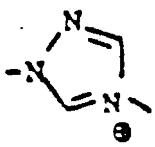, 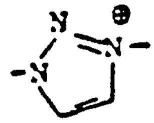; 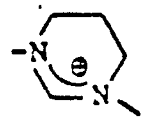,

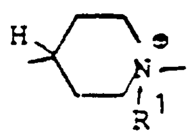 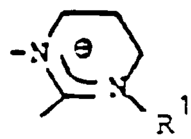, 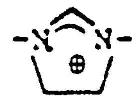, 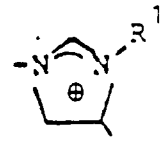

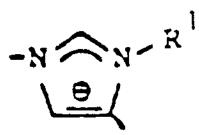 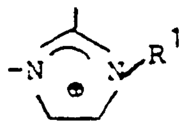, 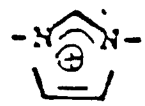 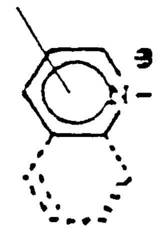

wherein these rings can be optionally substituted by 1-4 methyl groups, and each of the two free valencies can be bonded to $-X^1-Y^1-Z^1$ or to $-Z^2-Y^2-X^2-G-$,

$\overset{\oplus}{E}{}^1$ represents $-\overset{\oplus}{N}(R^1R^2R^2)$,

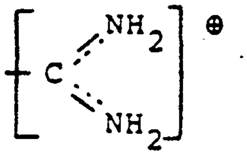, 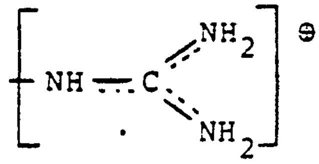

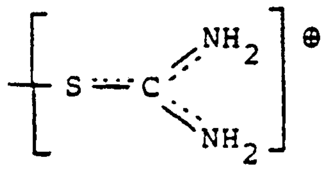, 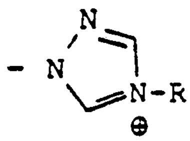, 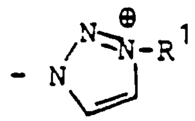,

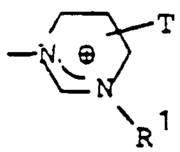, 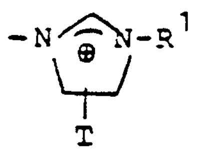, 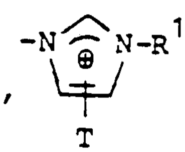, 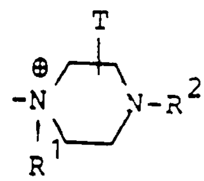,

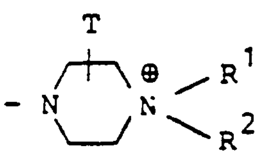, 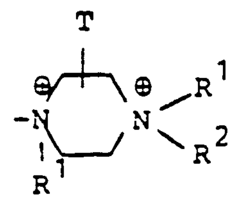, 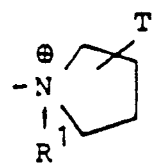,

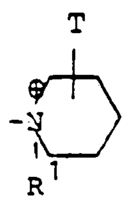, 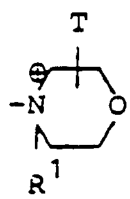, 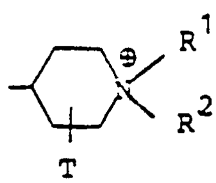,

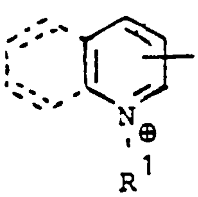, 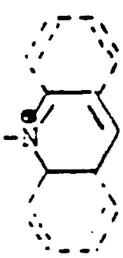, 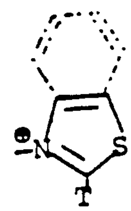 or 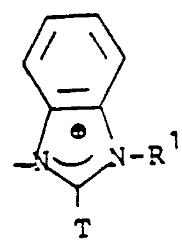

T represents hydrogen or - depending on the nature of the ring - represents 1-4 methyl groups, wherein the aromatic radicals, in addition to 1 or 2 nitro groups, can also be substituted by $C_1$-$C_4$-alkyl , halogen, cyano or carbamoyl and the amidinium, guanidinium and thiuronium radicals can also be substituted by 1-2 $R^2$ radicals,

$R^1$ and $R^2$, independently of each other, represent hydrogen, $C_1$-$C_4$-alkyl , which can be substituted by OH, $NH_2$, halogen, $C_1$-$C_4$-alkoxy, COON, $CONH_2$, CN or $C_1$-$C_4$-alkoxycarbonyl, $C_2$-$C_4$-alkenyl, cyclohexyl, phenyl-$C_1$-$C_3$-alkyl or benzoylmethyl which, in addition to nitro, can each also be ring-substituted by $C_1$-$C_4$-alkyl, $C_1$-$C_4$-alkoxy, Cl or Br,

$R^1$ also represents $-O]^{\ominus}$ or $A$-$X^1$-$Y^1$-$Z^1$,

R and $R^1$ can also be cyclised and then, together with -N-$Z^1$-N- or -N-$Z^2$-N-, form a piperazine radical which is optionally substituted by 1 or 2 methyl groups, or

$R^1$ and $R^2$ can also be cyclised and then, together with the nitrogen atom to which they are bonded,

form a pyrrolidine, piperidine, morpholine or piperazine ring, each of which is optionally substituted by 1 to 4 methyl groups and can be substituted on the second N atom by optionally OH- or $NH_2$-substituted $C_1$- to $C_3$-alkyl,

G represents -NH-, $-NH-CH_2-$, -O- or a single bond,

w represents an r-dentate radical from the group comprising benzene, naphthalene, anthracene, phenanthrene, 9,10-dihydrophenanthrene, cyclohexane, fluoren-9-one(3,6), thiophene (2,5), dibenzofuran(3,6), dibenzothiophene(3,6), dibenzothiophene-S-dioxide(2,7),9-H-thioxanthene-S-dioxide(3,6), carbazole(3,6), 9-H-xanthen-9-one(2,7), 9-acridone(2,7), 1,3,4-oxadiazole(2,5), 1,2,4-oxadiazole(3,5), 1,3,4-thiadiazole(2,5), s-triazine(2,4,6), piper-azine(1,4), 1,2-dihydro-1,2,4,5-tetrazine-(3,6) or represents a radical of

the formulae

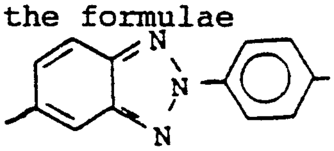 , 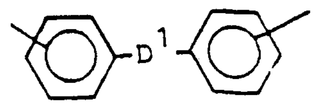 , 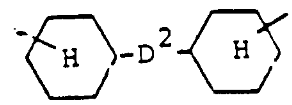

or for the case where $-\overset{\oplus}{E}-$ -denotes a bidentate pyridinium or quinolinium radical also represents a single bond,

$D^1$ and $D^2$, independently of each other, represent a straight-chain or branched $C_1$-$C_7$-alkylene radical which is optionally interrupted by -O- or represent $-O-C_2-C_4$-alkylene-O-, -O-, -NH-, $-N(C_1-C_2$-alkyl)-, -CO-, -CO-NH-, -NH-CO-NH-, 1,1-cyclohexylene or a direct bond,

$D^1$ can also represent $-CH(C_6H_5)-$, $-CH(C_6H_4-)-$, $-N(C_6H_5)-$, -CH=CH-, -S-, $-SO_2$-, -SO-, m- or p-phenylene, thiophene(2,5), 1,3,4-oxadiazole(2,5), 1,3,4-thiadiazole(2,5), oxazole(2,5), thiazole(2,5), 1,2-dihydro-1,2,4,5-tetrazine(3,6) or

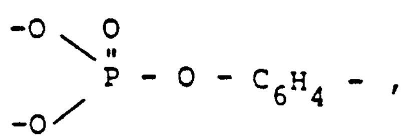 ,

$D^2$ can also represent $-CH(C_6H_{11})-$ or $-CH(C_6H_{10}-)-$, where the rings mentioned under W, $D^1$ and $D^2$, in addition to nitrogen, can also be substituted by $C_1$-$C_4$-alkyl, $C_1$-$C_4$-alkoxy, halogen, nitro and/or a sulpho group,

n represents an integer from 1 to 6, a represents the number of anionic and/or $\overset{\oplus}{N}$ $-\overset{\ominus}{C}$ groups and correspondingly represents 0, 1, 2 or 3,

r represents 2 or 3 and is $\geq$ a, and

$An^{\ominus}$, if present, represents a colourless anion, and wherein the chains occurring r times can be identical or different, and wherein, in the case of r = 2 the grouping $-\overset{\oplus}{E}-Z^2-Y^2-X^2-G-W-G-X^2-Y^2-Z^2-\overset{\oplus}{E}-$ as a whole can also represent

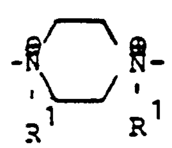 or 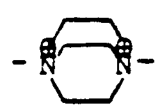

4. Process according to Claim 1, characterized in that the compounds have the general formula

$$(O_2N)_n \left[ (A-X^1-Y^1-Z^1-Y')_e W^1 (G^2-Y^2-Z^2-\overset{\oplus}{E}{}^1)_f \right] (An^{\ominus})_x$$

wherein

Y, represents -O-, -S-, -NH-, $-N(CH_3)-$ or a single bond,

e represents 1, 2 or , if $W^1$ and/or $\overset{\oplus}{E}{}^1$ as a whole contain at least one nitro group and $W^1 \neq W$, also

represents 0,
f represents 2 or 3,
x, depending on the number of anionic and/or $-\overset{\oplus}{N}-\overset{\ominus}{O}$ -groups in the molecule and depending on f, represents 0, 1, 2 or 3,
$W^1$ represents

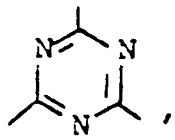 , 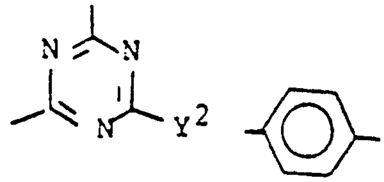

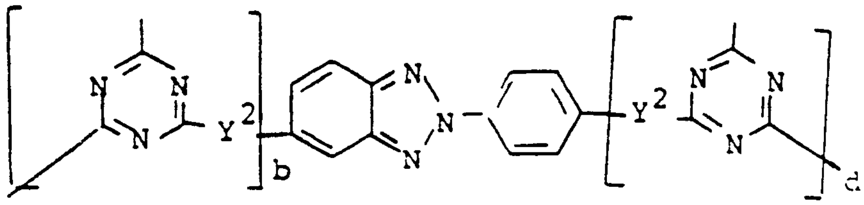 ,

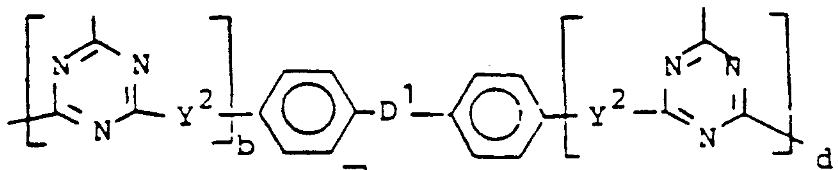 ,

or 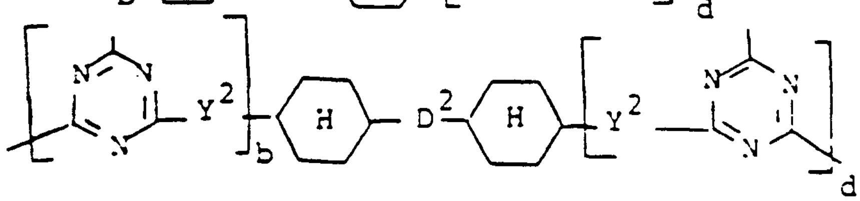

b and d represent 0 or 1 and
$G^2$ represents -NH-CO- or a single bond and wherein
n , A, $\overset{\oplus}{E}{}^1$, $X^1$, $Y^1$, $Y^2$, $Z^1$, $Z^2$ $R^1$, $R^2$, x and $An^{\ominus}$ have the same meaning as in Claim 3.

5. Process according to Claim 1, characterized in that use is made of oligomeric or polymeric compounds which contain per repeat unit 1-3 nitro groups and 1-2 cationic $\overset{\oplus}{E}{}^1$ groups, $\overset{\oplus}{E}{}^1$ having the same meaning as in Claim 4, characterized in that they are obtained:
a) by polymerisation or copolymerisation of one or more monomers from the group comprising maleic anhydride,

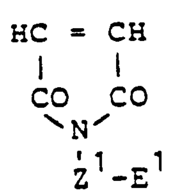 , 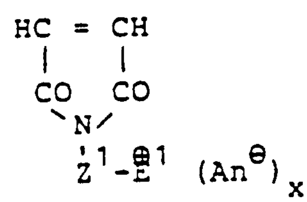 ,

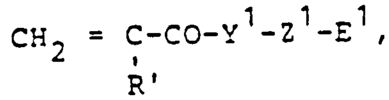

$$CH_2 = \underset{R'}{C}-CO-Y^1-Z^1-\overset{\oplus}{E}^1 \quad (An^{\ominus})_x,$$

N-allylpyridinium halide or its adduct with a nitrobenzaldehyde, $E^1$-$CH=CH_2$, such as 2- or 4-vinylpyridine, and optionally in addition vinyl chloride, vinyl acetate, styrene, $\alpha$-methylstyrene or 4,4'-divinylbenzene which can each be ring-substituted by methyl, chlorine, $E^1(CH_2)v$-, $\overset{\oplus}{E}{}^1$-$(CH_2)v$- and/or nitro,

wherein

R' represents hydrogen or methyl,

$E^1$ represents a basic group convertible by reaction with $R^1L$ into -$\overset{\oplus}{E}{}^1$,

L represents a group detachable as an anion and

v represents 0 or 1 and

$Y^1$, $Z^1$, $\overset{\oplus}{E}{}^1$, $An^{\ominus}$, X and $R^1$ have the abovementioned meaning, in the event that maleic anhydride is used as the monomer by further reaction with a compound of the formula

HN(R)-$Z^1$-$E^1$

or

HN(R)-$Z^1$-$\overset{\oplus}{E}{}^1$ $(An^{\ominus})_x$,

wherein R, $Z^1$, $E^1$, $\overset{\oplus}{E}{}^1$, $An^{\ominus}$ and X have the abovementioned meaning,

and, if non-cationic $E^1$ groups are still present, by their further reaction with $R^1$-L,

wherein $R^1$ and L have the abovementioned meaning, and optionally by subsequent mononitration or dinitration of existing aromatic groups

or

b) by polycondensation of di-($C_2$-$C_3$-alkylene)-triamines or tri-($C_2$-$C_3$-alkylene)-tetramines of the formula

$$H_2N-[(CH_2)_k-E]_w-(CH_2)_k-NH_2$$

wherein

k represents 2 or 3,

w represents 1 or 2 and

-E- represents a basic group which can be converted into -$\overset{\oplus}{E}$- by reaction with $R^1$-L, with dicarboxylic acids of the formula

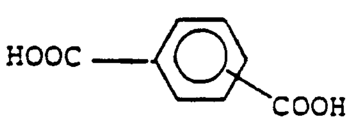

and/or HOOC-$(CH_2)_g$-COOH

or their derivatives (such as acid chlorides, anhydrides, lower alkyl esters, hydrochlorides of the imino derivatives of lower alkyl esters or nitriles) wherein g represents an integer from 0 to 6,

by further reaction with $R^1$-L,

wherein $R^1$ and L have the abovementioned meaning, and optionally subsequent mononitration or dinitration of existing aromatic groups.

formula

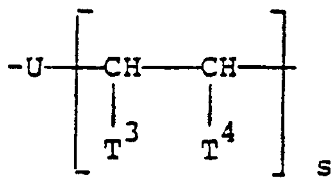

or

$-[NH-(CH_2)_k-B^1-CO]_z-B^2-$

wherein

U represents 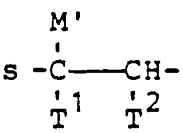 , 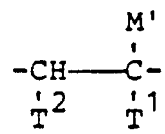 or 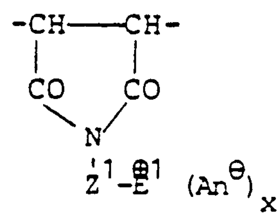

6. Process according to Claim 5, characterized in that the compounds used contain repeat units of the

s represents 0 or 1,

M' represents hydrogen or methyl,

$T^1$ represents $-CO-N(R)-Z^1-E^{\oplus 1}(An^{\ominus})_x$ or

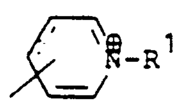 $(An^{\ominus})_x$ ,

$T^2$ represents $T^1$, COOH or - where $T^1$ =

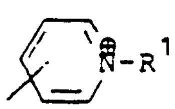 $(An^{\ominus})_x$ - represents hydrogen,

and one of the radicals $T^3$ and $T^4$ represents phenyl, toluyl or chlorophenyl which can each be substituted by nitro and the other represents hydrogen or methyl,

$B^1$ 1) represents 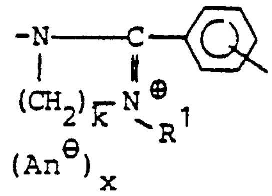 or

2) represents 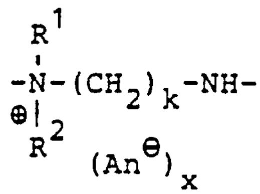

$B^2$ - if $B^1$ has the meaning given under 1) and z = 0 or 1 - represents

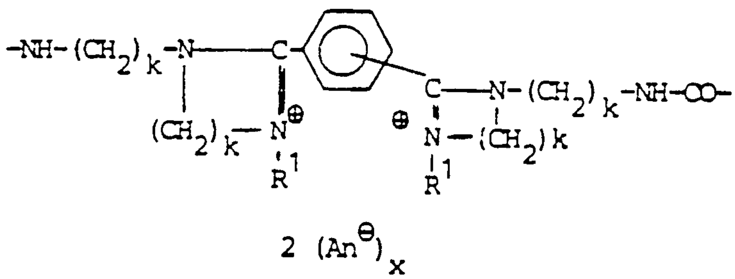

and - if B' has the meaning given under 2) and

z = 1 - represents 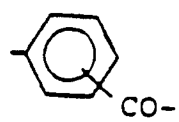 or $-(CH_2)_g-CO-$,

k represents 2 or 3 and
g represents an integer from 1 to 6 and wherein
x, Z', E', R', $R^2$ and $An^{\ominus}$ have the same meaning as in Claim 4, the number of acid and or $-o]^{\ominus}$ groups is equal to or smaller than that of the cationic charges and wherein 1 - 3 aromatically bonded nitro groups are present per repeat unit.

**7.** Nitro compounds of the general formula

$$(O_2N)_n [(A-X^1-Y^1-Z^1-\overset{\oplus}{E}-Z^2-Y^2-X^2-G)_r W] \quad (r-a).An^{\ominus}$$

wherein
A represents a benzene, biphenyl, napthalene, furan, benzofuran, thiophene, benzothiophene, thiazole, benzothiazole, benzimidazole, 1,3,4-thiadiazole or indole radical which, in addition to nitro, can also be substituted by $C_1$-$C_4$-alkyl, halogen, carbamoyl, sulphamoyl, cyano, hydroxyl, $C_1$-$C_4$-alkoxy or a sulpho group,
X' represents $-(CH_2)_t-CO-$, $-CH_2-Y^1-CO-$, $-(CH_2)_t-SO_2-$, $-O-CH_2-CO-$, $-O-CH_2-SO_2-$, $-S-CH_2-CO-$, $-S-CH_2-SO_2-$ or a single bond,
t represents 0, 1 or 2,
$X^2$ represents -CO-, -CO-NH-CO-, $SO_2$- or a single bond,
$Y^1$ and $Y^2$ each represent -O-, -S-, -N(R)-, -N(A)-NH- or a single bond,

R represents hydrogen, $C_1$,-$C_2$-alkyl or cyanoethyl or, in $Y^1$ and $Y^2$, also represents -CO- or -$SO_2$-, each of which can be bonded to the O- or peri position of A or of a benzene ring W,
$Z^1$ and $Z^2$ each represent $C_1$,-$C_6$-alkylene, p-benzylene, p-xylylene or a single bond,

$\overset{\oplus}{E}$ represents $-\overset{\oplus}{N}(R^1R^2)-$, 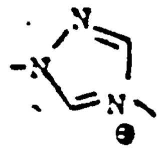 , 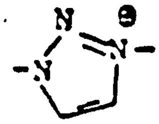 ; 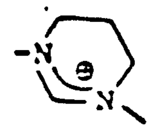 ,

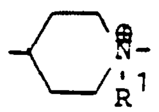 , 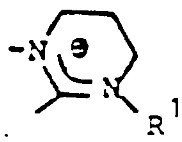 , 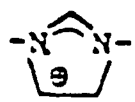 , 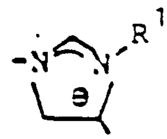 ,

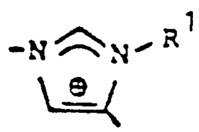 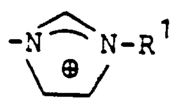 , 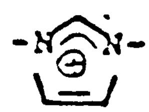 or

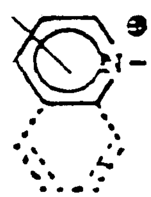

wherein these rings can be optionally substituted by 1 - 4 methyl groups, and each of the two free valencies can be bonded to -$X^1$-$Y^1$-$Z^1$- or to -$Z^2$-$Y^2$-$X^2$-G-,

$\overset{\oplus}{E}{}^1$ represents $-\overset{\oplus}{N}(R^1R^2R^2)$, 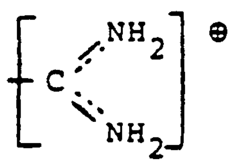 , 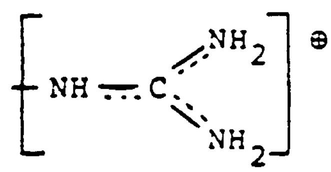

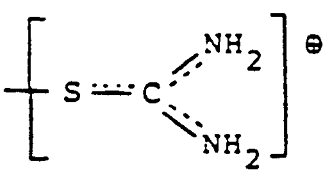 , 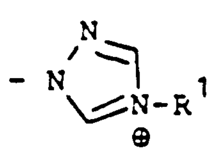 , 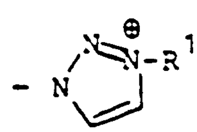 ,

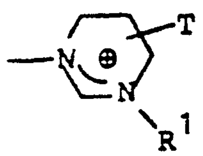 , 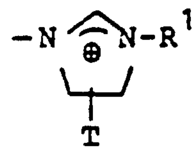 , 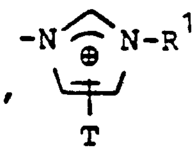 , 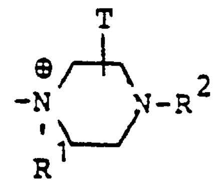 ,

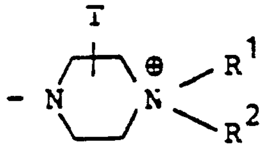 , 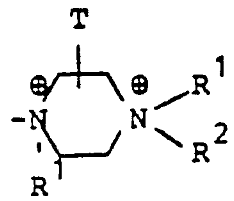 , 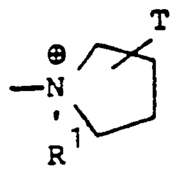 ,

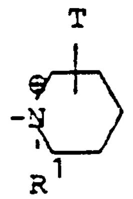 , 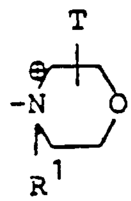 , 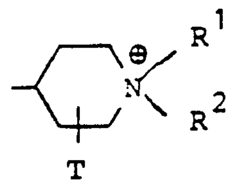 ,

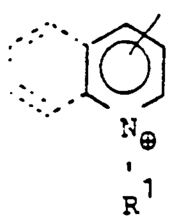 , 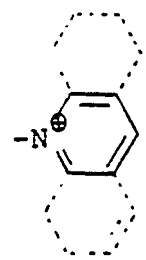 , 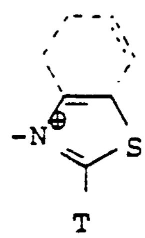 , 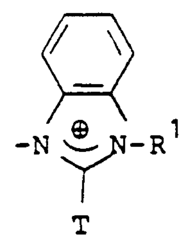

T represents hydrogen or - depending on the nature of the ring - represents 1-4 methyl groups, wherein the aromatic radicals, in addition to 1 or 2 nitro groups, can also be substituted by $C_1$-$C_4$-alkyl, halogen, cyano or carbamoyl and the amidinium, guanidinium and thiuronium radicals can also be substituted by 1-2 $R^2$ radicals, $R^1$ and $R^2$, independently of each other, represent hydrogen, $C_1$-$C_4$-alkyl, which can be substituted by OH, $NH_2$, halogen, $C_1$-$C_4$-alkoxy, COON, $CONH_2$, CN or $C_1$-$C_4$-alkoxycarbonyl, $C_2$-$C_4$-alkenyl, cyclohexyl, phenyl-$C_1$-$C_3$-alkyl or benzoylmethyl which, in addition to nitro, can each also be ring-substituted by $C_1$-$C_4$-alkyl, $C_1$-$C_4$-alkoxy, Cl or Br,

$R^1$ also represents $-O]^{\ominus}$ or A-$X^1$-$Y^1$-$Z^1$-, R and $R^1$ can also be cyclised and then, together with -N-$Z^1$-N- or -N-$Z^2$-N-, form a piperazine radical which is optionally substituted by 1 or 2 methyl groups, or

$R^1$ and $R^2$ can also be cyclised and then, together with the nitrogen atom to which they are bonded, form a pyrrolidine, piperidine, morpholine or piperazine ring, each of which is optionally substituted by 1 to 4 methyl groups and can be substituted on the second N atom by optionally OH- or $NH_2$-substituted $C_1$- to $C_3$-alkyl,

G represents -NH-, -NH-$CH_2$-, -O- or a single bond,

W represents an r-dentate radical from the group comprising benzene, naphthalene, anthracene, phenanthrene, 9,10-dihydrophenanthrene, cyclohexane, fluoren-9-one(3,6), thiophene(2,5), dibenzofuran(3,6), dibenzothiophene(3,6), dibenzothiophene-S-dioxide(2,7), 9-H-thioxanthene-S-dioxide(3,6), carbazole(3,6), 9-H-xanthen-9-one(2,7), 9-acridone(2,7), 1,3,4-oxadiazole(2,5), 1,2,4-oxadiazole(3,5), 1,3,4-thiadiazole(2,5), s-triazine(2,4,6), piperazine-(1,4), 1,2-dihydro-1,2,4,5-tetrazine-(3,6) or represents a radical of the formula

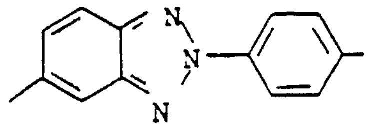 , 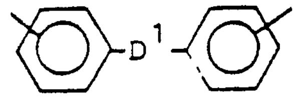

or

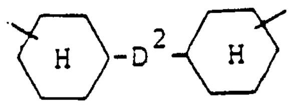 ,

or for the case where $-\overset{\oplus}{E}-$ denotes a bidentate pyridinium or quinolinium radical also represents a single bond,

$D^1$ and $D^2$, independently of each other, represent a straight-chain or branched $C_1$-$C_7$-alkylene radical which is optionally interrupted by -O- or represent -O-$C_2$-$C_4$-alkylene-O-, -O-, -NH-, -N($C_1$-$C_2$-alkyl)-, -CO-, -CO-NH-, -NH-CO-NH-, 1,1-cyclohexylene or a direct bond,

$D^1$ can also represent -CH($C_6H_5$)-, -CH($C_6H_4$)-, -N($C_6H_5$)-, -CH=CH-, -S-, $-SO_2-$, -SO-, m- or p-phenylene, thiophene(2,5), 1,3,4-oxadiazole(2,5), 1,3,4-thiadiazole(2,5), oxazole(2,5), thiazole(2,5), 1,2-dihydro-1,2,4,5-tetrazine(3,6) or

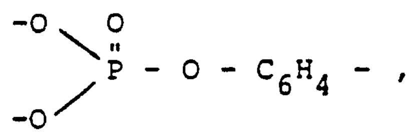 ,

$D^2$ can also represent -CH($C_6H_{11}$)- or -CH($C_6H_{10}$-)-, where the rings mentioned under W, $D^1$ and $D^2$, in addition to nitrogen, can also be substituted by $C_1$-$C_4$-alkyl, $C_1$-$C_4$-alkoxy, halogen and/or a sulpho group,

n represents an integer from 1 to 6,

a represents the number of anionic and/or $\overset{\oplus}{N}-\overset{\ominus}{O}$ groups and correspondingly represents 0, 1, 2 or 3,

r represents 2 or 3 and is > a, and

$An^{\ominus}$, if present, represents a colourless anion, and wherein the chains occurring r times can be identical or different, and wherein, in the case of r = 2 the grouping $-\overset{\oplus}{E}-Z^2-Y^2-X^2-G-W-G-X^2-Y^2-Z^2-\overset{\oplus}{E}-$ as a whole can also represent

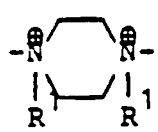 or 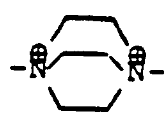

with the proviso that Z is different from $-CN_2-$ when $X^2$, $Y^2$ and G represent a single bond, r represents 2 and W represents phenylene or cyclohexylene.

8. Nitro compounds of the general formula

$$(O_2N)_n \left[ (A-X^1-Y^1-Z^1-Y')_e W^1-(G^2-Y^2-Z^2-\overset{\oplus}{E}^1)_f \right] (An^{\ominus})_x$$

wherein

y, represents -O-, -S-, -NH-, -N($CH_3$)- or a single bond,

e represents 1, 2 or , if $W^1$ and/or $\overset{\oplus}{E}{}^1$ as a whole contain at least one nitro group and $W^1 \neq W$, also represents 0,

f represents 2 or 3,

, x, depending on the number of anionic and/or $-\overset{\oplus}{N}-\overset{\ominus}{O}$ -groups in the molecule and depending on f, represents 0, 1, 2 or 3,

$W^1$ represents

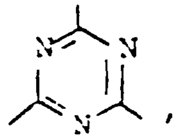 ,

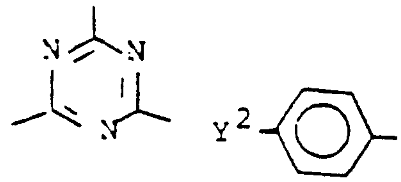 ,

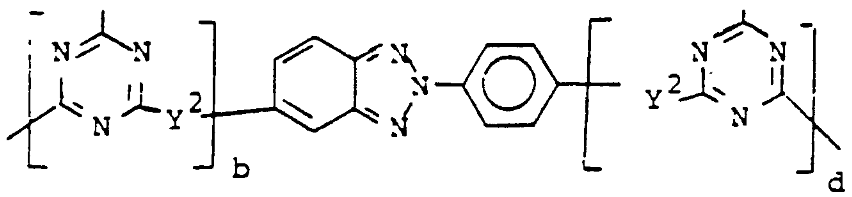 ,

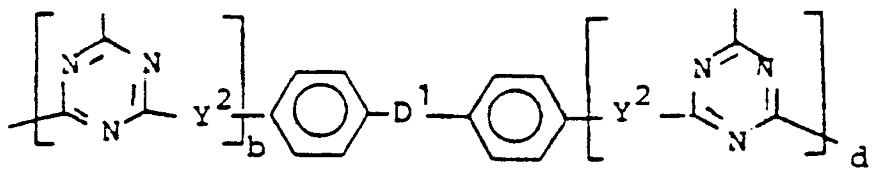 ,

or 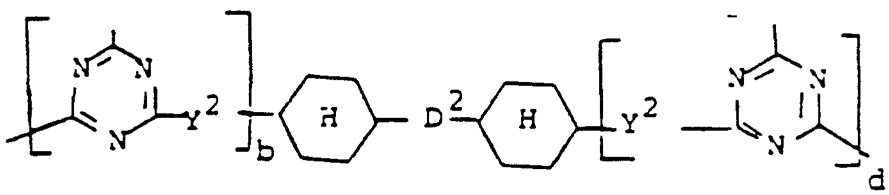

b and d represent 0 or 1 and
$G^2$ represents -NH-CO- or a single bond and wherein n , A, $\overset{\bullet}{E}{}^1$, $X^1$ $Y^1$, $Y^2$, $Z^1$, $Z^2$, $R^1$, $R^2$, x and $An^{\ominus}$ have the same meaning as in Claim 4.

9. Nitro compounds which contain repeat units of the formula

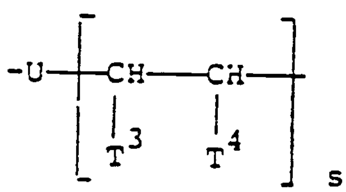

or

$-[NH-(CH_2)_k-B^1-CO]_z-B^2-$

wherein

U represents 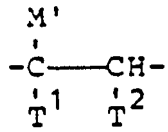 , 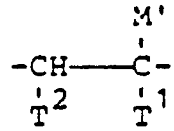 or 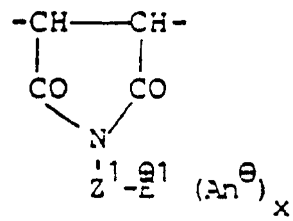

s represents 0 or 1,
M' represents hydrogen or methyl, $T^1$ represents $-CO-N(R)-Z^1-\overset{\oplus}{E}{}^1 (An^{\ominus})_x$ or

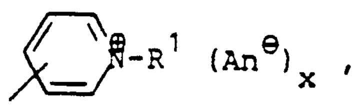

$T^2$ represents $T^1$, COOH or - where $T^1$ =

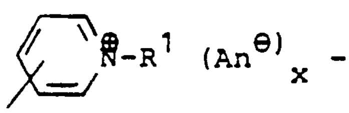 represents hydrogen

and one of the radicals $T^3$ and $T^4$ represents phenyl, toluyl or chlorophenyl which can each be substituted by nitro and the other represents hydrogen or methyl,

$B^1$ 1) represents 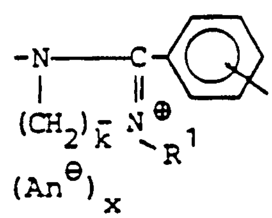 or

2) represents 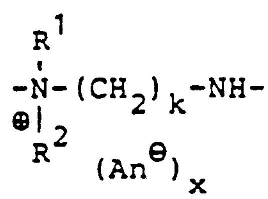

$B^2$ - if $B^1$ has the meaning given under 1) and
z = 0 or 1 - represents

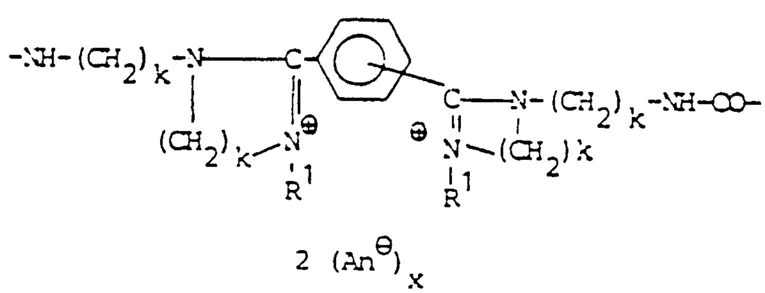 and

- if $B^1$ has the meaning given under 2) and

z = 1 - represents 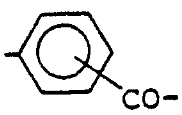 or $-(CH_2)_g-CO-$,

k represents 2 or 3 and
g represents an integer from 1 to 6 and wherein x, $Z^1$, $E^1$, $R^1$, $R^2$ and $An^{\ominus}$ have the same meaning as in Claim 4,
the number of acid and/or $-o]^{\ominus}$ groups is equal to or smaller than that of the cationic charges and wherein 1 - 3 aromatically bonded nitro groups are present per repeat unit.

## Revendications

1. Procédé pour supprimer la fluorescence produite par des azureurs optiques anioniques par action de composés cationiques ou amphotères solubles dans l'eau et pratiquement incolores, caractérisé en ce que les composés contiennent dans la molécule au moins un groupe ammonium, au moins deux groupes aryle, arylène, hétéroaryle et/ou hétéroarylène, et au moins un groupe nitro.

2. Procédé selon la revendication 1, caractérisé en ce que les composés contiennent:
2 à 6 groupes ammonium, acycliques ou combinés à l'état de chaînons dans des cycles saturés, partiellement saturés ou hétéroaromatiques,
2 à 12 groupes mono- ou di-valents de la série du benzène, du furanne, du thiophène, du pyrrole, du pyrazole, de l'oxazole, du thiazole, du 1,2,4-oxadiazole, du 1,3,4-oxadiazole, du 1,3,4-thiadiazole, de l'imidazole, du 1,2,4-triazole, du pyranne, de la pyridine, de la pyrimidine, de la pyrazine, ou de la s-triazine, qui peuvent être condensés avec des noyaux aromatiques ou cycloaliphatiques à 5 ou 6 chaînons et ou substitués par des substituants non ioniques,
et 2 à 9 groupes nitro, ou bien - lorsqu'il s'agit de composés oligomères ou polymères - ils contiennent 1 à 2 groupes ammonium du type mentionné ci-dessus, 1 à 3 groupes aryle ou arylène et 1 à 3 groupes nitro par motif répété.

3. Procédé selon la revendication 1, caractérisé en ce que les composés répondent à la formule générale

$$(O_2N)_n \left[ (\overset{\oplus}{R}^1-Z^2-Y^2-X^2-G-)_r \; W \right]^{(r-a)} \qquad An^{\ominus}$$

dans laquelle
$\overset{\oplus}{R}^1$ représente $\overset{\oplus}{E}^1$- ou $A-X^1-Y^1-Z^1-\overset{\oplus}{E}$-
A représente un radical de benzène, de biphényle, de naphtalène, de furanne, de benzofuranne, de thiophène, de benzothiophène, de thiazole, de benzothiazole, de benzimidazole, de 1,3,4-thiadiazole, d'indole, qui, outre les substituants nitro, peut porter des substituants alkyle en $C_1$-$C_4$, halogéno, carbamoyle, sulfamoyle, cyano, hydroxy, alcoxy en $C_1$-$C_4$ ou un substituant acide sulfonique, $X^1$ représente $-(CH_2)_t-CO-$, $-CH_2-Y^1-CO-$, $-(CH_2)-SO_2-$, $-O-CH_2-CO-$, $-O-CH_2-SO_2-$, $-S-CH_2-CO$, $-S-CH_2-SO_2-$ ou une liaison simple,
t est égal à 0, 1 ou 2, $X^2$ représente -CO-, -CO-NH-CO- ou $SO_2$- ou une liaison simple,
$Y^1$ et $Y^2$ représentent chacun -O-, -S-, -N(R)-, -N(R)-NH- ou une liaison simple,
R représente l'hydrogène, un groupe alkyle en $C_1$-$C_2$ ou cyanéthyle, ou bien encore, dans $Y^1$ et $Y^2$, -CO- ou $-SO^2-$, qui peuvent être reliés avec la position ortho ou péri de A ou d'un cycle benzénique w,
$Z^1$ et $Z^2$ représentent chacun un groupe alkylène en $C_1$-$C_6$, p-benzylène, p-xylylène ou une liaison simple,
$E^{\oplus}$ représente

$-\overset{\oplus}{N}(R^1R^2)-$, 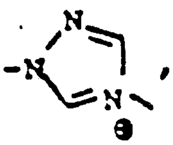, 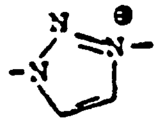; 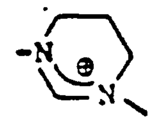,

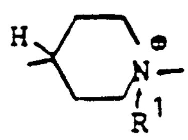 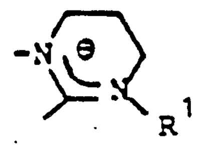, 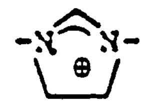, 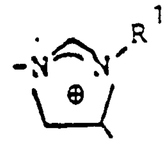

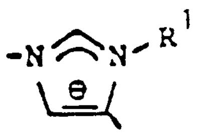 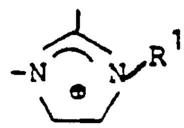, 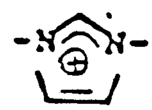 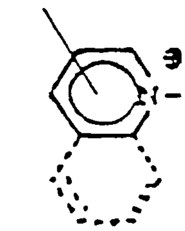

ces cycles pouvant éventuellement être substitués par 1 à 4 groupes méthyle, et chacune des deux valences libres peut être reliée à $-X^1-Y^1-Z^1-$ ou à $Z^2-Y^2-X^2-G-$,

$\overset{\oplus}{E}{}^1$ représente

$-\overset{\oplus}{N}(R^1R^2R^2)$, 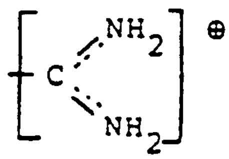, 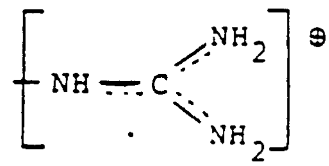,

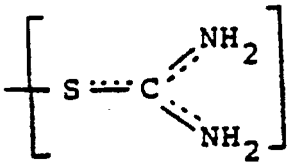 , 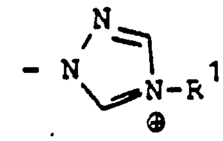 , 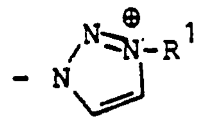 ,

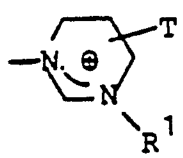 , 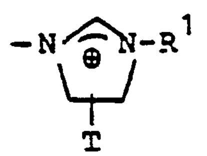 , 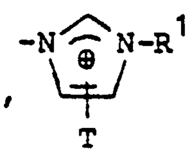 , 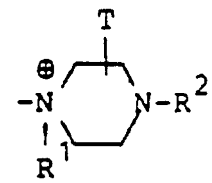 ,

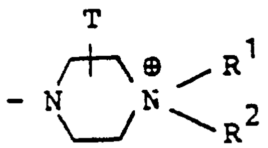 , 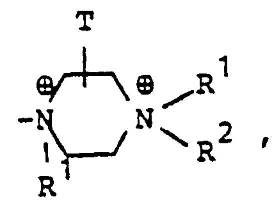 , 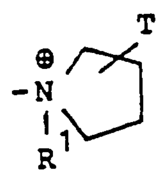 ,

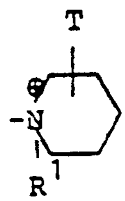 , 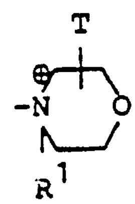 , 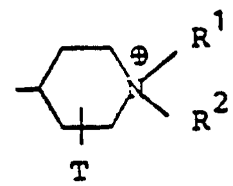 ,

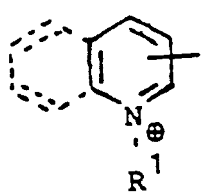 , 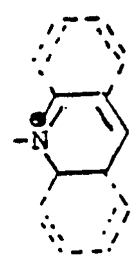 , 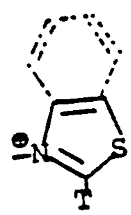 ou 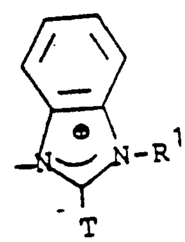

T représente l'hydrogène ou bien - sélon la nature du cycle - 1 a 4 groupes méthyle, les noyaux aromatiques pouvant être substitués non seulement par un ou deux groupes nitro mais également par des groupes alkyle en $C_1$-$C_4$, des halogènes, des groupes cyano ou carbamoyle, et les groupes amidinium, guanidinium et chiuronium également par un ou deux groupes $R^2$,

$R^1$ et $R^2$ représentent chacun, indépendamment l'un de l'autre, l'hydrogène, un groupe alkyle en $C_1$-$C_4$ qui peut être substitué par OH, $NH_2$, des halogènes, des groupes alcoxy en $C_1$-$C_4$, COOH, $CONH_2$, CN ou (alcoxy en $C_1$-$C_4$)-carbonyle, un groupe alcényle en $C_2$-$C_4$, cyclohexyle, phényl-alkyle en $C_1$-$C_3$ ou benzoylméthyle qui, outre les groupes nitro, peuvent être substitués par des groupes alkyle en $C_1$-$C_4$, alcoxy en $C_1$-$C_4$, Cl ou Br sur le noyau,

$R^1$ peut en outre représenter - $\overline{O}|^{\ominus}$ ou A-X'-Y'-Z'-,

R et $R^1$ peuvent également être reliés avec formation d'un cycle et former alors avec -N-Z'-N- ou -N-$Z^2$-N- un radical de pipérazine éventuellement substitué par un à deux groupes méthyle, ou bien

$R^1$ et $R^2$ peuvent également être reliés avec formation d'un cycle et former alors avec l'atome d'azote auquel ils sont reliés un cycle pyrrolidine, pipéridine, morpholine ou pipérazine éventuellement substitué par un à quatre groupes méthyle et qui peut être substitué sur le deuxième atome d'azote par un groupe alkyle en $C_1$-$C_3$ lui-même éventuellement substitué par OH ou $NH_2$,

G représente -NH-, -NH-$CH_2$-, -O- ou une liaison simple,

W représente un radical de valénce r de la série du benzène, du naphtalène, de l'anthracène, du phénanthrène, du 9,10-dihydrophénanthrène, du cyclohexane, de la fluorène-9-one-3,6, du thiophène-2,5, du dibenzofuranne-3,6, du dibenzothiophène-3,6, du S-dioxyde-2,7 du dibenzothiophène, du S-dioxyde-3,6 du 9-H-thioxanthène, du carbazole-3,6, de la 9-H-xanthène-9-one-2,7, de la 9-acridone-2,7,

du 1,3,4-oxadiazole-2,5, du 1,2,4-oxadiazole-3,5, du 1,3,4-thiadiazole-2,5, de la s-triazine-2,4,6, de la pipérazine-1,4, de la 1,2-dihydro-1,2,4,5-tétrazine-3,6, ou un groupe de formule

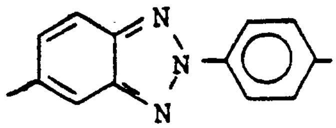 , 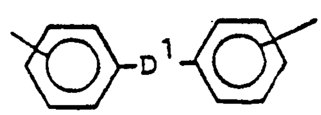 , 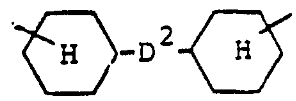

ou bien encore, dans le cas où $-\overset{\oplus}{E}-$ représente un groupe pyridinium ou quinoléinium m divalent, une liaison simple,
$D^1$ et $D^2$ représentent chacun, indépendamment t l'un de l'autre, un groupe alkylène en $C_1$-$C_7$ à chaîne droite ou ramifiée éventuellement interrompue par -O-, un groupe -O-(alkylène e en $C_2$-$C_4$)-O-, -O-, -NH-, -N-(alkyle en $C_1$-$C_2$)-, -CO, -CO-NH-, -NH-CO-NH-, 1,1-cyclohexylène ou une liaison directe,
$D^1$ peut en outre représenter -CH ($C_6H_5$)-, -(CH($C_6H_4$-)-, -N($C_6H_5$)-, -CH=CH-, -S-, -$SO_2$-, -SO-, m- ou p-phénylène, thiophène-2,5, 1,3,4-oxadiazole-2,5, 1 ,3,4-thiadiazole-2,5, oxazole-2,5, thiazole-2,5, 1,2-dihydro-1,2,4,5-tétrazine-3,6 ou

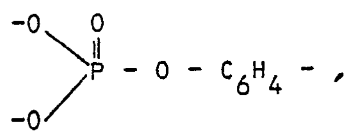

$D^2$ peut en outre représenter -CH($C_6H_{11}$)- ou -CH($C_6H_{10}$-)-, les cycles mentionnés en référence à W,D' et $D^2$ pouvant en outre être substitués, non seulement par des groupes nitro, mais par des groupes alkyle en $C_1$-$C_4$, alcoxy en $C_1$-$C_4$, des halogènes, des groupes nitro et/ou un groupe acide sulfonique,
n est un nombre entier allant de 1 à 6,
a est le nombre des groupes anioniques et/ou $\overset{\oplus}{N}-\overset{\ominus}{O}-$ correspondant à 0, 1, 2 ou 3,
r est égal à 2 ou 3, mais égal ou supérieur à a, et
$An^{\ominus}$, lorsqu' il existe, représente un anion incolore, les chaînes présentes r fois pouvant être identiques ou différentes,
et dans le cas où r=2, le groupement $-\overset{\oplus}{E}-Z^2-Y^2-X^2-G-W-G-X^2-Y^2-Z^2-\overset{\oplus}{E}-$, au total, pouvant également représenter

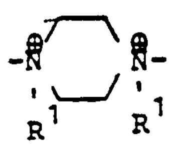 ou 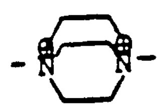

**4.** Procédé selon la revendication 1, caractérisé en ce que les composés répondent à la formule générale

$$(O_2N)_n\left[(A-X^1-Y^1-Z^1-Y')_e\,W^1\,(G^2-Y^2-Z^2-\overset{\oplus}{E}{}^1)_f\right](An^{\ominus})_x$$

dans laquelle
Y' représente -O-, -S-, -NH-, -N($CH_3$)- ou une liaison simple, e est égal à 1, 2 ou bien encore, lorsque $W^1$ et/ou $\overset{\oplus}{E}{}^1$ contiennent au total au moins un groupe nitro et que $W^1 \neq$ W, O,
f est égal à 2 ou 3,
x, selon le nombre des groupes anioniques et/ou $-\overset{\oplus}{N}-\overset{\ominus}{O}-$ dans la molécule et selon la valeur de f, représente 0, 1, 2 ou 3, $W^1$ représente

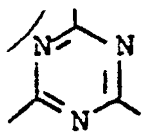, 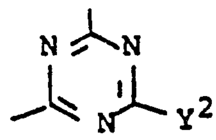 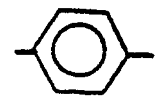

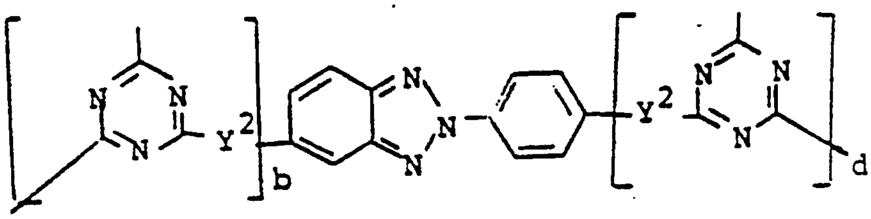,

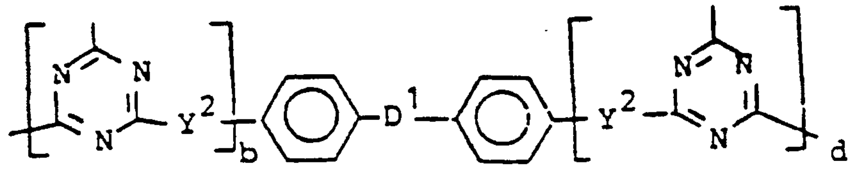,

ou 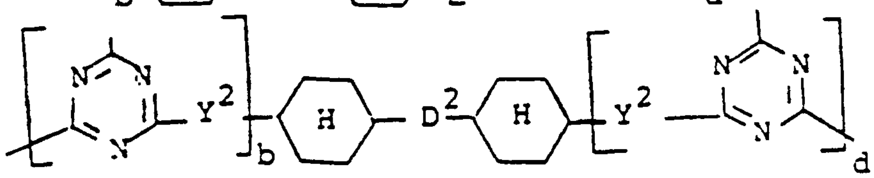

b et d sont égaux à 0 ou 1,
$G^2$ représente -NH-CO- ou une liaison simple, et n, A, $\overset{\oplus}{E}{}^1$, $X^1$, $Y^1$, $Y^2$, $Z^1$, $Z^2$, $R^1$, $R^2$, x et $An^{\ominus}$ ont les significations indiquées dans la revendication 3.

5. Procédé selon la revendication 1, caractérisé en ce que l'on utilise des composés oligomères ou polymères qui contiennent, par motif répété, un à trois groupes nitro et un à deux groupes cationiques $\overset{\oplus}{E}{}^1$, $\overset{\oplus}{E}{}^1$ ayant les significations indiquées dans la revendication 4, caractérisé en ce qu'ils sont obtenus :
a) par polymérisation ou copolymérisation d'un ou plusieurs monomères du groupe formé par l'anhydride maléique,

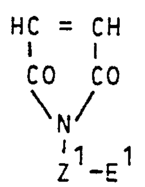, 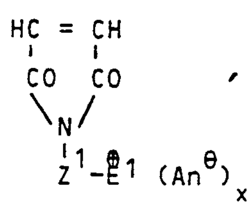,

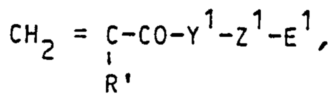,

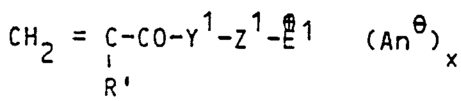

un halogénure de N-allylpyridinium ou son adduct avec un nitro-benzaldéhyde, $E^1-CH=CH_2$, par exemple la 2- ou la 4-vinylpyridine et le cas échéant en outre le chlorure de vinyle, l'acétate de vinyle, le styrène, l'α-méthylstyrène ou le 4,4-divinylbenzène, qui peuvent être substitués dans les noyaux par des groupes méthyle, le chlore, $E^1-(CH_2)_v-$, $\overset{\oplus}{E}{}^1-(CH_2)_v-$ et/ou nitro,
$R^1$ représentant l'hydrogène ou un groupe méthyle,
$E^1$ un groupe basique convertible par réaction avec $R^1$ L en $-\overset{\oplus}{E}{}^1$,
L est un groupe séparable à l'état d'anion, et
v est égal à 0 ou 1,

et $Y^1$, $Z^1$, $\overset{\oplus}{E}{}^1$, $An^\ominus$, X et $R^1$ ont les significations indiquées cidessus, et dans le cas où on a utilisé en tant que monomère l'anhydride maléique, par réaction complémentaire avec un composé de formule

$$HN(R)-Z^1-E^1$$

ou

$$HN(R)-Z^1-\overset{\oplus}{E}{}^1\ (An^\ominus)_X,$$

dans laquelle R, $Z^1$, $E^1$, $\overset{\oplus}{E}{}^1$, $An^\ominus$ et X ont les significations indiquées ci-dessus, et dans le cas où il subsiste encore des groupes $E^1$ non cationiques, par réaction complémentaire de ces groupes avec $R^1$ -L, $R^1$ et L ayant les significations indiquées ci-dessus, et le cas échéant par mono- ou di-nitration subséquente des groupes aromatiques présents, ou bien

b) par polycondensation de di-(alkylène en $C_2$-$C_3$)-tri-amines ou de tri-(alkylène en $C_2$-$C_3$)-tétramines de formule

$$H_2N-\left[(CH_2)_k-E-\right]_w(CH_2)_k-NH_2$$

dans laquelle

k est égal à 2 ou 3,

w est égal à 1 ou 2,

-E- représente un groupe basique convertible en $-\overset{\oplus}{E}-$ par réaction avec $R^1$-L, avec des acides dicarboxyliques de formule

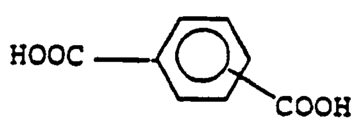 et/ou $HOOC-(CH_2)_g-COOH$

ou leurs dérivés (tels que les chlorures d'acides, les anhydrides, les esters alkyliques inférieurs, les chlorhydrates d'esters imino-alkyliques inférieurs ou les nitriles,

g représentant un nombre entier de 0 à 6,

par réaction complémentaire avec $R^1$-L,

R' et L ayant les significations indiquées ci-dessus,

et le cas échéant mono- ou di-nitration subséquente des groupes aromatiques présents.

**6.** Procédé selon la revendication 5, caractérisé en ce que l'on utilise des composés qui contiennent des motifs répétés de formule

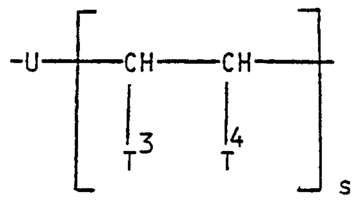 ou

$$-[NH-(CH_2)_k-B^1-CO]_z-B^2-$$

dans laquelle

U représente

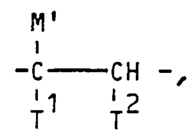, 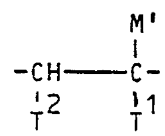 ou 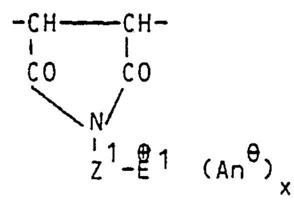

s est égal à 0 ou 1,

M' représente l'hydrogène ou un groupe méthyle,
$T^1$ représente $-CO-N(R)-Z^1-\overset{\oplus}{E}{}^1\ (An^{\ominus})_x$ ou

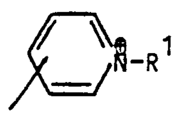 $(An^{\ominus})_x$,

$T^2$ représente $T^1$, COOH ou bien encore - dans le cas où

$T^1$ = 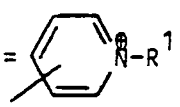 $(An^{\ominus})_x$, l'hydrogène,

et l'un des symboles $T^3$ et $T^4$ représente un groupe phényle, tolyle ou chlorophényle qui peuvent être substitués par des groupes nitro, et l'autre l'hydrogène ou un groupe méthyle.

$B^1$ représente 1) 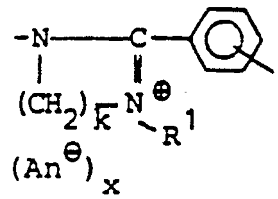 ou

2) 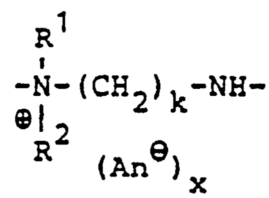

$B^2$ - lorsque $B^1$ a la signification indiquée ci-dessus sous 1) et que Z = 0 ou 1 - représente

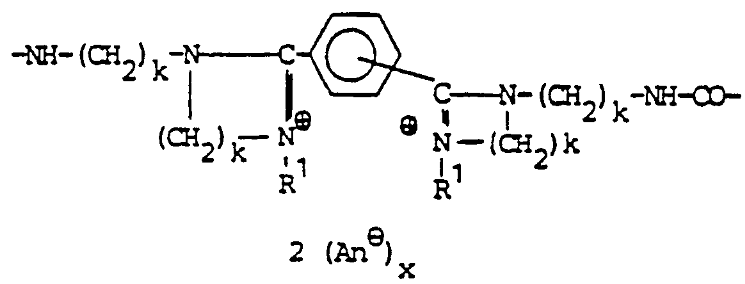

et - lorsque $B^1$ a la signification indiquée ci-dessus sous 2) et

que Z est égal à 1- 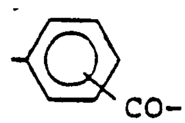 ou

$-(CH_2)_g-CO-$,

k est égal à 2 ou 3,
g est un nombre entier allant de 1 à 6,
x, Z', E', R', $R^2$ et $An^{\ominus}$ ont les significations indiquées dans la revendication 4, le nombre des groupes acides et ou - $O|^{\ominus}$ étant égal ou inférieur à celui des charges cationiques, et chaque motif répété contenant un à trois groupes nitro à liaisons aromatiques.

7. Composés nitrés de formule générale

$$(O_2N)_n - \left[(A-X^1-Y^1-Z^1-\overset{\oplus}{E}-Z^2-Y^2-X^2-G)_r W\right] \quad (r-a).An^{\ominus}$$

dans laquelle
A représente un radical de benzène, de biphényle, de naphtalène, de furanne, de benzofuranne, de thiophène, de benzothiophène, de thiazole, de benzothiazole, de-benzimidazole, de 1,3,4-thiadiazole ou d'indole qui peut être substitué non seulement par des groupes nitro mais également par des groupes alkyle en $C_1$-$C_4$, des halogènes, des groupes carbamoyle, sulfamoyle, cyano, hydroxy, alcoxy en $C_1$-$C_4$ ou un groupe acide sulfonique,
$X^1$ représente $-(CH_2)_t-CO-$, $-CH_2-Y^1-CO-$, $-(CH_2)_t-SO_2-$, $-O-CH_2-CO-$, $-O-CH_2-SO_2-$, $-S-CH_2-CO-$, -S-

$CH_2$-$SO_2$- ou une liaison simple,

t est égal à 0, 1 ou 2,

$X^2$ représente -CO-, -CO-NH-CO- ou -$SO_2$ - ou une liaison simple,

$Y^1$ et $Y^2$ représentent chacun -O-, -S-, -M(R)-, -N(R)-NH- ou une liaison simple,

R représente l'hydrogène, un groupe alkyle en $C_1$-$C_2$ ou cyanéthyle ou bien encore, dans $Y^1$ et $Y^2$ , un groupe -CO- ou -$SO_2$- qui peut être relié avec la position ortho ou péri de A ou d'un cycle benzénique w,

$Z^1$ et $Z^2$ représentent chacun un groupe alkylène en $C_1$-$C_6$, p-benzylène, p-xylylène ou une liaison simple,

$\overset{\oplus}{E}$ représente

$-\overset{\oplus}{N}(R^1R^2)-$, 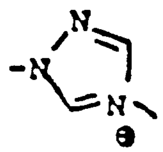 , 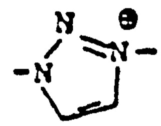 , 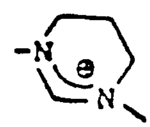

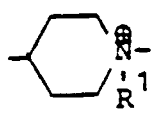 , 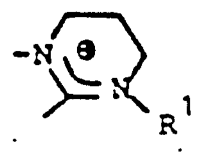 , 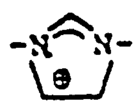 , 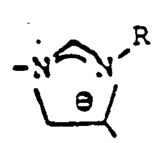 ,

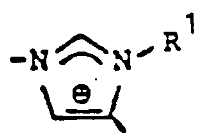 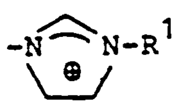 , 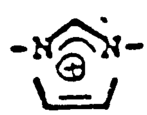 ou

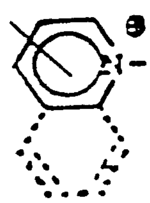

ces noyaux pouvant éventuellement être substitués par un à quatre groupes méthyle et chacune des deux valences libres pouvant être reliée à -X'-Y'-Z'- ou à -$Z^2$-$Y^2$-$X^2$-G-,

$\overset{\oplus}{E}$ ' représente

$-\overset{\oplus}{N}(R^1R^2R^2)$, 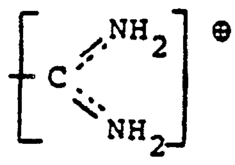 , 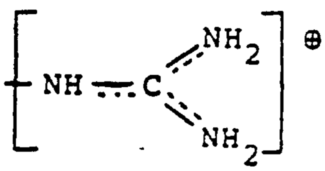 ,

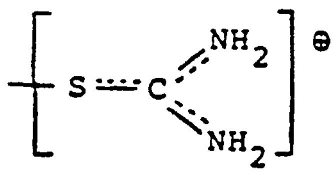 , 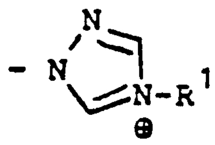 , 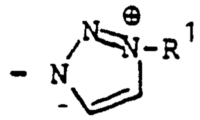 ,

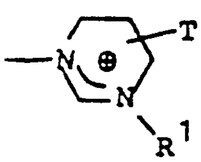 , 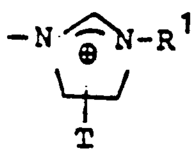 , 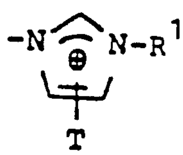 , 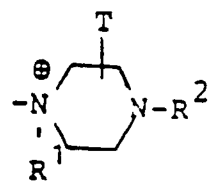 ,

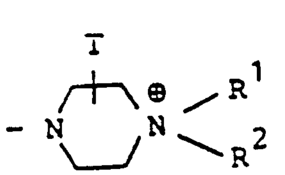 , 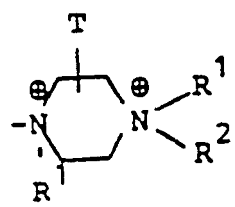 , 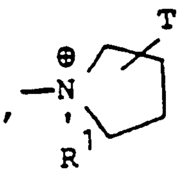 ,

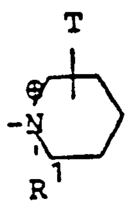 , 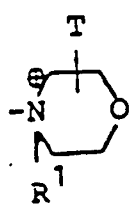 , 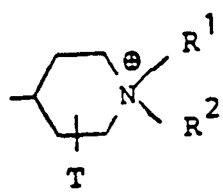 ,

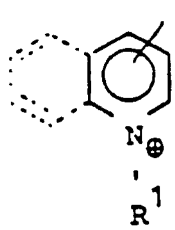 , 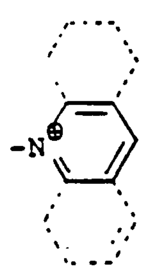 , 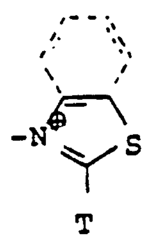 , 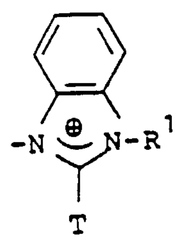

T représente l'hydrogène ou bien - selon la nature du cycle - un à quatre groupes méthyle, les noyaux aromatiques pouvant être substitués non seulement par un à deux groupes nitro mais également par des groupes alkyle en $C_1$-$C_4$, des halogènes, des groupes cyano ou carbamoyle et les radicaux amidinium,guanidinium et thiuronium pouvant également être substitués par un à deux groupes $R^2$,

$R^1$ et $R^2$ représentent chacun, indépendamment l'un de l'autre, l'hydrogène, un groupe alkyle en $C_1$-$C_4$ qui peut être substitué par OH, $NH_2$, des halogènes, des groupes alcoxy en $C_1$-$C_4$, COOH, $CONH_2$, CN ou (alcoxy en $C_1$-$C_4$)-carbonyle , un groupe alcényle en $C_2$-$C_4$, cyclohexyle, phényl-alkyle en $C_1$-$C_3$ ou benzoylméthyle qui peuvent être substitués sur le noyau non seulement par des groupes nitro mais

également par des groupes alkyle en $C_1$-$C_4$, alcoxy en $C_1$-$C_4$, Cl ou Br,

$R^1$ peut en outre représenter $-\overline{\underline{O}}|^{\ominus}$ ou $A$-$X^1$-$Y^1$-$Z^1$-,

R et $R^1$ peuvent également être reliés avec formation d'un cycle et former alors avec -N-$Z^1$ -N- ou -N-$Z^2$-N- un radical de pipérazine éventuellement substitué par un à deux groupes méthyle, ou bien

$R^1$ et $R^2$ peuvent également être reliés entre eux avec formation d'un cycle et former alors avec l'atome d'azote auquel ils sont reliés un cycle pyrrolidine, pipéridine, morpholine ou pipérazine éventuellement substitué par un à quatre groupes méthyle et qui peut le cas échéant être substitué sur le deuxième atome d'azote par un groupe alkyle en $C_1$-$C_3$ lui-même substitué par OH ou $NH_2$,

G représente -NH-, -NH-$CH_2$-, -O- ou une liaison simple,

W représente un radical de valence r de la série du benzène, du naphtalène, de l'anthracène, du phénanthrène, du 9,10-dihydrophénanthrène, du cyclohexane, de la fluorène-9-one-3,6, du thiophène-2,5, du dibenzofuranne-3,6, du dibenzothiophène-3,6, du S-dioxyde-2,7 du dibenzothiophène, du S-dioxyde-3,6 du 9-H-thioxanthène, du carbazole-3,6, de la 9-H-xanthène-9-one-2,7, de la 9-acridone-2,7, du 1,3,4-oxadiazole-2,5, du 1,2,4-oxadiazole-3,5, du 1,3,4-thiadiazole-2,5, de la s-triazine-2,4,6, de la pipérazine-1,4, de la 1,2-dihydro-1,2,4,5-tétrazine-3,6, ou un groupe de formule

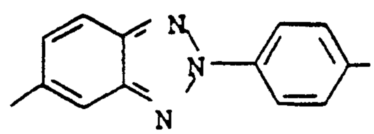 , 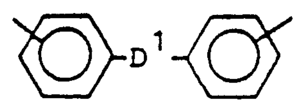 ou

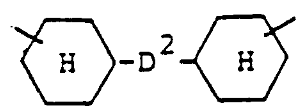 ,

ou bien encore, dans le cas où $-\overset{\oplus}{E}-$ représente un radical pyridinium ou quinoléinium divalent, une liaison simple,

$D^1$ et $D^2$ représentent chacun, indépendamment l'un de l'autre, un groupe alkylène en $C_1$-$C_7$ à chaîne droite ou ramifiée éventuellement interrompue par -O-, un groupe -O-(alkylène en $C_2$-$C_4$)-O-, -O-, -NH-, -N-(alkyle en $C_1$-$C_2$)-, -CO-, -CO-NH-, -NH-CO-NH-, 1,1-cyclohexylène ou une liaison simple,

$D^1$ pouvant en outre représenter -$CH(C_6H_5)$-, -$CH(C_6H_4)$-, -$N(C_6H_5)$-, -CH=CH-, -S-, -$SO_2$-, -SO-, m- ou p-phénylène, thiophène-2,5, 1,3,4-oxadiazole-2,5, 1,3,4-thiadiazole-2,5, oxazole-2,5, thiazole-2,5, 1,2-dihydro-1,2,4,5-tétrazine-3,6 ou

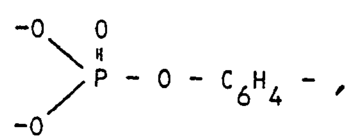 ,

$D^2$ peut en outre représenter -$CH(C_6H_{11})$- ou -$CH_2(C_6H_{10}$-)-, les noyaux mentionnés en référence à W, $D^1$ et $D^2$ pouvant être substitués non seulement par des groupes nitro mais également par des groupes alkyle en $C_1$-$C_4$, alcoxy en $C_1$-$C_4$, des halogènes et/ou un groupe acide sulfonique,

n est un nombre entier allant de 1 à 6,

a est le nombre des groupes anioniques et/ou des groupes $\overset{\oplus}{N}-\overset{\ominus}{O}$ -correspondant à 0, 1, 2 ou 3,

r est égal à 2 ou 3, mais est supérieur ou égal à a, et

$An^{\ominus}$, lorsqu'il existe, représente un anion incolore,

les chaînes présentes r fois pouvant être identiques ou différentes,

et dans le cas où r = 2, le groupement $-\overset{\oplus}{E}$-$Z^2$-$Y^2$-$X^2$-G-W-G-$X^2$-$Y^2$-$Z^2$-$\overset{\oplus}{E}-$ -peut au total représenter également

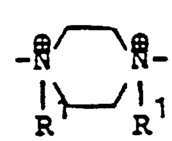 ou 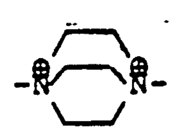

sous réserve que Z ne peut représenter $-CH_2-$ lorsque $X^2$ , $Y^2$ et G représentent une liaison simple, r est égal à 2 et W représente un groupe phénylène ou cyclohexylène.

**8.** Composés nitrés de formule générale

$$(O_2N)_{\overline{n}}\left[(A-X^1-Y^1-Z^1-Y')_{\overline{e}}W^1-(G^2-Y^2-Z^2-\overset{\oplus}{E}{}^1)_f\right] \quad (An^{\ominus})_x$$

dans laquelle

Y' représente -O-, -S-, -NH-, $-N(CH_3)-$ ou une liaison simple,

e est égal à 1, 2 ou bien encore, lorsque $W^1$ et/ou $\overset{\oplus}{E}{}^1$ contiennent au total au moins un groupe nitro et que $w^1 \neq W$, O,

f est égal à 2 ou 3,

x, selon le nombre des groupes anioniques et/ou des groupes $-\overset{\oplus}{N}-\overset{\ominus}{O}$ -dans la molécule et selon f, représente 0, 1, 2 ou 3,

$W^1$ représente

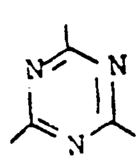,
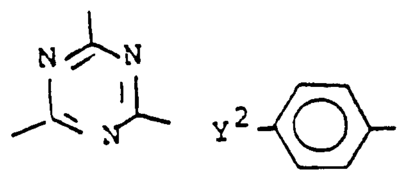,

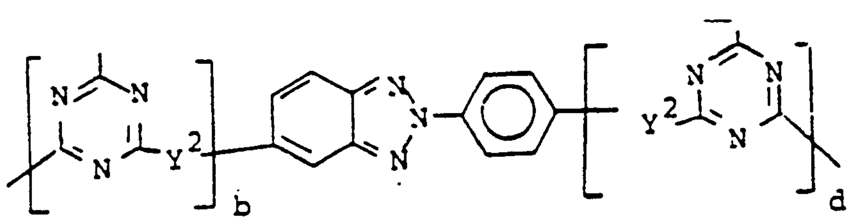,

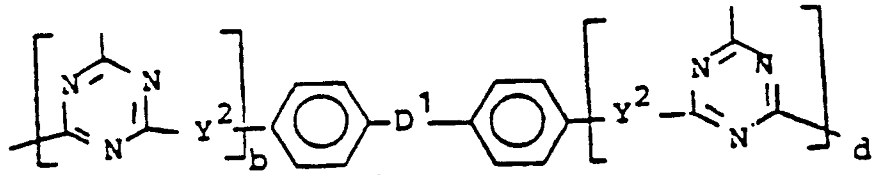,

ou 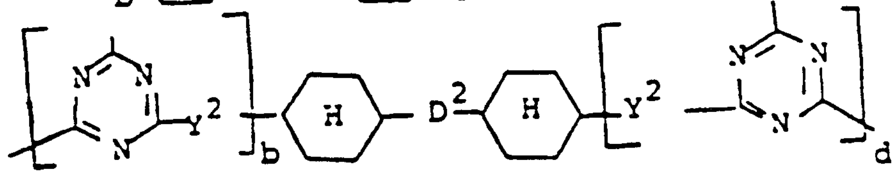

b et d sont égaux à 0 ou 1,

$G^2$ représente -NH-CO- ou une liaison simple, et n, A, $\overset{\oplus}{E}{}^1$, $X^1$, $Y^1$, $Y^2$,$Z^1$,$Z^2$,$R^1$,$R^2$, x et $An^{\ominus}$ ont lessignifications indiquées dans la revendication 4.

**9.** Composés nitrés contenant des motifs répétés de formule

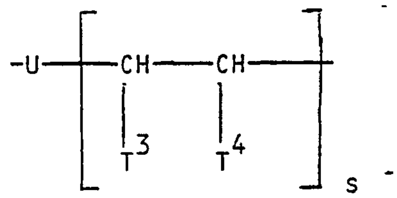

ou

$-[NH-(CH_2)_k-B^1-CO]_z-B^2-$

dans laquelle

U représente

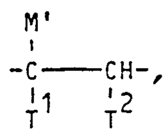

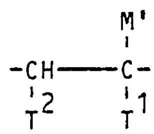 ou 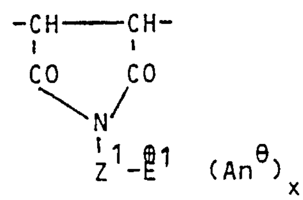

s est égal à 0 ou 1,

M' représente l'hydrogène ou un groupe méthyle,
T' représente $-CO-N(R)-Z'-\overset{\oplus}{E}{}^{1}(An^{\ominus})$ ou

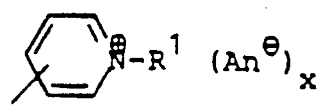

$T^2$ représente T' , COOH ou bien encore - dans le cas où

$T^1$ = 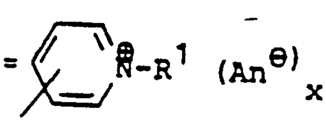 - l'hydrogène,

et l'un des symboles $T^3$ et $Y^4$ représente un groupe phényle, toluyle ou chlorophényle qui peuvent être substitués par des groupes nitro, et l'autre l'hydrogène ou un groupe méthyle,
$B^1$ représente

1) 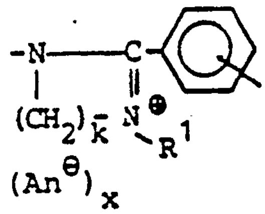 ou

2) 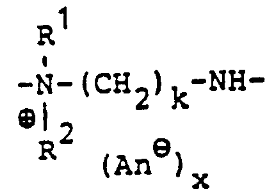

$B^2$ - lorsque $B^1$ a la signification indiquée ci-dessus sous 1) et que Z = 0 ou 1 - représente

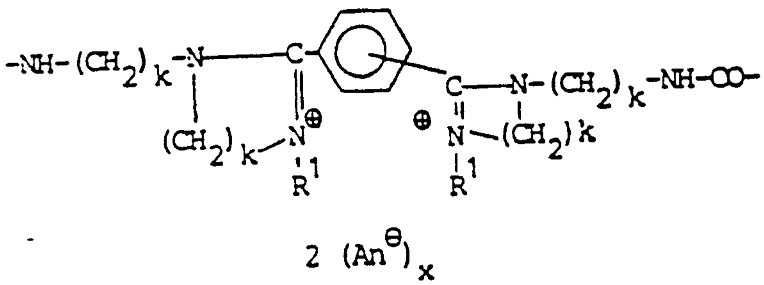 et

- lorsque $B^1$ a la signification indiquée ci-dessus sous 2) et que Z est égal à 1 -

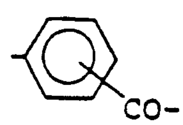 ou

$-(CH_2)_g-CO-$,

k est égal à 2 ou 3,
g est un nombre entier allant de 1 à 6,
x, Z', E', $R^1$, $R^2$ et $An^{\ominus}$ ont les significations indiquées dans la revendication 4, le nombre de groupes acides et/ou - $\bar{O}|^{\ominus}$ est égal ou inférieur à celui des charges cationiques et chaque motif répété contient un à trois groupes nitro à liaisons aromatiques.